# EUROPEAN PATENT APPLICATION

(11) **EP 4 682 146 A1**
(43) Date of publication of application: **21.01.2026**
(21) Application number: 24771254.0
(22) Date of filing: 14.03.2024
(51) Int. Cl.: C07D 471/04, A61K 31/444, A61K 31/497, A61K 31/506, A61K 31/437, A61K 31/5377, A61P 25/28, A23L 33/10

(54) **COMPOSITION INCLUDING NOVEL PYRIDOINDOLE DERIVATIVE COMPOUND AS ACTIVE INGREDIENT FOR PREVENTION OR TREATMENT OF NEURODEGENERATIVE DISEASES**

(30) Priority: 15.03.2023 KR 20230033938
(71) Applicant: Neurotarge Co., Ltd., Seoul 02455 (KR)
(72) Inventor: KIM, Taeuk, Seoul 02792 (KR); LIM, Sang Min, Seoul 02792 (KR); CHA, Eunji, Seoul 02792 (KR); KANG, Jeong Wook, Seoul 02792 (KR); KIM, Yunjae, Seoul 02792 (KR); CHOI, In Young, Seoul 02792 (KR)
(74) Representative: Mewburn Ellis LLP
(86) International application number: PCT/KR2024/095516
(87) International publication number: WO 2024/191255

(57) **Abstract**

The present invention provides novel pyridoindole derivative compounds and compositions for the preventing or treating neurodegenerative diseases comprising the same as active ingredients. The present invention provides a fundamental solution to neurodegeneration of various tauopathies caused by Tau protein aggregation by significantly inhibiting the interaction between Tau protein and synaptogyrin-3, thereby moving beyond conventional symptomatic treatments that have focused on managing peripheral symptoms.

## Description

### Technical Field

The present invention relates to novel pyridoindole compounds that may be used as therapeutic agents for various tauopathies including neurodegenerative diseases, by inhibiting the interaction between the tau protein and synaptogyrin 3.

### Background Art

Microtubule is an important component of the cytoskeleton involved in a variety of intracellular processes, including mitosis, cytokinesis, and vesicular transport. Tau protein is a microtubule-associated protein (MAP) that interacts with microtubules to stabilize them and induce their formation. Tau is present in various cells and tissues, but is particularly abundant in neurons. Since tau plays a role in stabilizing microtubules, changes in tau expression, activity, and function may affect a variety of intracellular processes and contribute to numerous neurodegenerative diseases, including tauopathies.

Tauopathy is a subset of proteinopathies and includes a variety of neurodegenerative disorders, where Tau protein aggregates due to hyperphosphorylation in the brain tissue. Tau protein's mis-localization to dendritic spines or interference with glutamate receptors has been reported to be the pathogenesis of tauopathies, but in addition to postsynaptic localization, pathological Tau is also present in the presynaptic compartment, therefore presynaptic Tau function is also predicted to contribute to the disease.

On the other hand, synaptogyrin-3, a transmembrane synaptic vesicle protein, mediates the binding between Tau and synaptic vesicles (Zhou et al, Nature Communications 8:15295 (2017)). Decrease of synaptogyrin-3 levels in neurons disrupts the binding of tau to synaptic vesicles, restoring tau-induced vesicle trafficking defects and neurotransmitter release (J. McInnes et al., Neuron 97(4):823-835 (2018); Largo-Barrientos et al., Neuron 109:767-777 (2021)) Inhibiting the expression or activity of synaptogyrin-3, or the interaction between tau and synaptogyrin-3, would be an efficient therapeutic strategy for tauopathies. Accordingly, there is a growing need for the discovery of such inhibitors.

Throughout the present specification, a number of publications and patent documents are referred to and cited. The disclosure of the cited publications and patent documents is incorporated herein by reference in its entirety to more clearly describe the state of the art to which the present invention pertains and the content of the present invention.

### DISCLOSURE

### Technical Problem

The present inventors have made intensive studies to search an effective therapeutic composition that fundamentally eliminates the pathogenesis of various neurodegenerative diseases caused by hyperphosphorylation and aggregation of Tau protein. As a result, the present inventors have found that the pyridoindole derivatives of Formula 1 described below significantly inhibit the interaction between Tau protein and synaptogyrin-3, thereby blocking the binding of Tau protein to synaptic vesicle and effectively restoring neurotransmitter release, leading to the completion of the present invention.

Accordingly, it is an object of the present invention to provide a novel pyridoindole derivative and a composition for the preventing or treating neurodegenerative disease comprising the same as an active ingredient.

Other objects and advantages of the present invention will become more apparent from the following detailed description, the appended claims, and the accompanying drawings.

### Technical Solution

In one aspect of this invention, there is provided a compound represented by the following Formula 1: 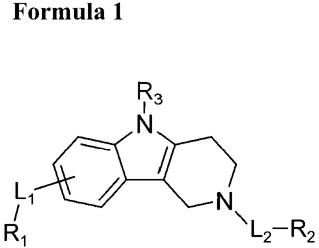 wherein,
R₁ is C₁-C₃ alkyl; or C₆-C₁₀ aryl or 5 to 12-membered heteroaryl unsubstituted or substituted with C₁-C₃ alkyl, C₃-C₇ cycloalkyl, C₁-C₃ alkoxy, halogen, -CN, -NO₂ or -NR₃ R₄ (where R₃ and R₄ are each independently hydrogen or C₁-C₃alkyl);
R₂ is C₆-C₁₀ aryl, 5 to 12-membered heteroaryl, C₃-C₇ cycloalkyl or 6-membered heterocycloalkyl unsubstituted or substituted with C₁-C₃ alkyl, C₁-C₃ haloalkyl, C₁-C₃ alkoxy, halogen, -CN, -NO₂ or -NR₅ R₆ (where R₅ and R₆ are each independently hydrogen or C₁-C₃ alkyl);
R₃ is hydrogen or C₁-C₃ alkyl;
L₁ is a direct bond or -NH-;
L₂ is a direct bond, -C(O)-, -C(S)-, -C(O)NH-, -C(S)NH- or -S(O₂)-.

The present inventors have made intensive studies to search an effective therapeutic composition that fundamentally eliminates the pathogenesis of various neurodegenerative diseases caused by hyperphosphorylation and aggregation of Tau protein. As a result, the present inventors have found that the pyridoindole derivatives of above Formula 1 significantly inhibit the interaction between Tau protein and synaptogyrin-3, thereby blocking the binding of Tau protein to synaptic vesicle mediated by this interaction and efficiently restoring the neurotransmitter secretion.

As used herein, the term "alkyl" refers to a straight-chain or branched saturated hydrocarbon group, and includes, for example, methyl, ethyl, propyl, isopropyl, etc. The term "C₁-C₃ alkyl" refers to an alkyl group having an alkyl unit having 1 to 3 carbon atoms, and when the C₁-C₃ alkyl is substituted, the carbon atom number of the substituent is not included.

As used herein, the term "halogen" refers to an element of the halogen group and includes, for example, fluoro, chloro, bromine, and iodine.

As used herein, the term "aryl" refers to a monocyclic or polycyclic carbon ring that is wholly or partially unsaturated and has an aromaticity.

As used herein, the term "heteroaryl" refers to a heterocyclic aromatic group that contains oxygen, sulfur or nitrogen as a heteroatom in the ring. The number of heteroatoms is 1-3, more specifically 1-2. The term "5 to 12-membered heteroaryl" refers to a heteroaryl having 5 to 12 atoms forming the ring, including both carbon and hetero atoms, which may be a monocycle or a bicycle.

As used herein, the term "haloalkyl" refers to an alkyl group substituted with a halogen. For example, C₁-C₃ haloalkyl refers to an alkyl with 1 to 3 carbons in which at least one hydrogen is substituted with a halogen. According to a specific embodiment, the haloalkyl of the present invention is trihalomethyl, and more specifically trifluoromethyl.

As used herein, the term "alkoxy" refers to a radical formed by the removal of hydrogen from an alcohol. When C₁-C₃ alkoxy is substituted, the number of carbons in the substituent is not included.

According to a specific embodiment of the invention, R₁ is C₁-C₂ alkyl; or a phenyl or 5 to 9-membered heteroaryl unsubstituted or substituted with C₁-C₃ alkyl, C₅-C₆ cycloalkyl, C₁-C₃ alkoxy, halogen, -CN, -NO₂, -NH₂ or -N(CH₃)₂. More specifically, said heteroaryl is 6-membered heteroaryl, and most specifically, pyridine.

According to a specific embodiment of the invention, R₂ is phenyl, 5 to 9-membered heteroaryl, C₅-C₆ cycloalkyl or morpholine unsubstituted or substituted with C₁-C₃ alkyl, trihalomethyl, C₁-C₃ alkoxy, -N(CH₃)₂), -CN or halogen.

More specifically, said heteroaryl is selected from the group consisting of pyridine, pyrimidine, pyrazine, thiazole and indole.

More specifically, said C₅-C₆ cycloalkyl is cyclohexyl.

According to a specific embodiment of the invention, when L₂ is -C(O)NH- or - C(S)NH-, R₂ is phenyl unsubstituted or substituted with C₁-C₂ alkyl or -CN.

According to a specific embodiment of the invention, when L₁ is -NH-, R₁ is phenyl substituted with a halogen, more specifically chlorophenyl.

According to a specific embodiment of the invention, when L₂ is -S(O) ₂-, R₂ is 6-membered heteroaryl, more specifically a pyridine.

According to a specific embodiment of the invention, the compound represented by Formula 1 is selected from the group consisting of the compounds represented by following Formula 2 to 77:

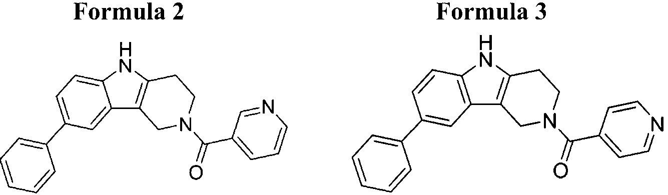

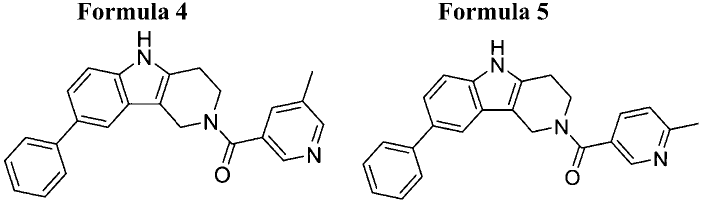

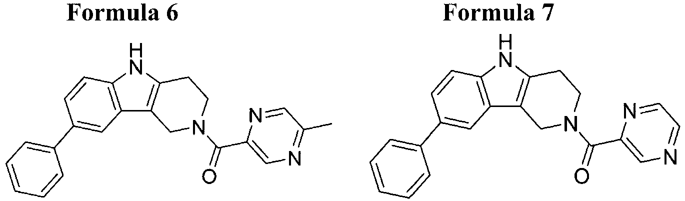

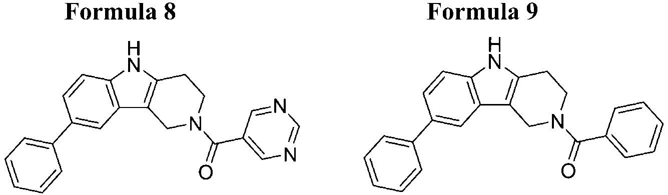

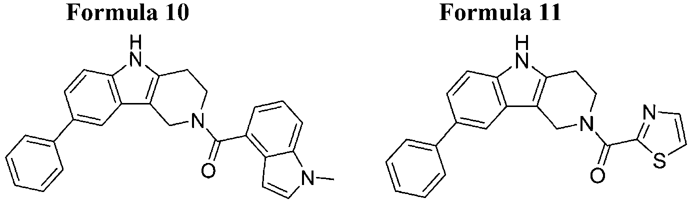

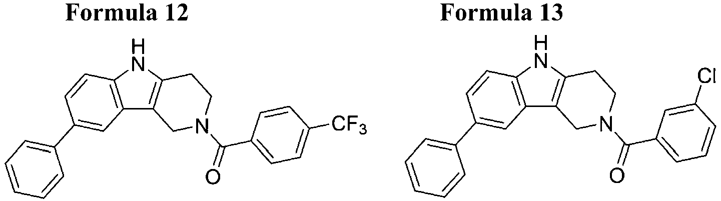

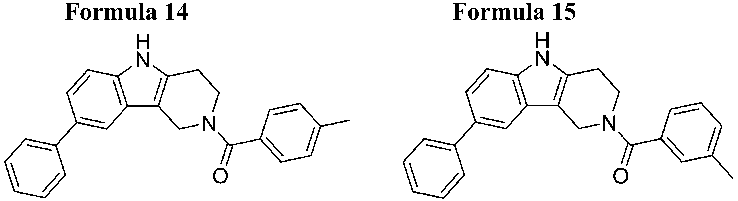

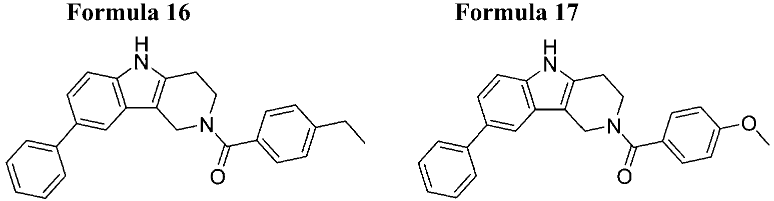

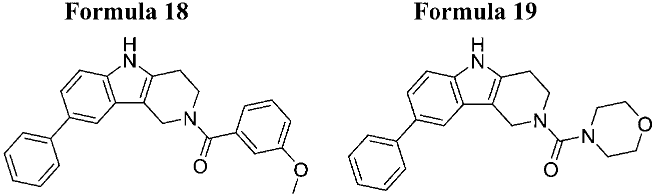

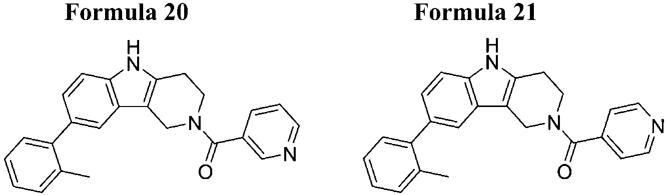

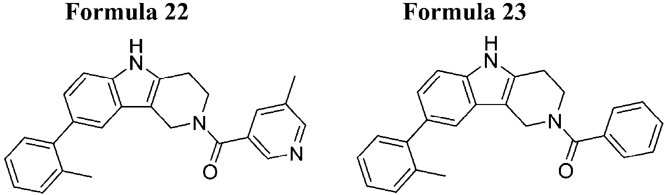

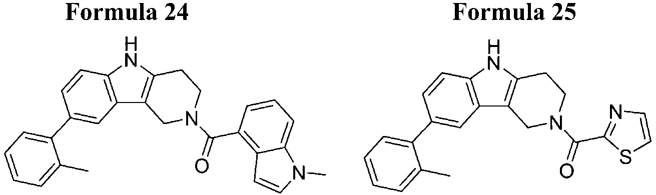

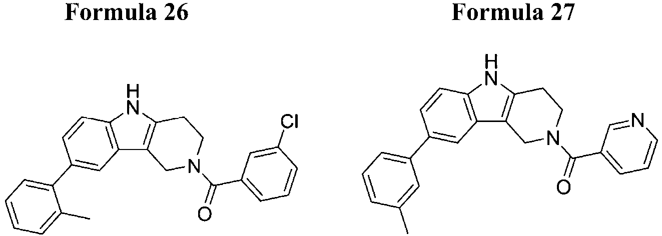

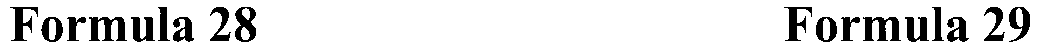

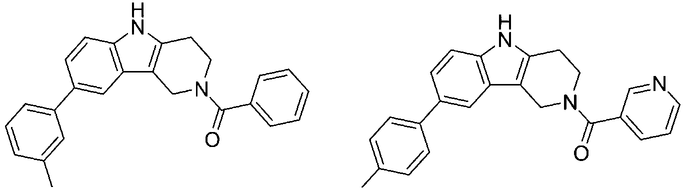

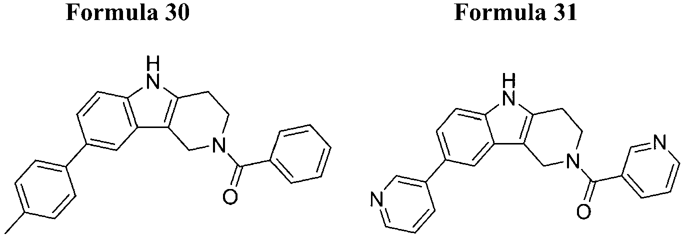

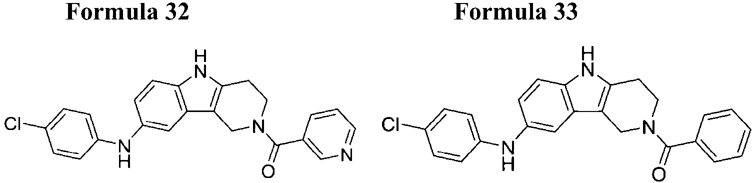

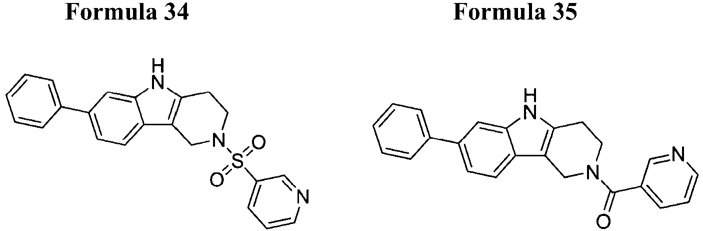

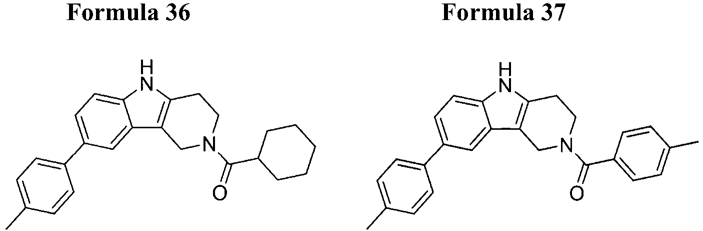

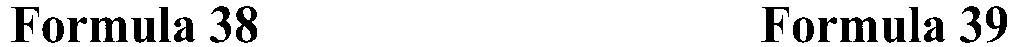

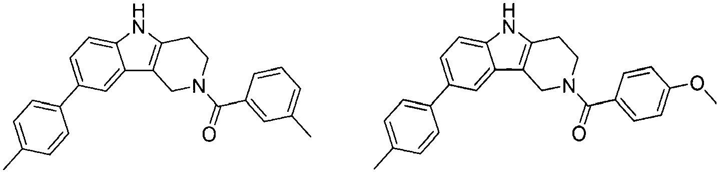

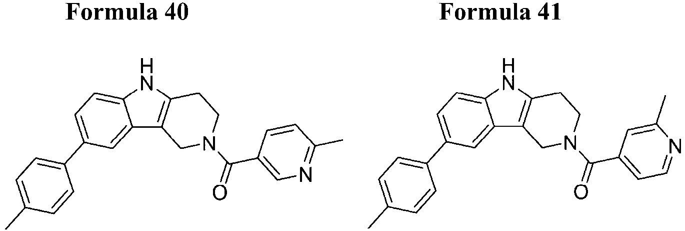

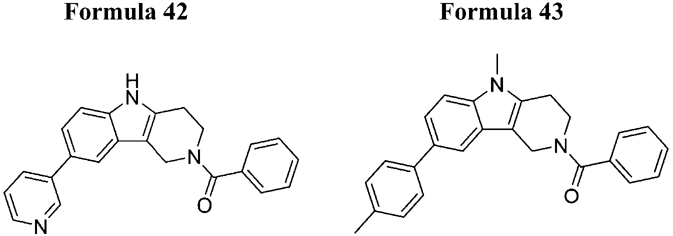

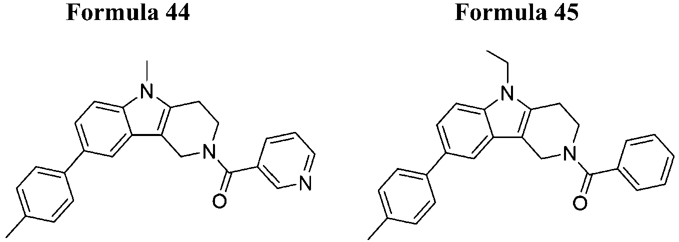

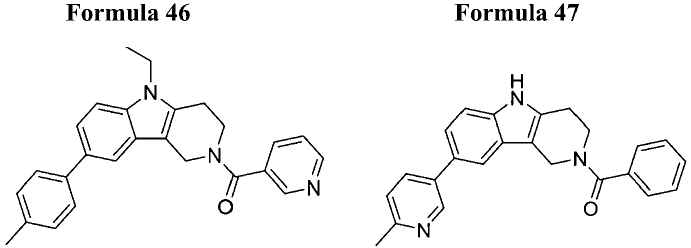

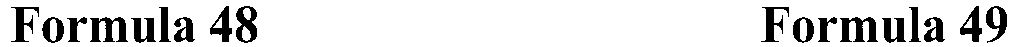

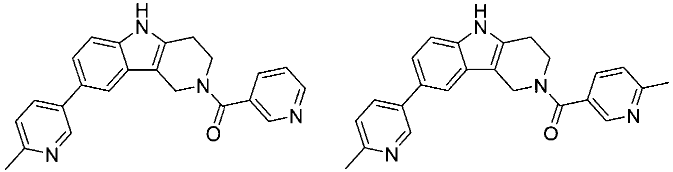

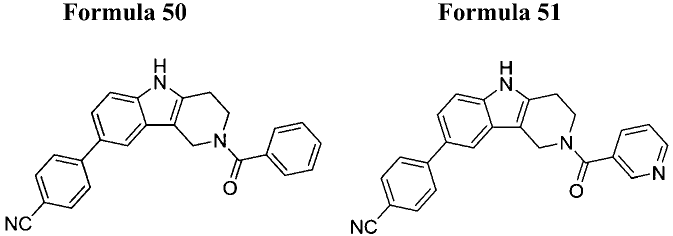

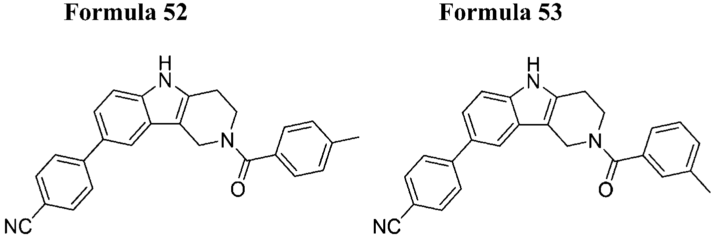

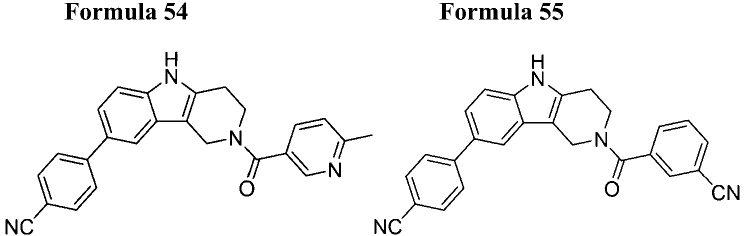

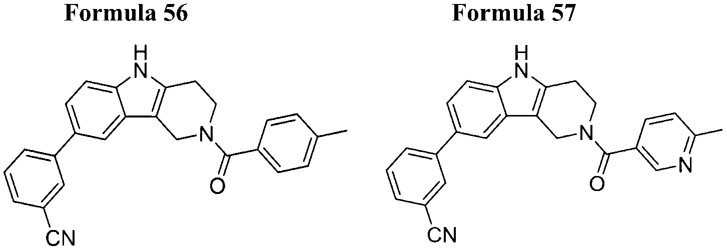

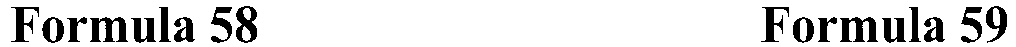

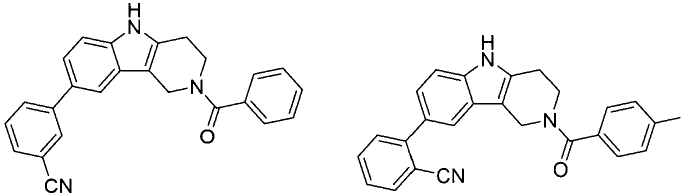

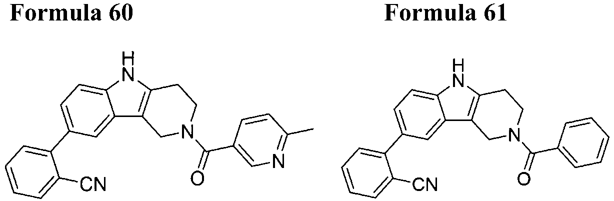

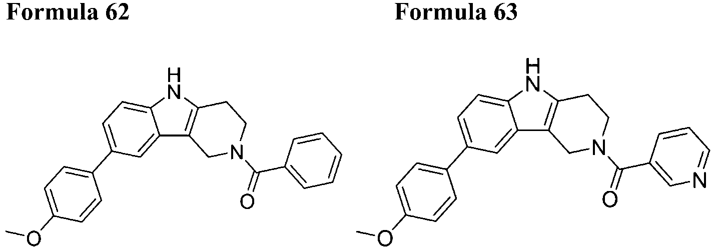

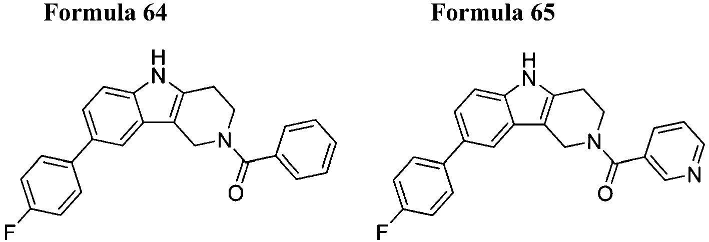

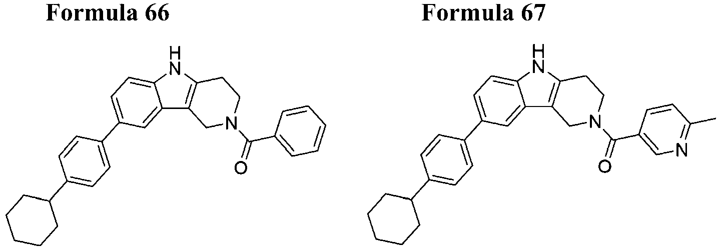

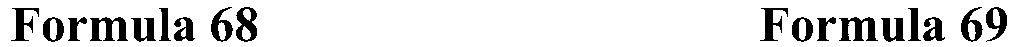

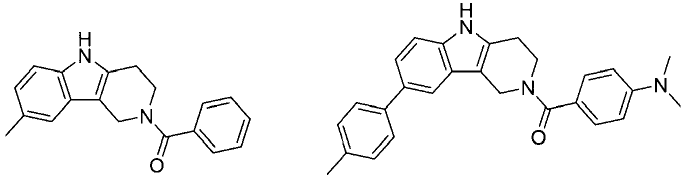

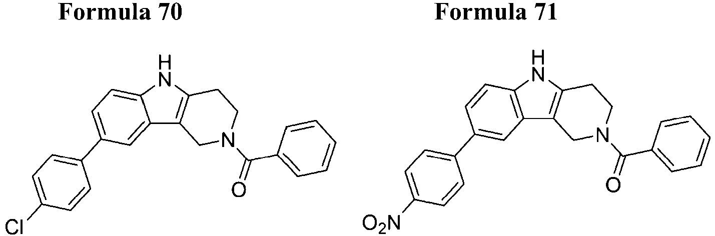

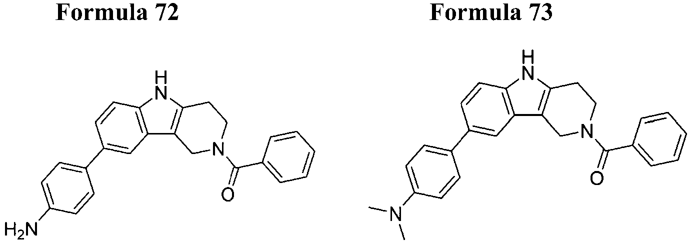

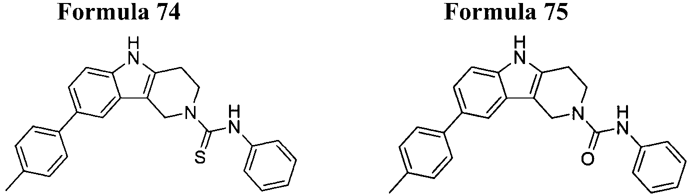

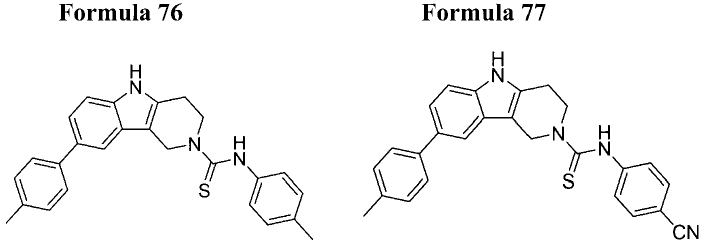

In another aspect of this invention, there is provided a composition for preventing or treating a neurodegenerative disease comprising a compound of the present invention described above or a pharmaceutically acceptable salt thereof, as an active ingredient.

In still another aspect of this invention, there is provided a method for preventing or treating a neurodegenerative disease comprising administering a compound of the present invention described above or a pharmaceutically acceptable salt thereof, to a subject in need thereof.

As used herein, the term "neurodegenerative disease" encompasses diseases characterized by structural and functional degeneration caused by the irreversible loss of brain tissue and the cells that constitute it. Specifically, the neurodegenerative diseases that may be prevented or treated using the composition of the present invention include tauopathy, which can be treated by inhibiting the binding of Tau protein to synaptic vesicles. As used herein, the term "tauopathy" refers to a disease or pathological condition caused by overexpression, hyperphosphorylation, aggregation, and/or accumulation of Tau protein in cells, tissues, or organs of the central nervous system.

According to a specific embodiment of the invention, the neurodegenerative disease that may be prevented or treated by the compositions of the present invention includes, but is not limited to, Alzheimer's disease, AGD (argyrophilic grain disease), Lewy body dementia, frontotemporal dementia, PSP (Progressive supranuclear palsy), PSP-P (progressive supranuclear palsy-Parkinsonism), Richardson's syndrome, Pick's disease, Niemann-Pick disease, Rasmussen's syndrome, Parkinson's disease, Atypical parkinsonism in Guadeloupe, FTDP-17 (Frontotemporal dementia with parkinsonism associated with chromosome 17), Progressive subcortical gliosis, Primary Progressive Aphasia, Globular gilal tauopathy, Lytico-Bodig disease, Neurodegeneration with brain iron accumulation, PKAN (Pantothenate kinase-associated neurodegeneration), Postencephalitic parkinsonism, CTE (chronic traumatic encephalopathy), Familial British dementia, Familial Danish dementia, Huntington's disease, Down's syndrome, Gerstmann-Straussler-Scheinker disease, Myotonic dystrophy, White matter tauopathy, ALS (Amyotrophic Lateral Sclerosis), Cerebral amyloid angiopathy, Senile dementia of the neurofibrillary tangle type, Motor neuron disease with neurofibrillary tangles, Diffuse neurofibrillary tangles with calcification, Corticobasal degeneration, Primary age-related tauopathy and Traumatic brain injury.

According to a specific embodiment of the invention, the composition of the present invention inhibits the interaction between tau and synaptogyrin 3 (Syngr3).

Syngr3 mediates the binding of Tau to synaptic vesicles. The compositions of the present invention significantly inhibit this interaction between Tau and Syngr3, and consequently block the binding of Tau to synaptic vesicles. As used herein, the term "inhibition of the interaction" refers to the measurable suppression, compared to a control group, of the binding between Tau and Syngr3 (or the mediation of binding between Tau and synaptic vesicles by synaptogyrin-3). Specifically, it refers to a level of inhibition sufficient to significantly restore vesicle motility defects and/or neurotransmitter release defects caused by Tau. More specifically, it refers to inhibition by 30% or more, even more specifically by 50% or more, and most specifically by 70% or more compared to the control group.

As used herein, the term "preventing" refers to inhibiting the occurrence of a disorder or disease in a subject who has never been diagnosed as having the disorder or disease, but is at risk of developing the condition or disease.

As used herein, the term "treating" refers to (a) inhibiting the progress of a disorder, disease or symptom; (b) alleviating the disorder, disease or symptom; or (c) eliminating the disorder, disease or symptom. When the composition of the present invention is administered to a subject, it functions to inhibit, eliminate or reduce the development of symptoms caused by hyperphosphorylation, aggregation, and excessive accumulation of Tau protein in the central nervous system by blocking the interaction between Tau and Syngr3 and inhibiting the binding of Tau protein to synaptic vesicles. Accordingly, the composition of the present invention may serve as a therapeutic composition for these diseases by itself, or may be administered in combination with other pharmacological ingredients and applied as a therapeutic aid for the diseases. Accordingly, as herein used, the term "treatment" or "therapeutic agent" is meant to encompass "treatment aid" or "therapeutic adjunct".

As used herein, the term "administer" refers to administration of a therapeutically effective amount of the composition of the present invention directly to a subject so that the same amount is formed in the subject's body.

As used herein, the term "therapeutically effective amount" refers to an amount of the composition sufficient to provide a therapeutic or prophylactic effect to a subject to whom/which the composition of the present invention is to be administered. Accordingly, the term "therapeutically effective amount" is meant to include a "prophylactically effective amount".

As used herein, the term "subject" includes, without limitation, humans, mice, rats, guinea pigs, dogs, cats, horses, cows, pigs, monkeys, chimpanzees, baboons or rhesus monkeys. Specifically, the subject of the present invention is a human.

As used herein, the term "pharmaceutically acceptable salt" includes a salt derived from a pharmaceutically acceptable inorganic acid, organic acid, or base. Examples of suitable acids include hydrochloric acid, hydrobromic acid, sulfuric acid, nitric acid, perchloric acid, fumaric acid, maleic acid, phosphoric acid, glycolic acid, lactic acid, salicylic acid, succinic acid, toluene-p-sulfonic acid, tartaric acid, acetic acid, trifluroacetic acid, citric acid, methanesulfonic acid, formic acid, benzoic acid, malonic acid, naphthalene-2-sulfonic acid, benzenesulfonic acid, and the like. Salts derived from suitable bases include salts of alkali metals such as sodium, alkaline earth metals such as magnesium, ammonium, and the like.

When the composition of the present invention is prepared as a pharmaceutical composition, the pharmaceutical composition of the present invention comprises a pharmaceutically acceptable carrier.

Examples of the pharmaceutically acceptable carrier that is comprised in the pharmaceutical composition of the present invention include, but are not limited to, lactose, dextrose, sucrose, sorbitol, mannitol, starch, gum acacia, calcium phosphate, alginate, gelatin, calcium silicate, microcrystalline cellulose, polyvinylpyrrolidone, cellulose, water, syrup, methyl cellulose, methylhydroxybenzoate, propylhydroxybenzoate, talc, magnesium stearate, and mineral oil, which are commonly used in formulation. The pharmaceutical composition of the present invention may further comprise a lubricant, a wetting agent, a sweetening agent, a flavoring agent, an emulsifying agent, a suspending agent, a preservative, and the like, in addition to the above-described ingredients. Suitable pharmaceutically acceptable carriers and agents are described in detail in Remington's Pharmaceutical Sciences (19^{th} ed., 1995).

The pharmaceutical composition of the present invention may be administered orally or parenterally. More specifically, it may be administered orally, intravenously or intraventricularly.

An appropriate dosage of the pharmaceutical composition of the present invention may vary depending on various factors such as formulation method, administration mode, patient's age, weight, sex, pathological condition, diet, administration time, administration route, excretion rate, and reaction sensitivity. A preferred dosage of the pharmaceutical composition of the present invention is within the range of 0.001 to 100 mg/kg for an adult.

The pharmaceutical composition of the present invention may be prepared in a unit dose form or prepared to be contained in a multi-dose container by formulating with a pharmaceutically acceptable carrier and/or excipient, according to a method that may be easily carried out by a person skilled in the art. In this case, the formulation of the pharmaceutical composition may be a solution, suspension, syrup or emulsion in oil or aqueous medium, or an extract, powder, granule, tablet or capsule, and may further comprise a dispersing agent or a stabilizer.

In still another aspect of this invention, there is provided a functional food composition for preventing or alleviating a neurodegenerative disease comprising a compound of the present invention described above or a sitologically acceptable salt thereof, as an active ingredient.

In still another aspect of this invention, there is provided a method for preventing or alleviating a neurodegenerative disease comprising administering a compound of the present invention described above or a sitologically acceptable salt thereof to a subject in need thereof.

The pyridoindole derivative of Formula 1 used in the present invention and the neurodegenerative diseases to be prevented or alleviated thereby have already been described above in detail and are therefore omitted to avoid undue redundancy.

As used herein, the term "sitologically acceptable salt" refers to any salt in which cations and anions bind by electrostatic attraction, in a form suitable for a food composition. Specific examples of food-acceptable salt would include the examples of "pharmaceutically acceptable salts" described above.

When the composition of the present invention is formulated as a food composition, it may include not only the compounds of the present invention as active ingredients, but also carbohydrates, seasonings and flavorings that are customarily added in the manufacture of food products. Examples of carbohydrates include, but are not limited to, monosaccharides such as glucose and fructose; disaccharides such as maltose and sucrose; polysaccharides such as dextrin and cyclodextrin; and sugar alcohols such as xylitol, sorbitol, erythritol. As flavoring agents, natural flavoring agents [such as thaumatin, stevia extract (e.g., rebaudioside A, glycyrrhizin, etc.)] and synthetic flavoring agents (such as saccharin, aspartame, etc.) can be used. For example, if the food composition of the present invention is formulated as a beverage, it may additionally include citric acid, liquid dextrose, sugar, glucose, acetic acid, malic acid, fruit juice, caterpillar extract, jujube extract, licorice extract, and the like, in addition to the active ingredient of the present invention, the pyridoindole compound of Formula 1.

### Advantageous Effects

The features and advantages of the present invention are summarized as follows:
(a) The present invention provides novel pyridoindole derivative compounds and compositions for the preventing or treating neurodegenerative diseases comprising the same as active ingredients.
(b) The present invention provides a fundamental solution to neurodegeneration of various tauopathies caused by Tau protein aggregation by significantly inhibiting the interaction between Tau protein and synaptogyrin-3, thereby moving beyond conventional symptomatic treatments that have focused on managing peripheral symptoms.

### Brief Description of Drawings

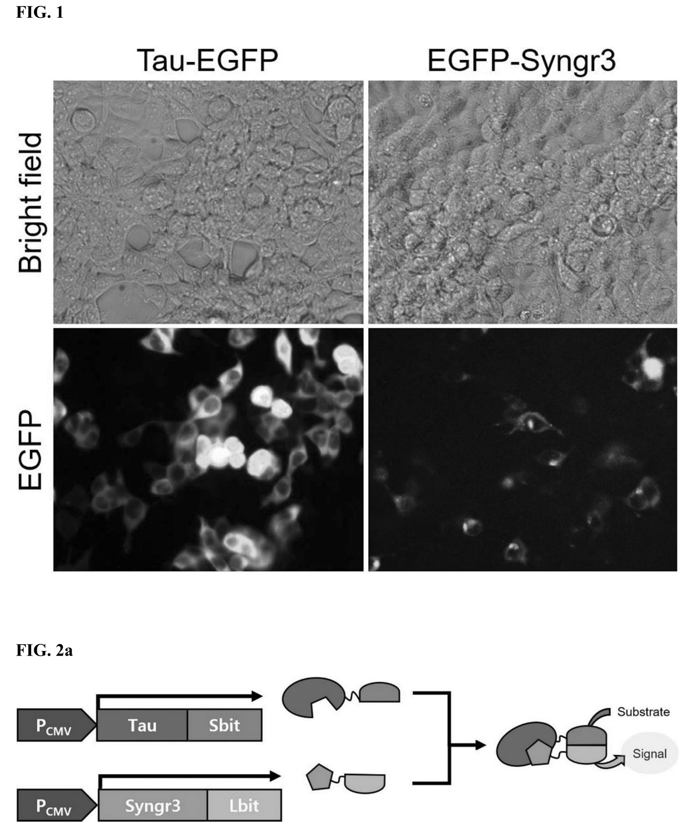

FIG. 1 is a fluorescence microscopy image of the HEK293T cell line transfected with EGFP-fused Tau (pTK231: P(CMV)-Tau-EGFP) and Synaptogyrin 3 (pTK233:P(CMV)-EGFP-Synaptogyrin 3), showing the expression of both proteins 48 hours after transfection.

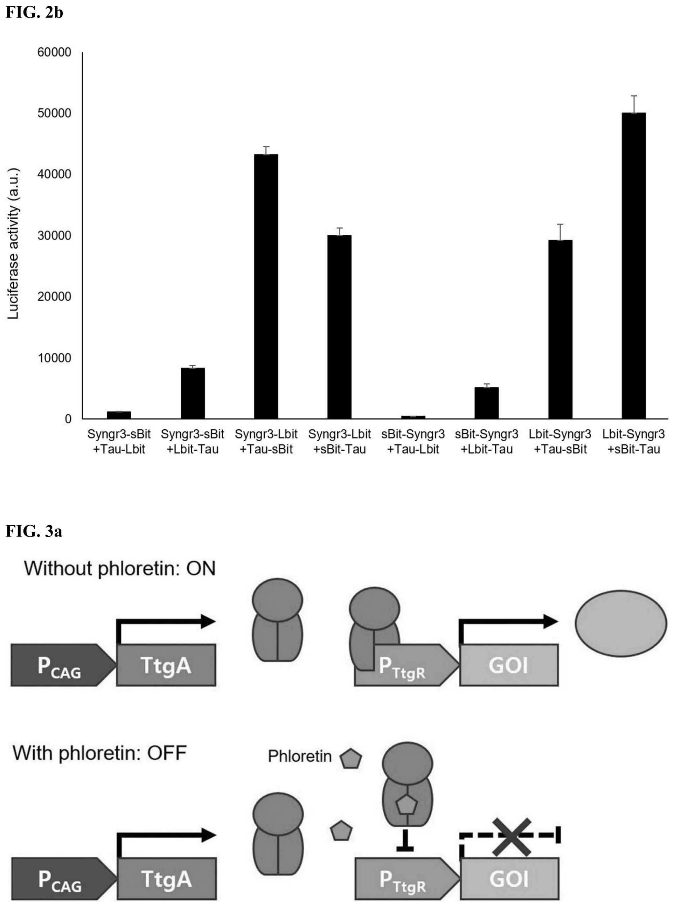

FIG. 2a is a schematic of the Protein Complementary Assay (PCA) process using the split luciferase method. Split luciferase consists of two fragments of the luciferase enzyme: a small bit (sBit) and a large bit (Lbit). These fragments are individually inactive under normal conditions, however, when brought into close proximity through the interaction of fused proteins, they reassemble to restore luciferase activity. Therefore, by fusing each fragment of the split luciferase to Tau and Synaptogyrin3, luciferase activity may be induced as a result of the interaction between Tau and Synaptogyrin3. FIG. 2b shows the results of co-transfection of all possible combinations of Tau and Synaptogyrin3, sBit and Lbit fusion proteins into HEK293T cells, followed by measurement of luciferase activity 48 hours later.

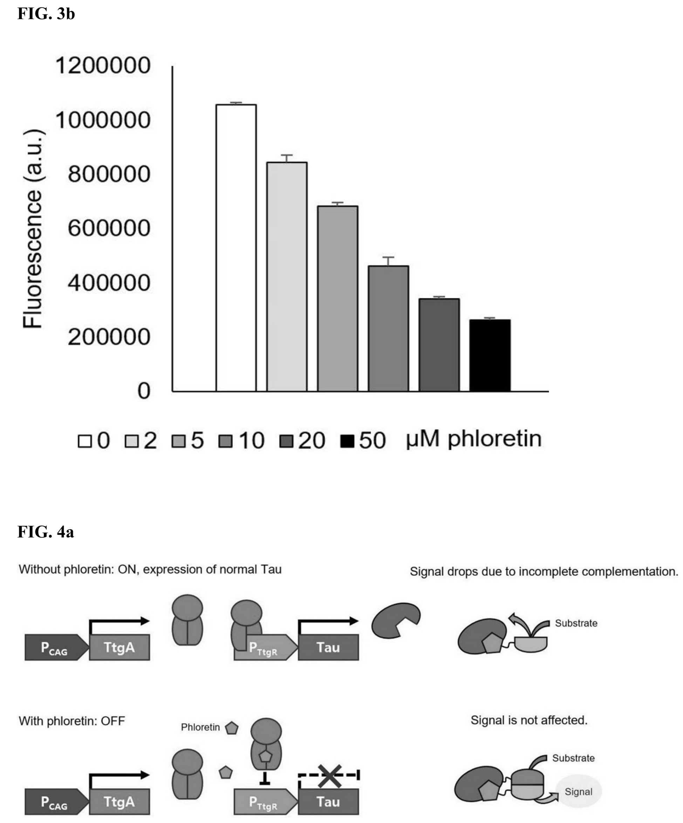

FIG. 3A is a schematic of a phloretin-regulated gene expression control system. The system includes a synthetic transcriptional activator, TtgA, and a synthetic promoter, P_{TtgR}, prepared based on the flavonoid-regulated TtgR operon of the Pseudomonas putida DOT-T1E strain. In the absence of phloretin, TtgA binds to P_{TtgR} and activates transcription (without phloretin), while in the presence of phloretin, TtgA dissociates from DNA and transcription is not activated (with phloretin). FIG. 3b shows the results of co-expressing phloretin-regulated EGFP expression plasmids (pTK269:P_{CAG}-TtgA and pTK274: P_{TtgR}-EGFP) in HEK293T and measuring EGFP values 48 hours after treatment with various concentrations of phloretin (0-50µM).

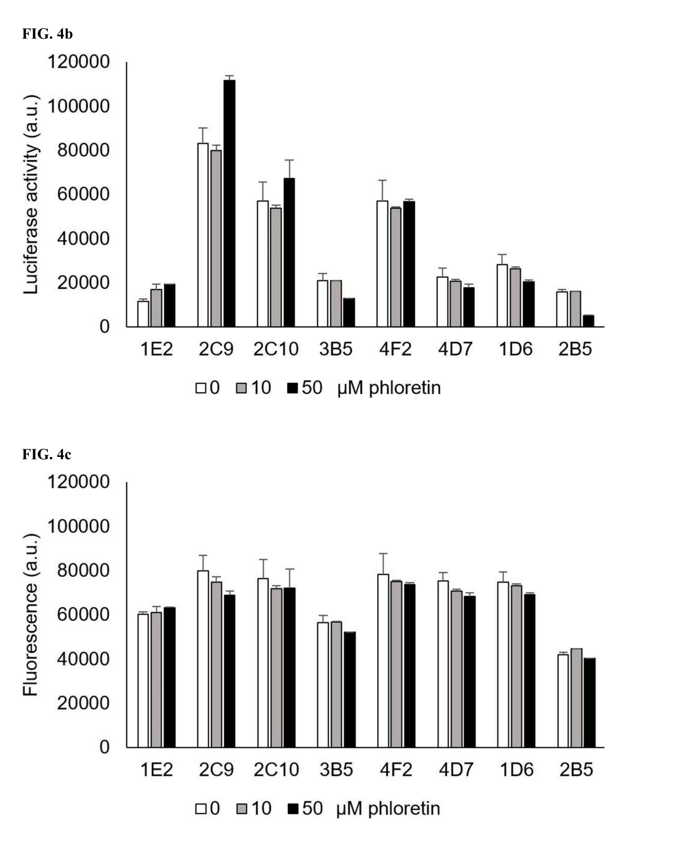

FIG. 4a is a schematic of a Tau competition assay using a phloretin-regulated gene expression system. In the stable cell line HEK293T-TK252 expressing Lbit-Tau and sBit-Synaptogyrin3, expression of Tau protein to which split luciferase is not fused, can lead to a decrease in luciferase signal. This occurs because the unfused Tau competes with Lbit-Tau for binding to sBit-Synaptogyrin3, thereby preventing the interaction of the split luciferase fragments and disrupting the Tau-Synaptogyrin3 interaction. Tau expression plasmids (pTK269: P_{CAG}-TtgA and pTK285:P_{TtgR}-Tau) were co-transfected into HEK293T-TK252 clones and treated with various concentrations (0, 10 and 50 µM) of phloretin. FIG. 4b shows the luciferase activity of each clone after 48 hours. FIG. 4c represents EGFP measurements after 48 hours for each clone.

### Mode for Invention

Hereinafter, the present invention will be described in more detail by way of examples. These examples are only for illustrating the present invention in more detail, and it will be apparent to those skilled in the art that the scope of the present invention according to the subject matter of the present invention is not limited by these examples.

### Examples

### Materials and methods

### Plasmid design

Comprehensive design and construction details of plasmids and oligonucleotides used are provided in Table 1 and Table 2, respectively.

**[Table 1]**

| Name | Description and cloning strategy | References |
|---|---|---|
| pcDNA3.1+ | Constitutive mammalian expression vector (BgIII-P_{CMV}-NheI-HindIII-KpnI-BamHI-EcoRI-EcoRV-NotI-XhoI-XbaI-ApaI). | Invitrogen |
| pCMV6-hTau-VC155 | Constitutive Tau-VC155 expression vector (P_{CMV}-Tau-VC155). | [1] |
| pGC551 | P_{GTA}-driven SEAP expression vector (P_{GTA}-SEAP). P_{GTA}-SEAP (BglII-P_{GTA}-NheI-HindIII-SEAP-BamHI-EcoRI) was synthesized (Genscript. NJ, USA) and restricted with BglII/EcoRI and inserted into corresponding sites of pcDNA3.1+. | [2], Present invention |
| pL48 | Constitutive expression vector (P_{CAG}-XbaI-XhoI-NotI-BglII). | Unpublished |
| pTK106 | Constitutive BMP2 expression, lentiviral vector (P_{EF1alpha}-XbaI-BMP2-EcoRI-IRES-NheI-EGFP-BamHI/P_{PGK}[SpeI]-puroR-XhoI). | Unpublished |
| pTK107 | Constitutive BMP2 expression, lentiviral vector (P_{PGK}-NheI-BamHI-BMP2-EcoRV-NotI-IRES-copGFP-T2A-puroR-EcoRI-SalI). | Unpublished |
| pTK201 | Constitutive Tau expression vector (P_{CMV}-Tau). Tau was PCR-amplified from pCMV6-hTau-VC155 using oligonucleotides oTK205/oTK206, and amplified DNA (KpnI-XbaI-Tau-BamHI-EcoRI-stop-NotI-ApaI) was restricted with KpnI/ApaI and inserted into corresponding sites of pcDNA3.1+. | Present invention |
| pTK202 | Constitutive Tau expression vector (P_{CMV}-Tau). Tau was PCR-amplified from pCMV6-hTau-VC155 using oligonucleotides oTK207/oTK208, and amplified DNA (KpnI-XbaI-XhoI-BamHI-Tau-stop-NotI-ApaI) was restricted with KpnI/ApaI and inserted into corresponding sites of pcDNA3.1+. | Present invention |
| pTK225 | Constitutive Tau-sBit expression vector (P_{CMV}-Tau-sBit). Tau was excised from pTK201 with XbaI/BamHI, and inserted into corresponding sites of pTK245. | Present invention |
| pTK226 | Constitutive Tau-Lbit expression vector (P_{CMV}-Tau-Lbit). Tau was excised from pTK201 with XbaI/BamHI, and inserted into corresponding sites of pTK246. | Present invention |
| pTK227 | Constitutive sBit-Tau expression vector (P_{CMV}-sBit-Tau). Tau was excised from pTK202 with BamHI/NotI, and inserted into corresponding sites of pTK247. | Present invention |
| pTK228 | Constitutive Lbit-Tau expression vector (P_{CMV}-Lbit-Tau). Tau was excised from pTK202 with BamHI/NotI, and inserted into corresponding sites of pTK248. | Present invention |
| pTK231 | Constitutive Tau-EGFP expression vector (P_{CMV}-Tau-EGFP). EGFP was PCR-amplified from pTK106 using oligonucleotides oTK237/oTK238, and amplified DNA (BamHI-XbaI-EGFP-EcoRI-BamHI) was restricted with BamHI/EcoRI and inserted into corresponding sites of pTK201. | Present invention |
| pTK233 | Constitutive EGFP-Synaptogyrin3 expression vector (P_{CMV}-EGFP-Synaptogyrin3). EGFP was PCR-amplified from pTK106 using oligonucleotides oTK237/oTK238, and amplified DNA (BamHI-XbaI-EGFP-EcoRI-BamHI) was restricted with XbaI/BamHI and inserted into corresponding sites of pTK248. | Present invention |
| pTK236 | Constitutive sBit-Tau expression, lentiviral vector (P_{EF1alpha}-sBit-Tau-IRES-EGFP/P_{PGK}[SpeI]-puroR-XhoI). sBit-Tau was PCR-amplified from pTK227 using oligonucleotides oTK243/pTK244, and amplified DNA (XbaI-sBit-Tau-stop-EcoRI) was restricted with Xbal/EcoRI and inserted into corresponding sites of pTK106. | Present invention |
| pTK238 | Constitutive Lbit-Synaptogyrin3, lentiviral expression vector (P_{PGK}[SpeI]-Lbit-Synaptogyrin3-IRES-copGFP-T2A-puroR-EcoRI-SalI). Lbit-Synaptogyrin3 was excised from pTK248 with NheI/NotI, inserted into corresponding sites of pTK107. | Present invention |
| pTK245 | Constitutive Synaptogyrin3-sBit expression vector (P_{CMV}-Synaptogyrin3-sBit). Synaptogyrin3-sBit (XbaI-Synaptogyrin3-BamHI-linker-sBit-stop-EcoRI) was synthesized (Cosmogenetech. Seoul, Korea) and restricted with Xbal/EcoRI and inserted into corresponding sites of pTK201. | Present invention |
| pTK246 | Constitutive Synaptogyrin3-Lbit expression vector (P_{CMV}-Synaptogyrin3-Lbit). Synaptogyrin3-Lbit (XbaI-Synaptogyrin3-BamHI-linker-Lbit-stop-EcoRI) was synthesized (Cosmogenetech. Seoul, Korea) and restricted with Xbal/EcoRI and inserted into corresponding sites of pTK201. | Present invention |
| pTK247 | Constitutive sBit-Synaptogyrin3 expression vector (P_{CMV}-sBit-Synaptogyrin3). sBit-Synaptogyrin3 (XhoI-sBit-linker-BamHI-Synpatogyrin3-stop-NotI) was synthesized (Cosmogenetech. Seoul, Korea) and restricted with XhoI/NotI and inserted into corresponding sites of pTK202. | Present invention |
| pTK248 | Constitutive Lbit-Synaptogytin3 expression vector (P_{CMV}-Lbit-Synaptogytin3). Lbit-Synaptogyrin3 (XhoI-Lbit-linker-BamHI-Synpatogyrin3-stop-NotI) was synthesized (Cosmogenetech. Seoul, Korea) and restricted with XhoI/NotI and inserted into corresponding sites of pTK202. | Present invention |
| pTK252 | Constitutive sBit-Tau and Lbit-Synpatogyrin3 expression, lentiviral vector (P_{EF1alpha}-sBit-Tau-IRES-EGFP/P_{PGK}-Lbit-Synaptogyrin3-IRES-copGFP-T2A-puroR). P_{PGK}-Lbit-Synaptogyrin3-IRES-copGFP-T2A-puroR was excised from pTK238 with SpeI/SalI and inserted into compatible sites (SpeI/XhoI) of pTK236. | Present invention |
| pTK263 | P_{ttg}-driven SEAP expression vector (P_{ttg}-SEAP). P_{ttg} was PCR-amplified from pGC551 using oligonucleotides oTK267/oTK268, and amplified DNA (BgIII-P_{ttg}-XhoI-HindIII-BamHI-stop-EcoRI-stop-XbaI) was restricted with BglII/HindIII and inserted into corresponding sites of pGC551. | [3], Present invention |
| pTK269 | Constitutive TtgA expression vector (P_{CAG}-TtgA). TtgA (KpnI-XhoI-TtgA-stop-BamHI) was synthesized (Cosmogenetech. Seoul, Korea) and restricted with XhoI/BamHI, and inserted into compatible sites (Xhol/BglII) of pL48. | [3], Present invention |
| pTK273 | P_{TtgR}-driven expression vector (P_{TtgR}-MCS). P_{ttg} was amplified from pGC551 using oligonucleotides oTK267/oTK268, and amplified DNA (BglII-P_{TtgR}-XhoI-HindIII-BamHI-stop-EcoRI-stop-XbaI) restricted with BglII/XbaI and inserted into corresponding sites of pGC551. | [3], Present invention |
| pTK274 | P_{TtgR}-driven EGFP expression vector (P_{TtgR}-EGFP). EGFP was excised from pTK258 (p P_{EF1alpha}-KpnI-XhoI-Tau[0N4R]-BamHI-XbaI-EGFP-EcoRI) with BamHI-EcoRI, and inserted into corresponding sites of pTK273. | [3], Present invention |
| pTK285 | P_{TtgR}-driven Tau expression vector (P_{TtgR}-Tau[1N4R]). Tau[1N4R] was synthesized by assembly PCR using pTK201 and oligonucleotides oTK251/oTK252/oTK253/oTK254, and synthesized DNA (KpnI-XhoI-Tau[1N4R]-BamHI-EcoRI) was restricted with XhoI/BamHI and inserted into corresponding site of pTK273. | Present invention |

**GTA:** cyclic-GMP-responsive transcription activator, **IRES:** Internal ribosome entry site, **Lbit:** Large Bit (Large fragment of split luciferase), **MCS:** Multiple cloning site, **P_{CMV}:** Synthetic mammalian promoter with cytomegalovirus early enhancer and promoter of chicken beta actin gene, **P_{CMV}:** Mammalian promoter from human cytomegalovirus, **P_{EF1alpha}:** Mammalian promoter from human elongation factor 1 alpha, **P_{GTA}:** GTA-driven cyclic-GMP-responsive promoter (GTA-specific operator site and P_{CMV} minimal promoter), **P_{PGK}:** Mammalian promoter from phosphoglycerate kinase gene, **P_{PGK}[SpeI]:** To display a SpeI restriction site in P_{PGK} promoter, **P_{TtgR}:** TtgA-driven phloretin-responsive promoter (TtgA-specific operator site and P_{CMV} minimal promoter), **puroR:** Puromycin-resistance Gene, **sBit:** Small Bit (Small fragment of split luciferase), **Tau:** Microtubule-associated protein tau 2N4R isoform (UniProt P10636-8 [Tau-F], GenBank NM_005910), **Tau[0N4R]:** Microtubule-associated protein tau 0N4R isoform (UniProt P10636-6 [Tau-D]), **Tau[1N4R]:** Microtubule-associated protein tau 1N4R isoform (UniProt P10636-7 [Tau-E]), **TtgA:** Phloretin-responsive transcription activator (VP16-fused *Peudomonas putida* TtgR), **VC155:** Fluorescent protein Venus fragment for BiFC (Bimolecular fluorescence complementation)

**[Table 2]**

| Name | Sequence |
|---|---|
| oTK205 | atgaggtacctcttctagagccaccatggctgagccccgccag |
| oTK206 | ttatgggccctgcggccgcacttcagaattcacctgcggatcccaaaccctgcttggccag |
| oTK207 | atgaggtacctcttctagagacatcctcgagaatgtaggatccgccaccatggctgagccccgccag |
| oTK208 | ttatgggccctgcggccgcacttcacaaaccctgcttggccag |
| oTK237 | aataggatcctcaggcagctctagagccaccatggtgagcaagggcgagga |
| oTK238 | ataaggatccgcttccgctgaattccttgtacagctcgtccatgcc |
| oTK243 | aattctagagccaccatggtgaccggctatcgcct |
| oTK244 | attattgaattcctacaaaccctgcttggccaggg |
| oTK251 | aatggtacctccgcactcgaggccaccatggctgagccccgccaggag |
| oTK252 | gccgattccggcctcctcggcttccgctgttggagtgctctt |
| oTK253 | gccgaggaggccggaatcggcgacacccccagcctggaagac |
| oTK254 | aatagaattcggcagcggatcccaaaccctgcttggccaggga |
| oTK267 | aagagatctcagtatttacaaacaaccatgaatgtaagtatattccgtcgacgtcgagctcggtacccgggtc |
| oTK268 | aatctagagctttagaattcgctttaggatccgctaagcttgctgctctcgaggctccctatagtgagtcg |

### Cell culture and transfection

Human embryonic kidney cells (HEK293T, ATCC: CRL-11268) were cultivated with OptiMEM (Cat# 51985091/Gibco. ThermoFisher Korea, Seoul, Korea) supplemented with 8% (v/v) fetal bovine serum (FBS, US origin Cat# 16140-089, Lot# 2394274P/Gibco. ThermoFisher Korea, Seoul, Korea) at 37°C in a humidified CO₂ (5%) incubator. 10⁶ cells were seeded with 10 ml culture media in a 100 mm culture dish 18 hours prior to transfection. 10 µg of DNA was added into 2 ml OptiMEM, then 30 µg of PEI (1 µg/µl in DW, pH 7.4. Cat# 23966-100. Polyscience, PA, USA) was added and vortexed immediately for 3 seconds. After incubation at room temperature for 30 minutes, the DNA-PEI mixture was added to the cells. When a different type of cell culture plates was used, cell number, DNA and PEI amount was adjusted to the culture volume of the plate.

### Measurements

SEAP: Secreted alkaline phosphatase (SEAP) production in the cell culture media was quantified by a light absorbance assay using 4-nitrophenylphosphate (pNpp. CarboSynth, Berkshire, UK) [1].

Live cell luciferase assay: Live cell luciferase activity was measured using NanoGlo^{®} Live Cell Assay System (Cat# N2012. Promega, WI, USA), according to manufacturer's instruction. All microplate-based measurement was performed with BioTek Synergy H1 (Agilent. CA, USA).

### Cell line

For producing HEK293T-TK252 stable cell line for screening, BglII-linearized pTK252 was transfected to HEK293T cells. After 24 hrs transfection, the transfected cells were cultured in the culture media containing 1 µg/ml puromycin (10 mM in DMSO, Cat# J61278/ Alfa Aesar. ThermoFisher Korea, Seoul, Korea) for 9 days. Regular media change was performed in every 3 days with fresh puromycin of indicating concentration during the whole selection period. The selected cells were trypsinized and diluted to 5 cells/ml and seeded to 4x 96 well cell culture plates (100 µl/well), and cultured with the cell culture media containing 1 µg/ml puromycin for 11 days. 18 wells that contained only GFP-positive cells were selected for expansion, and cultured with the cell culture media containing 0.1-0.2 µg/ml puromycin for 14 days. 10 clones were discarded during the expansion due to emerging of non-GFP cells, and 8 clones were validated. The selected clone (1E2) was cultured with the cell culture media containing 0.5 µg/ml puromycin for further experiments.

### Library and screening

3 µmol of 1,728 compounds were purchased from Otava Chemicals MB (Vilniaus, Lithuania), dissolved into 300 µl DMSO (10 mM) and stored at -80°C. 4x10⁴ HEK293T-TK252-1E2 cells were seeded with 100 µl of the cell culture media into 96 white well cell culture plates (Cat# 30196. SPL, Seoul, Korea), 48 hrs prior to compound treatment. For 10 µM compound treatment, 10 mM compound stock was diluted to 50 µM with the cell culture media, and 25 µl of the diluted compound was added to the designated wells of the 96 well plates. For dose-response experiments, the amount of compounds for dilution was adjusted to fit the volume indicated above. After 48 hrs compound treatment, the cells were washed once with OptiMEM and live cell luciferase activity, fluorescent intensity and fluorescence area scan was measured.

**[Table 3]**

| Formula # | Structure | Formula # | Structure |
|---|---|---|---|
| 2 | 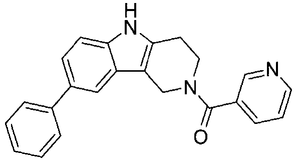 | 3 | 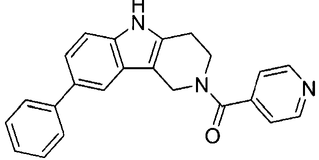 |
| 4 | 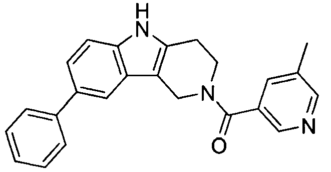 | 5 | 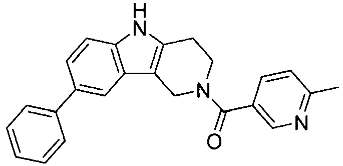 |
| 6 | 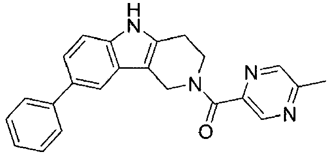 | 7 | 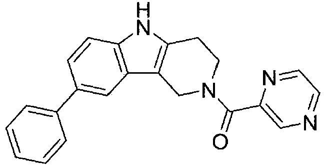 |
| 8 | 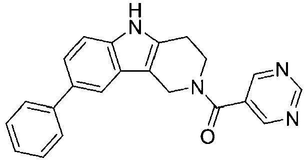 | 9 | 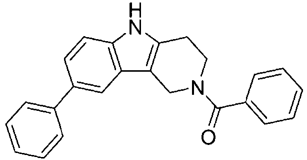 |
| 10 | 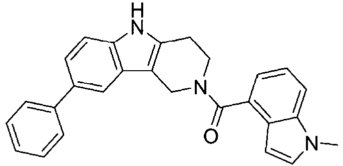 | 11 | 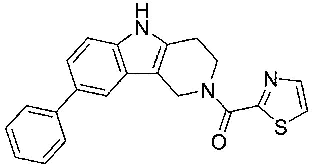 |
| 12 | 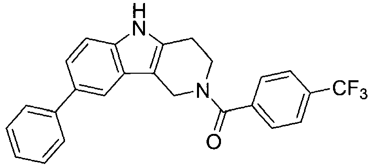 | 13 | 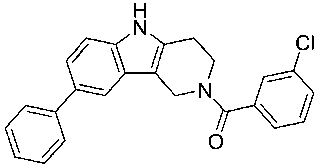 |
| 14 | 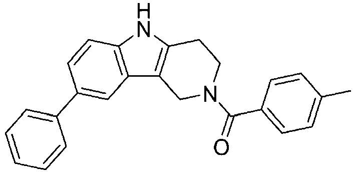 | 15 | 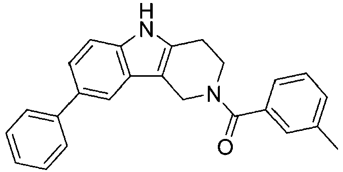 |
| 16 | 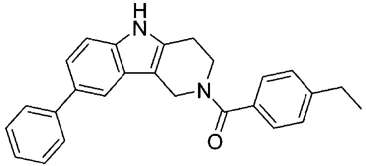 | 17 | 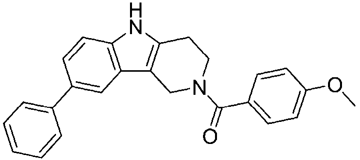 |
| 18 | 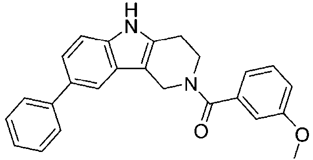 | 19 | 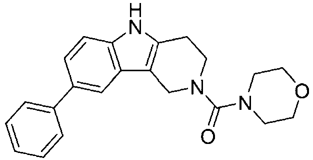 |
| 20 | 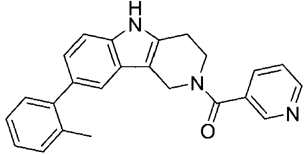 | 21 | 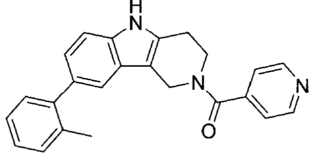 |
| 22 | 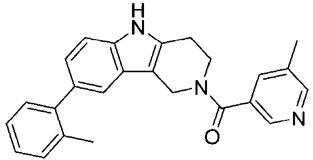 | 23 | 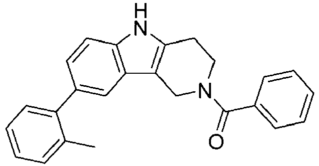 |
| 24 | 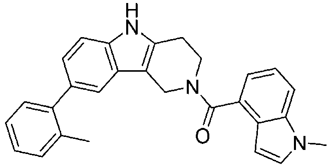 | 25 | 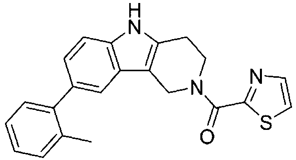 |
| 26 | 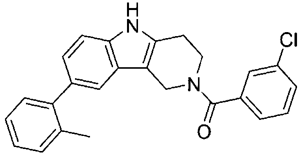 | 27 | 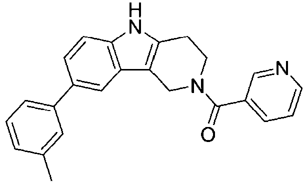 |
| 28 | 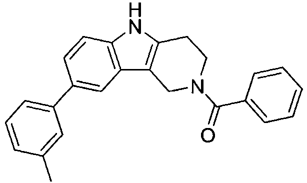 | 29 | 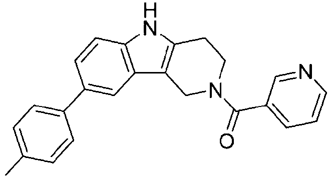 |
| 30 | 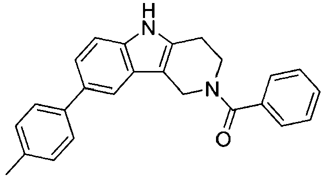 | 31 | 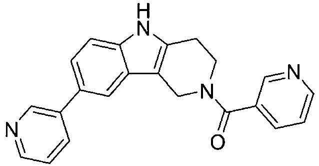 |
| 32 | 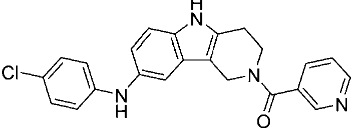 | 33 | 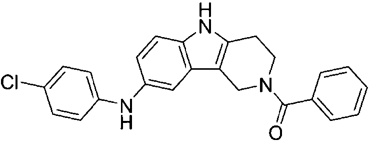 |
| 34 | 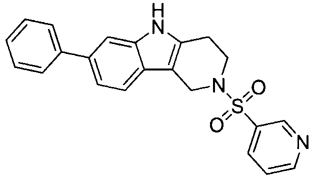 | 35 | 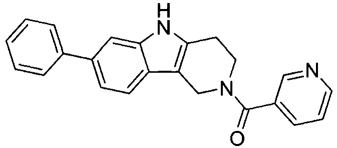 |
| 36 | 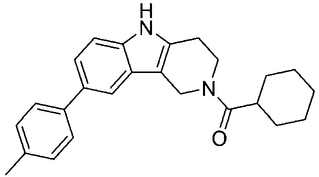 | 37 | 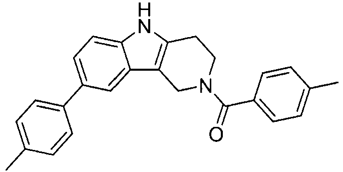 |
| 38 | 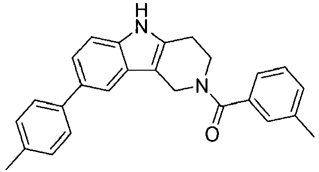 | 39 | 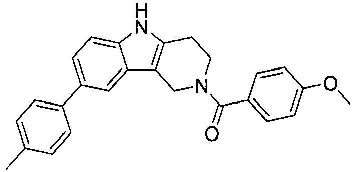 |
| 40 | 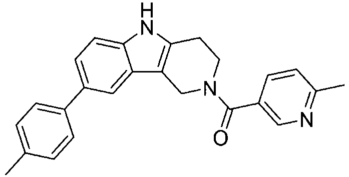 | 41 | 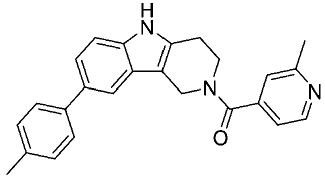 |
| 42 | 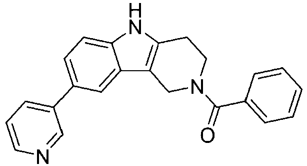 | 43 | 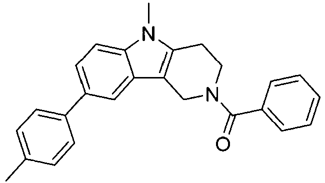 |
| 44 | 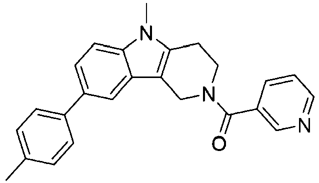 | 45 | 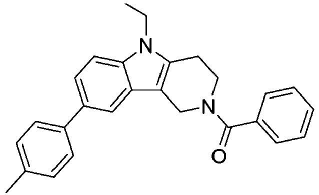 |
| 46 | 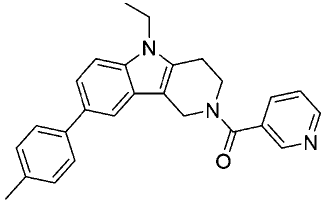 | 47 | 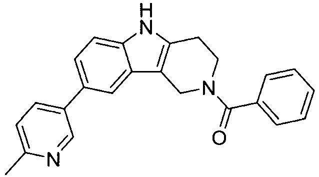 |
| 48 | 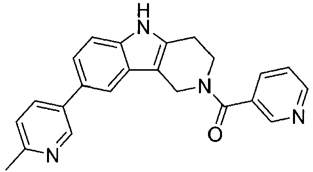 | 49 | 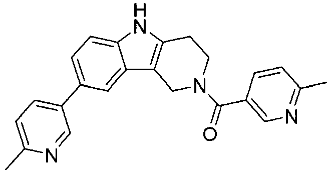 |
| 50 | 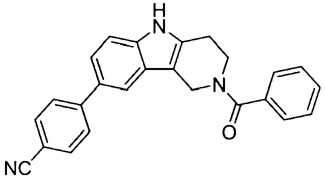 | 51 | 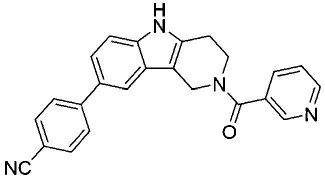 |
| 52 | 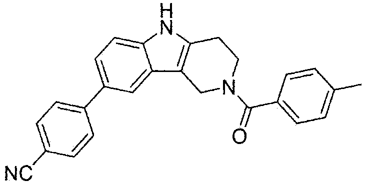 | 53 | 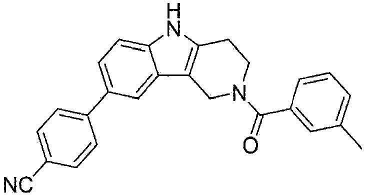 |
| 54 | 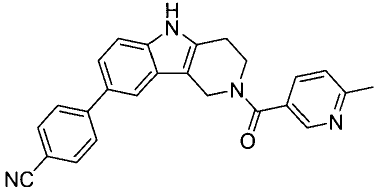 | 55 | 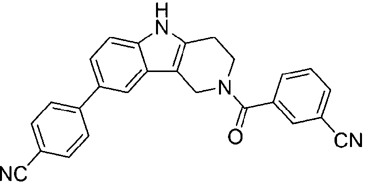 |
| 56 | 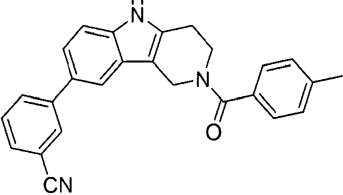 | 57 | 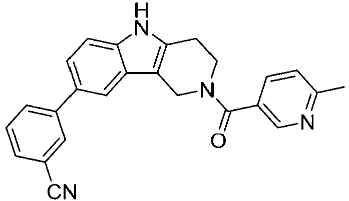 |
| 58 | 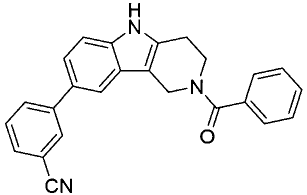 | 59 | 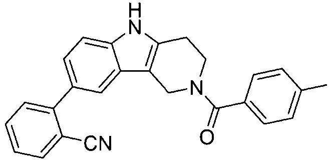 |
| 60 | 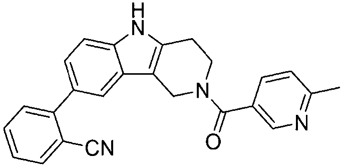 | 61 | 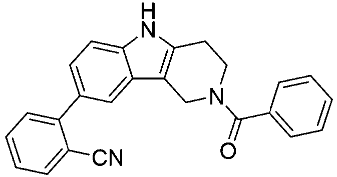 |
| 62 | 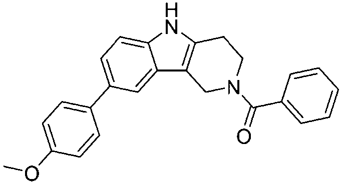 | 63 | 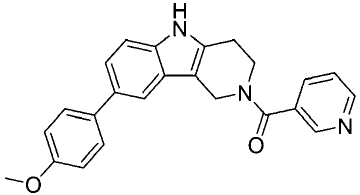 |
| 64 | 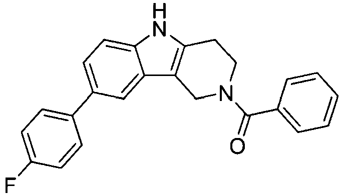 | 65 | 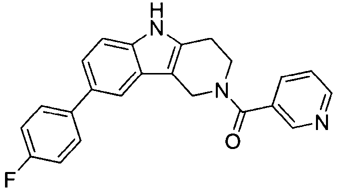 |
| 66 | 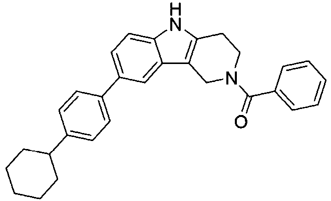 | 67 | 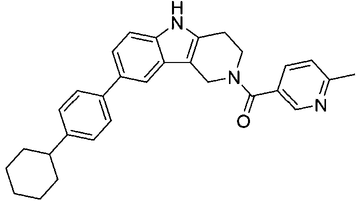 |
| 68 | 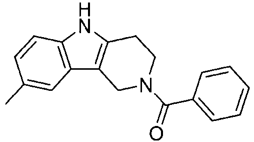 | 69 | 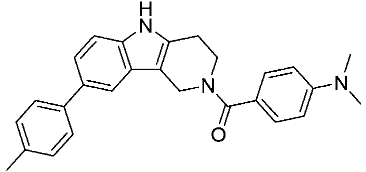 |
| 70 | 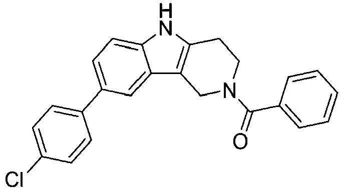 | 71 | 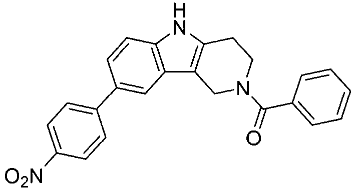 |
| 72 | 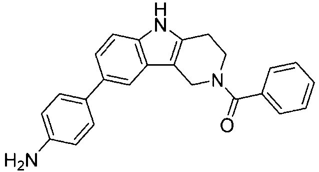 | 73 | 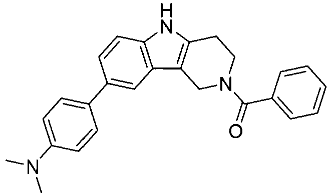 |
| 74 | 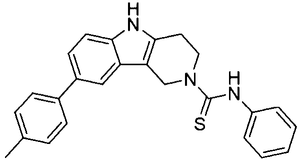 | 75 | 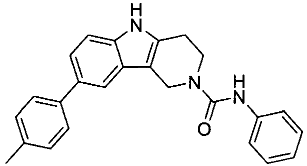 |
| 76 | 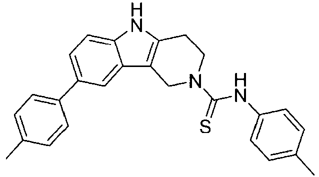 | 77 | 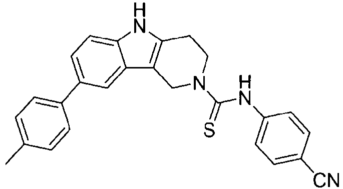 |

### Preparative Example 1: 8-phenyl-2,3,4,5-tetrahydro-1H-pyrido[4,3-b]indole hydrochloride (Intermediate)

### Preparative Example 1-1: 8-bromo-2,3,4,5-tetrahydro-1H-pyrido[4,3-b]indole (Intermediate 1-3)

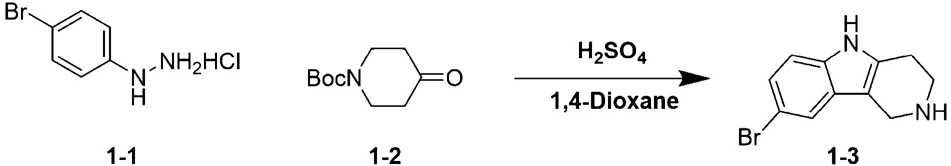

To a solution of (4-bromophenyl)hydrazine hydrochloride (1 g, 4.47 mmol) and *tert-*butyl 4-oxopiperidine-1-carboxylate (936 mg, 4.7 mmol) in 1,4-dioxane was added conc. H₂SO₄ (0.56 mL, 8 M) at 0 °C. Then the reaction mixture was heated at 110 °C for 3 hours using a sealed tube. After the reaction was completed, the precipitate was filtered with 1,4-dioxane. The precipitate was dissolved in distilled water and then basified with 1N sodium hydroxide solution. The mixture was extracted using distilled water and dichloromethane, and the organic layer was dried over anhydrous magnesium sulfate and filtered. The filtrate was concentrated under reduced pressure to obtain 584 mg of the title compound (yield: 65%).

¹H NMR (400 MHz, DMSO) δ 10.94 (s, 1H), 7.49 (d, J = 1.9 Hz, 1H), 7.23 (d, J = 8.5 Hz, 1H), 7.09 (dd, J = 8.5, 2.0 Hz, 1H), 3.82 (t, J = 1.6 Hz, 2H), 3.01 (t, J = 5.7 Hz, 2H), 2.67 (tt, J = 5.7, 1.6 Hz, 2H).

### Preparative Example 1-2: tert-butyl 8-bromo-1,3,4,5-tetrahydro-2H-pyrido[4,3-b]indole-2-carboxylate (Intermediate 1-4)

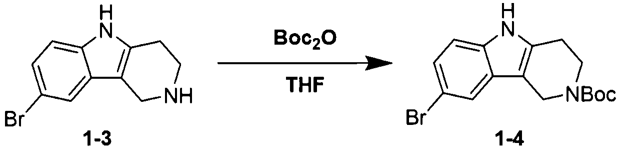

To a solution of 8-bromo-2,3,4,5-tetrahydro-1H-pyrido[4,3-b]indole (1.13 g, 4.51 mmol) in THF solution was added Boc₂O (1.14 mL, 4.96 mmol) and the mixture was stirred for 12 h under nitrogen. After the reaction was completed, the mixture was extracted using distilled water and ethyl acetate. The organic layer was dried over anhydrous magnesium sulfate and filtered. The filtrate was concentrated under reduced pressure, and the concentrate was subjected to column chromatography to obtain 1.02 g of the title compound (yield: 64%).

¹H NMR (400 MHz, DMSO) δ 11.14 (s, 1H), 7.60 (d, J = 1.9 Hz, 1H), 7.26 (d, J = 8.6 Hz, 1H), 7.15 (dd, J = 8.6, 2.0 Hz, 1H), 4.51 (s, 2H), 3.70 (t, J = 5.7 Hz, 2H), 2.78 (t, J = 5.8 Hz, 2H), 1.44 (s, 9H).

### Preparative Example 1-3: tert-butyl 8-phenyl-1,3,4,5-tetrahydro-2H-pyrido[4,3-b]indole-2-carboxylate (Intermediate 1-6)

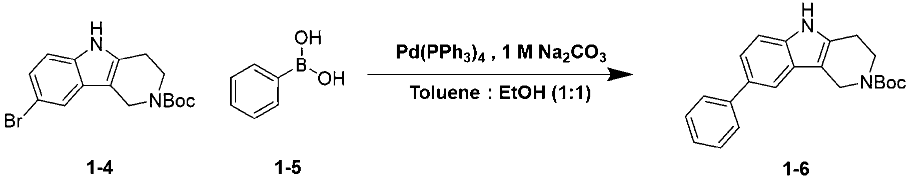

To a solution of *tert-*butyl 8-bromo-1,3,4,5-tetrahydro-2H-pyrido[4,3-b]indole-2-carboxylate (200 mg, 0.57 mmol), phenylboronic acid (208 mg, 1.71 mmol) and Pd(PPh₃)₄ (33 mg, 0.028 mmol) dissolved in Toluene : EtOH (1:1) solution was added 1M Na₂CO₃ (1.42 mL, 1.42 mmol). The reaction mixture was stirred at 100 °C for 24 hours using a sealed tube. After the reaction was completed, the mixture was extracted using distilled water and ethyl acetate. The organic layer was dried over anhydrous magnesium sulfate and filtered. The filtrate was concentrated under reduced pressure, and the concentrate was subjected to column chromatography to obtain 79 mg of the title compound (yield: 39%).

¹H NMR (400 MHz, DMSO) δ 10.93 (s, 1H), 7.67 - 7.60 (m, 3H), 7.39 (dd, J = 8.4, 7.1 Hz, 2H), 7.32 (t, J = 1.0 Hz, 2H), 7.29 - 7.20 (m, 1H), 4.54 (s, 2H), 3.68 (t, J = 5.7 Hz, 2H), 2.75 (t, J = 5.8 Hz, 2H), 1.41 (s, 9H).

### Preparative Example 1-4: 8-phenyl-2,3,4,5-tetrahydro-1H-pyrido[4,3-b]indole hydrochloride (Intermediate 1-7)

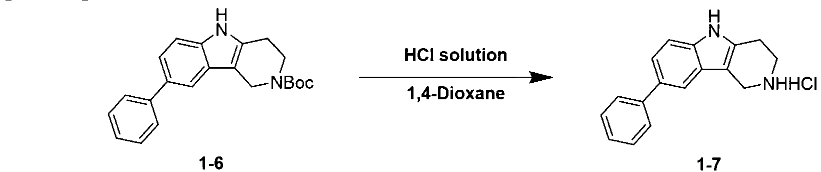

To a solution of *tert-*butyl 8-phenyl-1,3,4,5-tetrahydro-2H-pyrido[4,3-b]indole-2-carboxylate (78 mg, 0.22 mmol) in 1,4-dioxane was added HCl solution (4 M in dioxane) (1.12 mL, 4.48 mmol). Then the reaction was stirred at room temperature for 6 hours. After the reaction is complete, the solvent was removed under reduced pressure. The resulting solid was filtered with ethyl acetate to obtain 38 mg of the title compound (yield: 59%).

¹H NMR (400 MHz, DMSO) δ 11.25 (s, 1H), 7.77 (d, J = 1.4 Hz, 1H), 7.69 - 7.65 (m, 2H), 7.44 (d, J = 7.8 Hz, 4H), 7.32 - 7.26 (m, 1H), 4.34 (t, J = 4.3 Hz, 2H), 3.47 (d, J = 6.6 Hz, 2H), 3.04 (t, J = 6.1 Hz, 2H).

### Preparative Example 2: (8-phenyl-1,3,4,5-tetrahydro-2H-pyrido[4,3-b]indol-2-yl)(pyridin-3-yl)methanone (Compound 2)

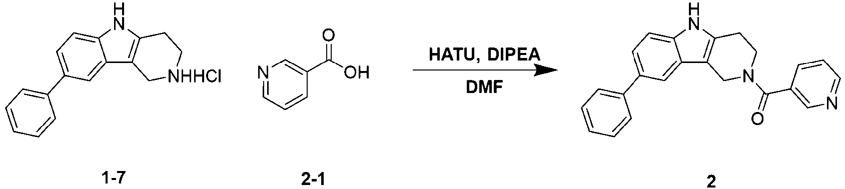

To a solution of 8-phenyl-2,3,4,5-tetrahydro-1H-pyrido[4,3-b]indole hydrochloride (22 mg, 0.077 mmol) in dimethylformamide under nitrogen was added nicotinic acid (11.41 mg, 0.093 mmol), HATU (58 mg, 0.15 mmol) and DIPEA (0.027 mL, 0.15 mmol). The reaction mixture was stirred at room temperature for 12 hours. After the completion of the reaction, the solvent was extracted using distilled water and ethyl acetate, and the organic layer was dried over anhydrous magnesium sulfate and filtered. The filtrate was concentrated under reduced pressure, and the concentrate was subjected to column chromatography to obtain 15.7 mg of the title compound (yield: 57%).

¹H NMR (400 MHz, DMSO) δ 11.02 (s, 1H), 8.67 (s, 2H), 7.95 - 7.20 (m, 12H), 4.86 (s, 1H), 4.65 (s, 1H), 4.03 (s, 1H), 3.66 (s, 1H), 2.89 (s, 2H).

### Preparative Example 3: 8-phenyl-1,3,4,5-tetrahydro-2H-pyrido[4,3-b]indol-2-yl)(pyridin-4-yl)methanone (Compound 3)

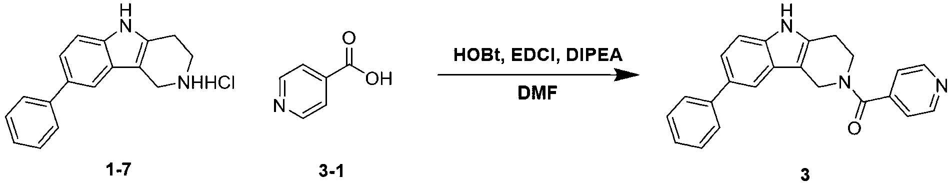

To a solution of 8-phenyl-2,3,4,5-tetrahydro-1H-pyrido[4,3-b]indole hydrochloride (20 mg, 0.07 mmol) in dimethylformamide under nitrogen was added isonicotinic acid (10.38 mg, 0.084 mmol), HOBt (18.98 mg, 0.14 mmol), EDCI (26.93 mg, 0.14 mmol) and DIPEA (0.049 mL, 0.28 mmol). The reaction mixture was stirred at room temperature for 12 hours. After the completion of the reaction, the solvent was extracted using distilled water and ethyl acetate, and the organic layer was dried over anhydrous magnesium sulfate and filtered. The filtrate was concentrated under reduced pressure, and the concentrate was subjected to column chromatography to obtain 5.6 mg of the title compound (yield: 22%).

¹H NMR (400 MHz, DMSO) δ 11.06 (s, 1H), 8.71 (dd, J = 8.9, 5.1 Hz, 2H), 7.80 (s, 1H), 7.72 (d, J = 7.6 Hz, 1H), 7.61 (d, J = 7.8 Hz, 1H), 7.59 - 7.33 (m, 6H), 7.34 - 7.23 (m, 1H), 4.89 (s, 1H), 4.60 (s, 1H), 4.07 (s, 1H), 3.63 (d, J = 5.9 Hz, 1H), 2.93 (s, 1H), 2.88 (s, 1H).

### Preparative Example 4: (5-methylpyridin-3-yl)(8-phenyl-1,3,4,5-tetrahydro-2H-pyrido[4,3-b]indol-2-yl)methanone (Compound 4)

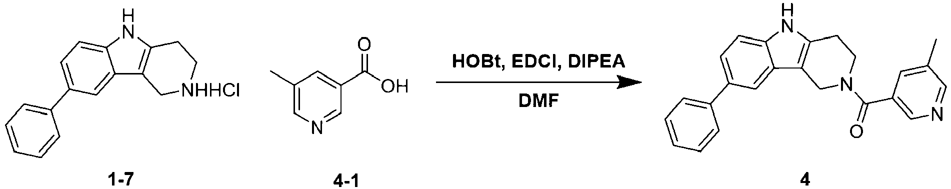

In the same method as in Preparative Example 3, 8-phenyl-2,3,4,5-tetrahydro-1H-pyrido[4,3-b]indole hydrochloride (20 mg, 0.07 mmol), 5-methylnicotinic acid (11.56 mg) , 0.084 mmol), HOBt (18.98 mg, 0.14 mmol), EDCI (26.93 mg, 0.14 mmol) and DIPEA (0.049 mL, 0.28 mmol) were used to obtain 12.4 mg of the above title compound (yield: 48%).

¹H NMR (400 MHz, DMSO) δ 11.03 (s, 1H), 8.50 (d, J = 14.1 Hz, 2H), 7.80 - 7.53 (m, 4H), 7.49 - 7.22 (m, 5H), 4.87 (s, 1H), 4.65 (s, 1H), 4.03 (s, 1H), 3.68 (s, 1H), 2.89 (d, J = 7.4 Hz, 2H), 2.36 (s, 3H).

### Preparative Example 5: (6-methylpyridin-3-yl)(8-phenyl-1,3,4,5-tetrahydro-2H-pyrido[4,3-b]indol-2-yl)methanone (Compound 5)

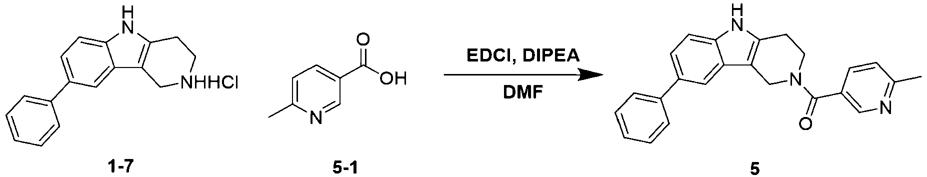

To a solution of 8-phenyl-2,3,4,5-tetrahydro-1H-pyrido[4,3-b]indole hydrochloride (20 mg, 0.07 mmol) in dimethylformamide under nitrogen was added 6-methylnicotinic acid (11.56 mg, 0.084 mmol), EDCI (53.83 mg, 0.28 mmol) and DIPEA (0.049 mL, 0.28 mmol). The reaction mixture was stirred at room temperature for 12 hours. After the completion of the reaction, the solvent was extracted using distilled water and ethyl acetate, and the organic layer was dried over anhydrous magnesium sulfate and filtered. The filtrate was concentrated under reduced pressure, and the concentrate was subjected to column chromatography to obtain 12.6 mg of the title compound (yield: 48%).

¹H NMR (400 MHz, DMSO) δ 11.06 (s, 1H), 8.58 (s, 1H), 7.80 (s, 2H), 7.72 (s, 1H), 7.61 (d, J = 16.8 Hz, 1H), 7.44 (s, 1H), 7.39 (s, 4H), 7.29 (s, 1H), 4.86 (s, 1H), 4.70 (s, 1H), 4.06 - 4.01 (m, 1H), 3.70 (s, 1H), 2.92 (s, 2H), 2.54 (s, 3H).

### Preparative Example 6: (5-methylpyrazin-2-yl)(8-phenyl-1,3,4,5-tetrahydro-2H-pyrido[4,3-b]indol-2-yl)methanone (Compound 6)

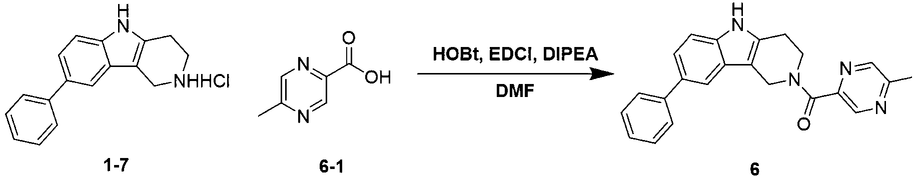

In the same method as in Preparative Example 3, 8-phenyl-2,3,4,5-tetrahydro-1H-pyrido[4,3-b]indole hydrochloride (20 mg, 0.07 mmol), 5-methylpyrazine-2-carboxylic acid (11.64 mg, 0.084 mmol), HOBt (18.98 mg, 0.14 mmol), EDCI (26.93 mg, 0.14 mmol) and DIPEA (0.049 mL, 0.28 mmol) were used to obtain 9.8 mg of the title compound (37% yield).

¹H NMR (400 MHz, DMSO) δ 11.03 (s, 1H), 8.80 - 8.70 (m, 1H), 8.60 (d, J = 6.1 Hz, 1H), 7.79 (s, 1H), 7.71 (d, J = 7.8 Hz, 1H), 7.64 - 7.51 (m, 1H), 7.46 - 7.33 (m, 4H), 7.27 (dt, J = 14.7, 7.4 Hz, 1H), 4.90 (s, 1H), 4.72 (s, 1H), 4.07 (t, J = 6.0 Hz, 1H), 3.77 (t, J = 5.7 Hz, 1H), 2.95 - 2.88 (m, 2H), 2.57 (d, J = 8.5 Hz, 3H).

### Preparative Example 7: (8-phenyl-1,3,4,5-tetrahydro-2H-pyrido[4,3-b]indol-2-yl)(pyrazin-2-yl)methanone (Compound 7)

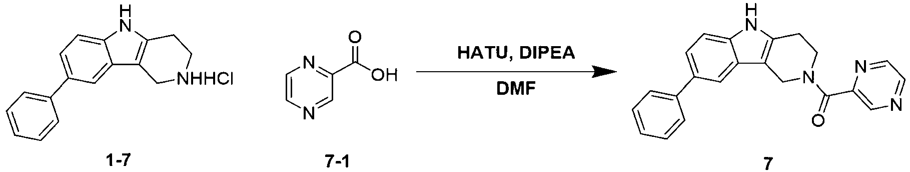

In the same method as in Preparative Example 2, 8-phenyl-2,3,4,5-tetrahydro-1H-pyrido[4,3-b]indole hydrochloride (20 mg, 0.07 mmol), pyrazine-2-carboxylic acid (10.46 mg, 0.084 mmol), HATU (106.81 mg, 0.28 mmol) and DIPEA (0.049 mL, 0.28 mmol) were used to obtain 9.2 mg of the title compound (yield : 36%).

¹H NMR (400 MHz, DMSO) δ 11.03 (s, 1H), 8.88 (dd, J = 23.3, 1.5 Hz, 1H), 8.77 (dd, J = 10.9, 2.6 Hz, 1H), 8.73 - 8.68 (m, 1H), 7.79 (s, 1H), 7.74 - 7.67 (m, 1H), 7.59 (d, J = 7.7 Hz, 1H), 7.47 - 7.31 (m, 4H), 7.26 (dt, J = 15.6, 7.3 Hz, 1H), 4.91 (s, 1H), 4.69 (s, 1H), 4.07 (t, J = 5.7 Hz, 1H), 3.75 (t, J = 5.6 Hz, 1H), 2.96 - 2.87 (m, 2H).

### Preparative Example 8: (8-phenyl-1,3,4,5-tetrahydro-2H-pyrido[4,3-b]indol-2-yl)(pyrimidin-5-yl)methanone (Compound 8)

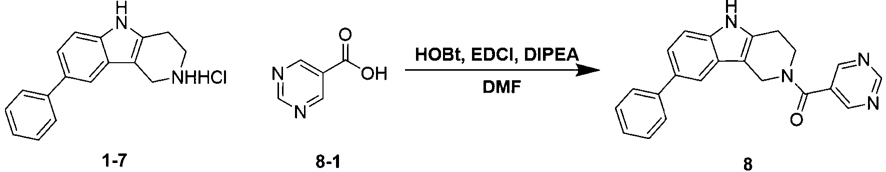

In the same method as in Preparative Example 3, 8-phenyl-2,3,4,5-tetrahydro-1H-pyrido[4,3-b]indole hydrochloride (20 mg, 0.07 mmol), pyrimidine-5-carboxyl Acid (10.46 mg, 0.084 mmol), HOBt (18.98 mg, 0.14 mmol), EDCI (26.93 mg, 0.14 mmol) and DIPEA (0.049 mL, 0.28 mmol) were used to obtain 9.1 mg of the title compound (yield : 36%).

¹H NMR (400 MHz, DMSO) δ 11.06 (s, 1H), 9.31 (d, J = 6.6 Hz, 1H), 8.98 (d, J = 14.3 Hz, 2H), 7.80 (s, 1H), 7.72 (d, J = 7.8 Hz, 1H), 7.62 (d, J = 8.6 Hz, 1H), 7.47 - 7.34 (m, 4H), 7.32 - 7.23 (m, 1H), 4.90 (s, 1H), 4.72 (s, 1H), 4.06 (s, 1H), 3.72 (s, 1H), 2.94 (s, 2H).

### Preparative Example 9: Phenyl(8-phenyl-1,3,4,5-tetrahydro-2H-pyrido[4,3-b]indol-2-yl)methanone (Compound 9)

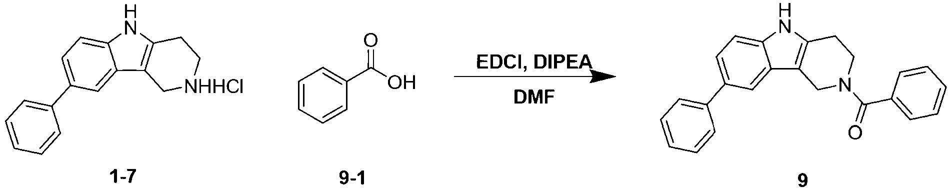

In the same method as in Preparative Example 5, 8-phenyl-2,3,4,5-tetrahydro-1H-pyrido [4,3-b] indole hydrochloride (20 mg, 0.07 mmol), benzoic acid (10.29 mg, 0.084 mmol), EDCI (53.83 mg, 0.28 mmol) and DIPEA (0.049 mL, 0.28 mmol) were used to obtain 7.5 mg of the title compound (yield: 30%).

¹H NMR (400 MHz, DMSO) δ 11.03 (s, 1H), 7.69 (t, J = 35.9 Hz, 3H), 7.43 (d, J = 41.2 Hz, 10H), 4.84 (s, 1H), 4.64 (s, 1H), 4.03 (s, 1H), 3.67 (s, 1H), 2.88 (s, 2H).

### Preparative Example 10: (1-methyl-1H-indol-4-yl)(8-phenyl-1,3,4,5-tetrahydro-2H-pyrido[4,3-b]indol-2-yl)methanone (Compound 10)

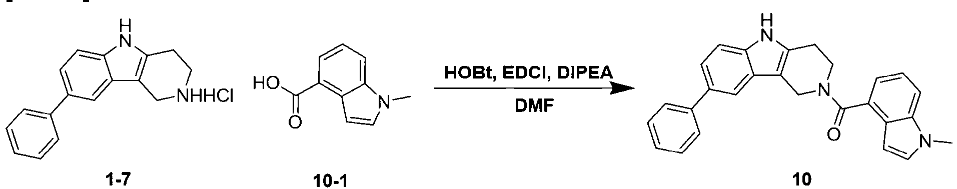

In the same method as in Preparative Example 3, 8-phenyl-2,3,4,5-tetrahydro-1H-pyrido[4,3-b]indole hydrochloride (20 mg, 0.07 mmol), 1-methyl-1H-indole-4-carboxylic acid (14.76 mg, 0.084 mmol), HOBt (18.98 mg, 0.14 mmol), EDCI (26.93 mg, 0.14 mmol) and DIPEA (0.049 mL, 0.28 mmol) were used to obtain 20.3 mg of the title compound (yield : 71%).

¹H NMR (400 MHz, DMSO) δ 11.02 (s, 1H), 7.82 - 7.21 (m, 11H), 7.10 (d, J = 7.1 Hz, 1H), 6.35 (s, 1H), 4.95 (s, 1H), 4.56 (s, 1H), 4.11 (s, 1H), 3.84 (s, 3H), 3.62 (s, 1H), 2.86 (d, J = 82.9 Hz, 2H).

### Preparative Example 11: (8-phenyl-1,3,4,5-tetrahydro-2H-pyrido[4,3-b]indol-2-yl)(thiazol-2-yl)methanone (Compound 11)

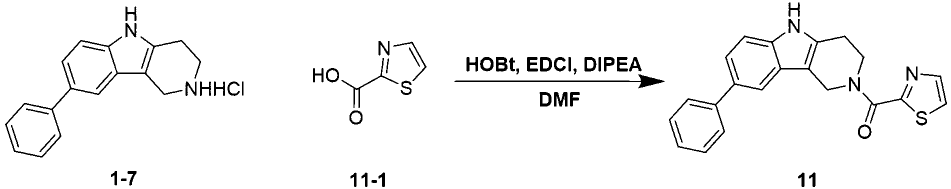

In the same method as in Preparative Example 3, 8-phenyl-2,3,4,5-tetrahydro-1H-pyrido[4,3-b]indole hydrochloride (20 mg, 0.07 mmol), thiazole-2-carboxyl Acid (10.89 mg, 0.084 mmol), HOBt (18.98 mg, 0.14 mmol), EDCI (26.93 mg, 0.14 mmol) and DIPEA (0.049 mL, 0.28 mmol) were used to obtain 7.5 mg of the title compound (yield : 29%).

¹H NMR (400 MHz, DMSO) δ 11.06 (s, 1H), 8.11 - 8.02 (m, 2H), 7.80 - 7.59 (m, 3H), 7.48 - 7.35 (m, 4H), 7.29 (t, J = 7.4 Hz, 1H), 5.49 (s, 1H), 4.92 (s, 1H), 4.57 (t, J = 5.4 Hz, 1H), 4.08 (s, 1H), 2.97 (d, J = 17.7 Hz, 2H).

### Preparative Example 12: (8-phenyl-1,3,4,5-tetrahydro-2H-pyrido[4,3-b]indol-2-yl)(4-(trifluoromethyl)phenyl)methanone (Compound 12)

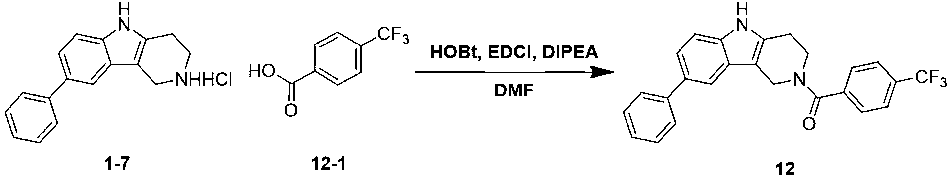

In the same method as in Preparative Example 3, 8-phenyl-2,3,4,5-tetrahydro-1H-pyrido[4,3-b]indole hydrochloride (20 mg, 0.07 mmol), 4-(trifluoromethyl ) Benzoic acid (16.03 mg, 0.084 mmol), HOBt (18.98 mg, 0.14 mmol), EDCI (26.93 mg, 0.14 mmol) and DIPEA (0.049 mL, 0.28 mmol) were used to obtain 16.2 mg of the title compound (yield : 54%).

¹H NMR (400 MHz, DMSO) δ 11.05 (s, 1H), 7.89 - 7.52 (m, 7H), 7.48 - 7.22 (m, 5H), 4.88 (s, 1H), 4.62 (s, 1H), 4.05 (s, 1H), 3.63 (s, 1H), 2.90 (d, J = 20.3 Hz, 2H).

### Preparative Example 13: (3-chlorophenyl)(8-phenyl-1,3,4,5-tetrahydro-2H-pyrido[4,3-b]indol-2-yl)methanone (Compound 13)

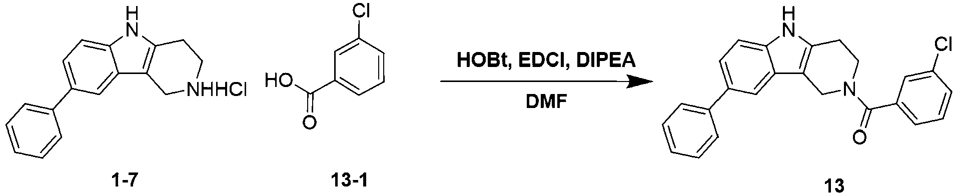

In the same method as in Preparative Example 3, 8-phenyl-2,3,4,5-tetrahydro-1H-pyrido [4,3-b] indole hydrochloride (20 mg, 0.07 mmol), 3-chlorobenzoic acid (13.2 mg , 0.084 mmol), HOBt (18.98 mg, 0.14 mmol), EDCI (26.93 mg, 0.14 mmol) and DIPEA (0.049 mL, 0.28 mmol) were used to obtain 15.8 mg of the title compound (yield : 58%).

¹H NMR (400 MHz, DMSO) δ 11.02 (s, 1H), 7.79 - 7.23 (m, 12H), 4.85 (s, 1H), 4.61 (s, 1H), 4.02 (s, 1H), 3.65 (s, 1H), 2.88 (s, 2H).

### Preparative Example 14: (8-phenyl-1,3,4,5-tetrahydro-2H-pyrido[4,3-b]indol-2-yl)(p-tolyl)methanone (Compound 14)

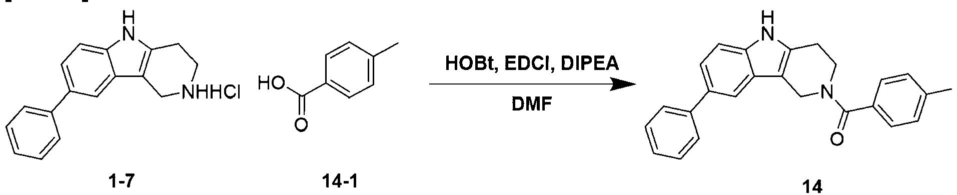

In the same method as in Preparative Example 3, 8-phenyl-2,3,4,5-tetrahydro-1H-pyrido [4,3-b] indole hydrochloride (20 mg, 0.07 mmol), 4-methylbenzoic acid (11.47 mg , 0.084 mmol), HOBt (18.98 mg, 0.14 mmol), EDCI (26.93 mg, 0.14 mmol) and DIPEA (0.049 mL, 0.28 mmol) were used to obtain 13.2 mg of the title compound (yield : 51%).

¹H NMR (400 MHz, DMSO) δ 11.02 (s, 1H), 7.69 (s, 3H), 7.37 (d, J = 7.8 Hz, 5H), 7.27 (d, J = 7.4 Hz, 3H), 4.81 (s, 1H), 4.66 (s, 1H), 3.99 (s, 1H), 3.68 (s, 1H), 2.87 (s, 2H), 2.35 (s, 3H).

### Preparative Example 15: (8-phenyl-1,3,4,5-tetrahydro-2H-pyrido[4,3-b]indol-2-yl)(m-tolyl)methanone (Compound 15)

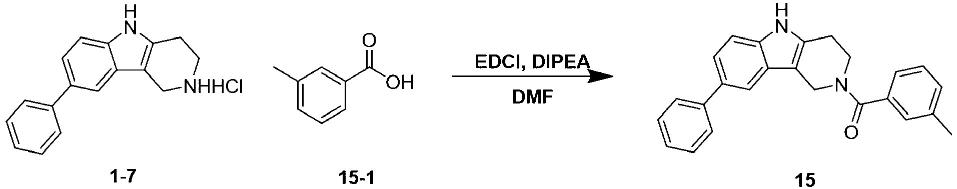

In the same method as in Preparative Example 5, 8-phenyl-2,3,4,5-tetrahydro-1H-pyrido [4,3-b] indole hydrochloride (20 mg, 0.07 mmol), 3-methylbenzoic acid (11.47 mg , 0.084 mmol), EDCI (53.83 mg, 0.28 mmol) and DIPEA (0.049 mL, 0.28 mmol) were used to obtain 10.2 mg of the title compound (yield: 39%).

¹H NMR (400 MHz, DMSO) δ 11.04 (s, 1H), 7.70 (t, J = 34.9 Hz, 3H), 7.54 - 7.22 (m, 9H), 4.85 (s, 1H), 4.64 (s, 1H), 4.03 (s, 1H), 3.68 (s, 1H), 2.89 (s, 2H), 2.37 (s, 3H).

### Preparative Example 16: (4-ethylphenyl)(8-phenyl-1,3,4,5-tetrahydro-2H-pyrido[4,3-b]indol-2-yl)methanone (Compound 16)

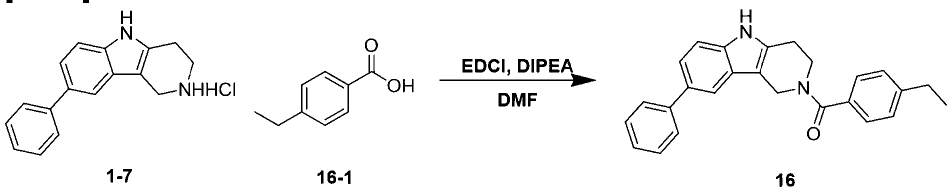

In the same method as in Preparative Example 5, 8-phenyl-2,3,4,5-tetrahydro-1H-pyrido [4,3-b] indole hydrochloride (20 mg, 0.07 mmol), 4-ethylbenzoic acid (12.65 mg , 0.084 mmol), EDCI (53.83 mg, 0.28 mmol) and DIPEA (0.049 mL, 0.28 mmol) were used to obtain 4.4 mg of the title compound (yield: 16%).

¹H NMR (400 MHz, DMSO) δ 11.05 (s, 1H), 7.71 (t, J = 31.6 Hz, 3H), 7.48 - 7.36 (m, 6H), 7.32 (d, J = 8.0 Hz, 3H), 4.83 (s, 1H), 4.69 (s, 1H), 4.04 (s, 1H), 3.69 (s, 1H), 2.90 (s, 2H), 2.67 (q, J = 7.7 Hz, 2H), 1.22 (t, J = 7.6 Hz, 3H).

### Preparative Example 17: (4-methoxyphenyl)(8-phenyl-1,3,4,5-tetrahydro-2H-pyrido[4,3-b]indol-2-yl)methanone (Compound 17)

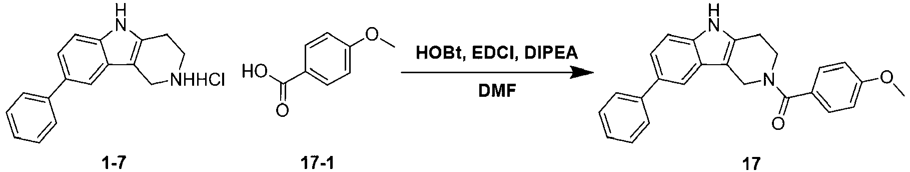

In the same method as in Preparative Example 3, 8-phenyl-2,3,4,5-tetrahydro-1H-pyrido [4,3-b] indole hydrochloride (20 mg, 0.07 mmol), 4-methoxybenzoic acid (12.82 mg, 0.084 mmol), HOBt (18.98 mg, 0.14 mmol), EDCI (26.93 mg, 0.14 mmol) and DIPEA (0.049 mL, 0.28 mmol) were used to obtain 10.7 mg of the titled compound (yield: 39%).

¹H NMR (400 MHz, DMSO) δ 10.99 (s, 1H), 7.63 (s, 3H), 7.46 - 7.32 (m, 6H), 7.24 (t, J = 7.3 Hz, 1H), 6.98 (d, J = 8.4 Hz, 2H), 4.73 (s, 2H), 3.78 (s, 5H), 2.86 (s, 2H).

### Preparative Example 18: (3-methoxyphenyl)(8-phenyl-1,3,4,5-tetrahydro-2H-pyrido[4,3-b]indol-2-yl)methanone (Compound 18)

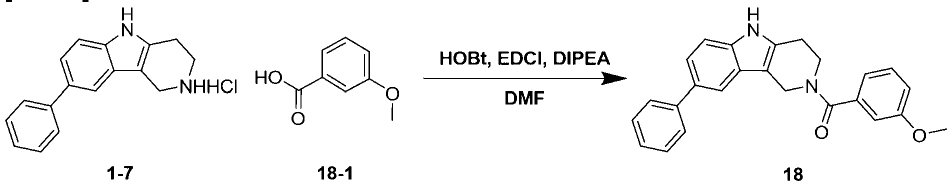

In the same method as in Preparative Example 3, 8-phenyl-2,3,4,5-tetrahydro-1H-pyrido [4,3-b] indole hydrochloride (20 mg, 0.07 mmol), 4-methoxybenzoic acid (12.82 mg, 0.084 mmol), HOBt (18.98 mg, 0.14 mmol), EDCI (26.93 mg, 0.14 mmol) and DIPEA (0.049 mL, 0.28 mmol) were used to obtain 16.3 mg of the title compound (yield : 60%).

¹H NMR (400 MHz, DMSO) δ 11.04 (s, 1H), 7.71 (t, J = 33.9 Hz, 3H), 7.34 (d, J = 41.8 Hz, 6H), 7.09 - 6.97 (m, 3H), 4.85 (s, 1H), 4.64 (s, 1H), 4.03 (s, 1H), 3.80 (d, J = 10.9 Hz, 3H), 3.68 (s, 1H), 2.89 (s, 2H).

### Preparative Example 19: Morpholino(8-phenyl-1,3,4,5-tetrahydro-2H-pyrido[4,3-b]indol-2-yl)methanone (Compound 19)

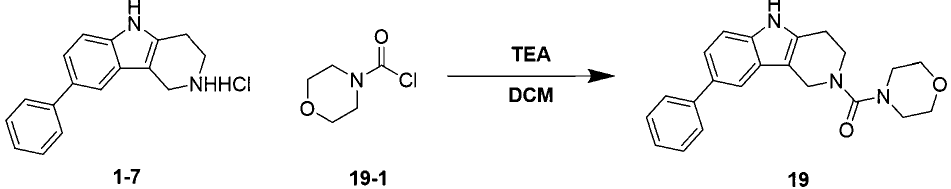

To a solution of the 8-phenyl-2,3,4,5-tetrahydro-1H-pyrido[4,3-b]indole hydrochloride (60 mg, 0.21 mmol) in dichloromethane under nitrogen was added Morpholine-4-carbonyl chloride (0.03 mL, 0.27 mmol) and TEA (0.12 mL, 0.84 mmol). The reaction mixture was stirred at room temperature for 12 hours. After the completion of the reaction, the solvent was extracted using distilled water and ethyl acetate, and the organic layer was dried over anhydrous magnesium sulfate and filtered. The filtrate was concentrated under reduced pressure, and the concentrate was subjected to column chromatography to obtain 9.3 mg of the title compound (yield: 12%).

¹H NMR (400 MHz, DMSO) δ 10.95 (s, 1H), 7.72 - 7.63 (m, 3H), 7.42 (t, J = 7.7 Hz, 2H), 7.35 (t, J = 1.0 Hz, 2H), 7.32 - 7.23 (m, 1H), 4.46 (s, 2H), 3.62 - 3.59 (m, 4H), 3.54 (d, J = 5.6 Hz, 2H), 3.19 (t, J = 4.7 Hz, 4H), 2.85 (d, J = 5.7 Hz, 2H).

### Preparative Example 20: Preparation of Compound 20

### Preparative Example 20-1: tert-butyl 8-(o-tolyl)-1,3,4,5-tetrahydro-2H-pyrido[4,3-b]indole-2-carboxylate (Compound 20-2)

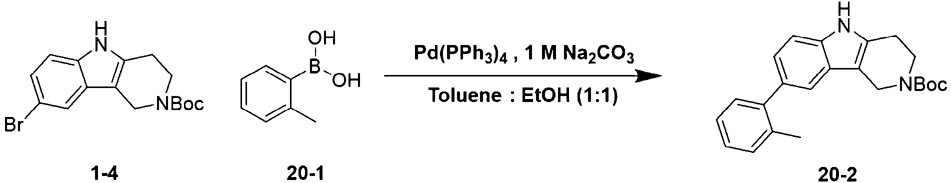

In the same method as in Preparative Example 1-3, *tert-*butyl 8-bromo-1,3,4,5-tetrahydro-2H-pyrido[4,3-b]indole-2-carboxylate (300 mg, 0.85 mmol), o-tolylboronic acid (696.74 mg, 5.12 mmol), Pd(PPh₃)₄ (49.35 mg, 0.043 mmol) and 1 M Na₂CO₃ (2.14 mL, 2.14 mmol) were used to obtain 258.2 mg of the title compound (yield : 83%).

¹H NMR (400 MHz, DMSO) δ 10.93 (s, 1H), 7.34 - 7.16 (m, 6H), 6.98 (dd, J = 8.3, 1.7 Hz, 1H), 4.51 (s, 2H), 3.69 (t, J = 5.7 Hz, 2H), 2.77 (t, J = 5.8 Hz, 2H), 2.21 (s, 3H), 1.41 (s, 9H).

### Preparative Example 20-2: 8-(o-tolyl)-2,3,4,5-tetrahydro-1H-pyrido[4,3-b]indole hydrochloride (Compound 20-3)

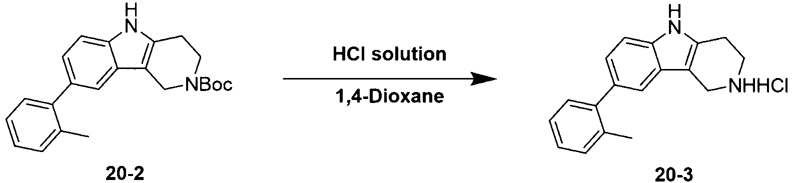

In the same method as in Preparative Example 1-4, *tert-*butyl 8-(o-tolyl)-1,3,4,5-tetrahydro-2H-pyrido[4,3-b]indole-2-carboxylate ( 258 mg, 0.71 mmol) and HCl solution (4 M in dioxane) (3.56 mL, 14.24 mmol) were used to obtain 167 mg of the title compound (yield : 78%).

¹H NMR (400 MHz, DMSO) δ 11.19 (s, 1H), 7.38 - 7.31 (m, 2H), 7.25 - 7.16 (m, 4H), 7.01 (dd, J = 8.3, 1.7 Hz, 1H), 4.25 (d, J = 4.6 Hz, 2H), 3.43 (d, J = 6.7 Hz, 2H), 3.02 (t, J = 6.1 Hz, 2H), 2.19 (s, 3H), 1.93 (s, 9H).

### Preparative Example 20-3: Pyridin-3-yl(8-(o-tolyl)-1,3,4,5-tetrahydro-2H-pyrido[4,3-b]indol-2-yl)methanone (Compound 20)

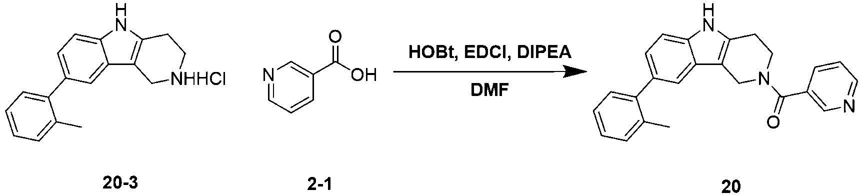

In the same method as in Preparative Example 3, 8-(o-tolyl)-2,3,4,5-tetrahydro-1H-pyrido[4,3-b]indole hydrochloride (20 mg, 0.067 mmol), nicotinic acid (9.88 mg, 0.08 mmol), HOBt (18.08 mg, 0.13 mmol), EDCI (25.65 mg, 0.13 mmol) and DIPEA (0.05 mL, 0.27 mmol) were used to obtain 16.2 mg of the title compound (yield: 66%).

¹H NMR (400 MHz, DMSO) δ 11.04 (s, 1H), 8.70 (s, 2H), 7.92 (s, 1H), 7.52 (s, 1H), 7.42 - 7.16 (m, 6H), 7.03 (d, J = 9.1 Hz, 1H), 4.84 (s, 1H), 4.63 (s, 1H), 4.04 (s, 1H), 3.69 (s, 1H), 2.91 (d, J = 9.4 Hz, 2H), 2.22 (d, J = 35.3 Hz, 3H).

### Preparative Example 21: Pyridin-4-yl(8-(o-tolyl)-1,3,4,5-tetrahydro-2H-pyrido[4,3-b]indol-2-yl)methanone (Compound 21)

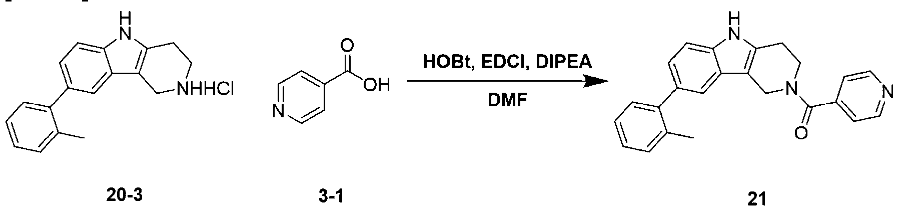

In the same method as in Preparative Example 3, 8-(o-tolyl)-2,3,4,5-tetrahydro-1H-pyrido[4,3-b]indole hydrochloride (20 mg, 0.067 mmol), isonicotinic acid ( 9.88 mg, 0.08 mmol), HOBt (18.08 mg, 0.13 mmol), EDCI (25.65 mg, 0.13 mmol) and DIPEA (0.05 mL, 0.27 mmol) were used to obtain 4.3 mg of the title compound (yield: 17%).

¹H NMR (400 MHz, DMSO) δ 11.04 (s, 1H), 8.68 (dd, J = 21.4, 5.2 Hz, 2H), 7.50 - 7.46 (m, 1H), 7.44 - 7.12 (m, 7H), 7.00 (dd, J = 20.7, 8.3 Hz, 1H), 4.83 (s, 1H), 4.54 (s, 1H), 4.04 (s, 1H), 3.61 (t, J = 5.5 Hz, 1H), 2.89 (d, J = 20.4 Hz, 2H), 2.21 (d, J = 36.8 Hz, 3H).

### Preparative Example 22: (5-methylpyridin-3-yl)(8-(o-tolyl)-1,3,4,5-tetrahydro-2H-pyrido[4,3-b]indol-2-yl)methanone (Compound 22)

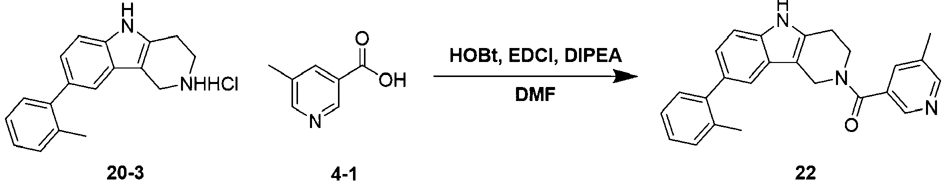

In the same method as in Preparative Example 3, 8-(o-tolyl)-2,3,4,5-tetrahydro-1H-pyrido[4,3-b]indole hydrochloride (20 mg, 0.07 mmol), 5-methyl Nicotinic acid (11.01 mg, 0.08 mmol), HOBt (18.08 mg, 0.13 mmol), EDCI (25.65 mg, 0.13 mmol) and DIPEA (0.05 mL, 0.27 mmol) were used to obtain 13.7 mg of the title compound (yield : 55%).

¹H NMR (400 MHz, DMSO) δ 11.03 (s, 1H), 8.50 (d, J = 18.1 Hz, 2H), 7.71 (d, J = 17.9 Hz, 1H), 7.41 - 7.16 (m, 6H), 7.02 (d, J = 9.0 Hz, 1H), 4.82 (s, 1H), 4.61 (s, 1H), 4.03 (s, 1H), 3.67 (s, 1H), 2.90 (s, 2H), 2.34 (d, J = 20.3 Hz, 3H), 2.17 (s, 2H).

### Preparative Example 23: Phenyl(8-(o-tolyl)-1,3,4,5-tetrahydro-2H-pyrido[4,3-b]indol-2-yl)methanone (Compound 23)

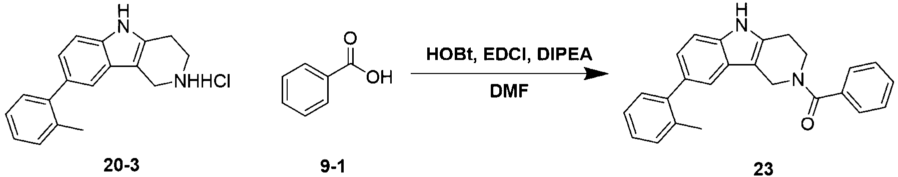

In the same method as in Preparative Example 3, 8-(o-tolyl)-2,3,4,5-tetrahydro-1H-pyrido[4,3-b]indole hydrochloride (20 mg, 0.07 mmol), benzoic acid (9.8 mg, 0.08 mmol), HOBt (18.08 mg, 0.13 mmol), EDCI (25.65 mg, 0.13 mmol) and DIPEA (0.05 mL, 0.27 mmol) were used to obtain 12.9 mg of the title compound (yield : 52%).

¹H NMR (400 MHz, DMSO) δ 11.02 (s, 1H), 7.47 (s, 5H), 7.35 (s, 1H), 7.23 (s, 5H), 7.01 (s, 1H), 4.80 (s, 1H), 4.60 (s, 1H), 4.03 (s, 1H), 3.67 (s, 1H), 2.89 (s, 2H), 2.22 (d, J = 31.2 Hz, 3H).

### Preparative Example 24: (1-methyl-1H-indol-4-yl)(8-(o-tolyl)-1,3,4,5-tetrahydro-2H-pyrido[4,3-b]indol-2-yl)methanone (Compound 24)

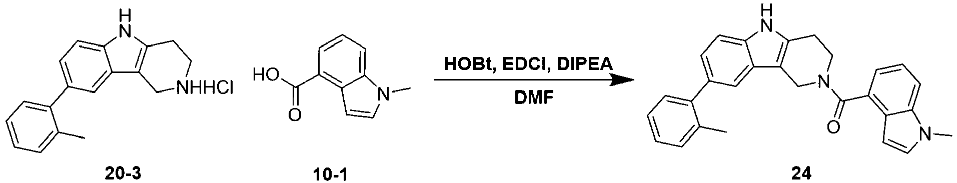

In the same method as in Preparative Example 3, 8-(o-tolyl)-2,3,4,5-tetrahydro-1H-pyrido[4,3-b]indole hydrochloride (20 mg, 0.07 mmol), 1-methyl -1H-indole-4-carboxylic acid (14.06 mg, 0.08 mmol), HOBt (18.08 mg, 0.13 mmol), EDCI (25.65 mg, 0.13 mmol) and DIPEA (0.05 mL, 0.27 mmol) were used to obtain 14.8 mg of the title compound (yield 52%).

¹H NMR (400 MHz, DMSO) δ 11.00 (s, 1H), 7.54 (d, J = 8.2 Hz, 1H), 7.45 - 6.85 (m, 11H), 6.34 (s, 1H), 4.89 (s, 1H), 4.51 (s, 1H), 4.04 (s, 1H), 3.83 (s, 3H), 3.61 (s, 1H), 2.86 (d, J = 78.4 Hz, 2H), 2.27 (s, 3H).

### Preparative Example 25: Thiazol-2-yl(8-(o-tolyl)-1,3,4,5-tetrahydro-2H-pyrido[4,3-b]indol-2-yl)methanone (Compound 25)

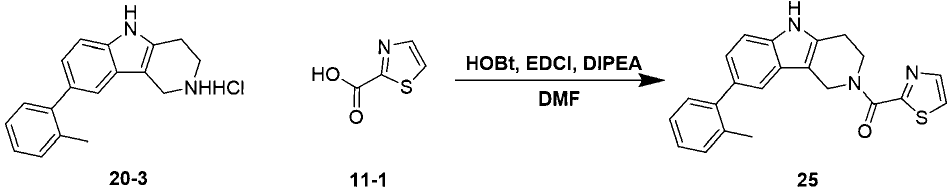

In the same method as in Preparative Example 3, 8-(o-tolyl)-2,3,4,5-tetrahydro-1H-pyrido[4,3-b]indole hydrochloride (20 mg, 0.07 mmol), thiazole-2-carboxylic acid (10.37 mg, 0.08 mmol), HOBt (18.08 mg, 0.13 mmol), EDCI (25.65 mg, 0.13 mmol) and DIPEA (0.05 mL, 0.27 mmol) were used to obtain 12.7 mg of the title compound (yield : 50 %).

¹H NMR (400 MHz, DMSO) δ 11.04 (s, 1H), 8.10 - 7.99 (m, 2H), 7.43 - 7.31 (m, 2H), 7.29 - 7.19 (m, 4H), 7.02 (d, J = 8.3 Hz, 1H), 5.46 (s, 1H), 4.87 (s, 1H), 4.56 (s, 1H), 4.08 (s, 1H), 2.96 (d, J = 16.6 Hz, 2H), 2.24 (d, J = 14.1 Hz, 3H).

### Preparative Example 26: (3-chlorophenyl)(8-(o-tolyl)-1,3,4,5-tetrahydro-2H-pyrido[4,3-b]indol-2-yl)methanone (Compound 26)

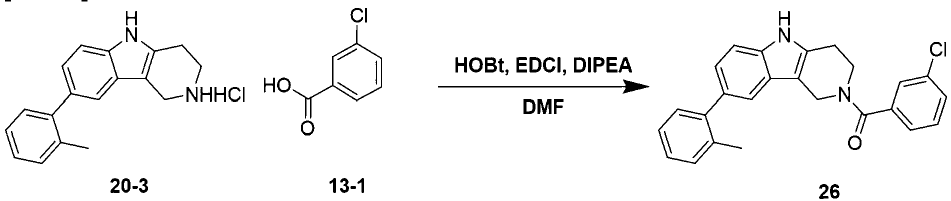

In the same method as in Preparative Example 3, 8-(o-tolyl)-2,3,4,5-tetrahydro-1H-pyrido[4,3-b]indole hydrochloride (20 mg, 0.07 mmol), 3-chloro benzoic acid (12.57 mg, 0.08 mmol), HOBt (18.08 mg, 0.13 mmol), EDCI (25.65 mg, 0.13 mmol) and DIPEA (0.05 mL, 0.27 mmol) were used to obtain 19.9 mg of the title compound (yield : 74%).

¹H NMR (400 MHz, DMSO) δ 11.04 (s, 1H), 7.61 - 7.14 (m, 10H), 7.02 (s, 1H), 4.82 (s, 1H), 4.58 (s, 1H), 4.03 (s, 1H), 3.66 (s, 1H), 2.89 (s, 2H), 2.27 (s, 3H).

### Preparative Example 27: Preparation of Compound 27

### Preparative Example 27-1: tert-butyl 8-(m-tolyl)-1,3,4,5-tetrahydro-2H-pyrido[4,3-b]indole-2-carboxylate (Compound 27-2)

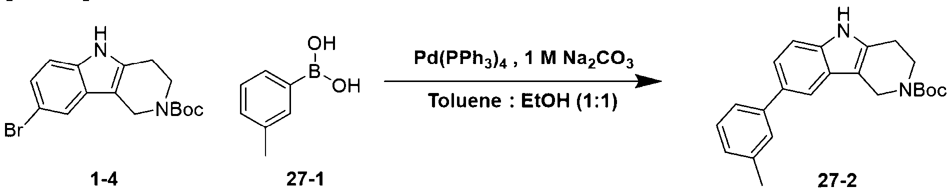

In the same method as in Preparative Example 1-3, *tert-butyl* 8-bromo-1,3,4,5-tetrahydro-2H-pyrido[4,3-b]indole-2-carboxylate (200 mg, 0. 75 mmol), m-tolylboronic acid (464.49 mg, 3.42 mmol), Pd(PPh₃)₄ (32.9 mg, 0.028 mmol) and 1 M Na₂CO₃ (1.42 mL, 1.42 mmol) were used to obtain 137 mg of the title compound (yield : 66%).

¹H NMR (400 MHz, DMSO) δ 10.96 (s, 1H), 7.66 (s, 1H), 7.53 - 7.43 (m, 2H), 7.40 - 7.27 (m, 3H), 7.10 (d, J = 7.5 Hz, 1H), 4.59 (s, 2H), 3.72 (t, J = 5.8 Hz, 2H), 2.78 (d, J = 6.1 Hz, 2H), 2.38 (s, 3H), 1.45 (d, J = 1.2 Hz, 9H).

### Preparative Example 27-2: 8-(m-tolyl)-2,3,4,5-tetrahydro-1H-pyrido[4,3-b]indole hydrochloride (Compound 27-3)

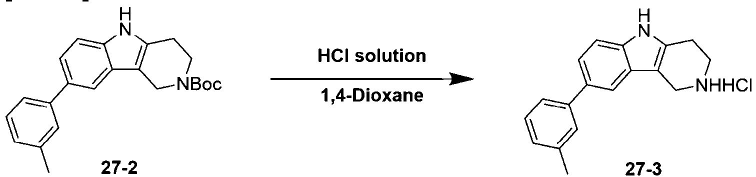

In the same method as in Preparative Example 1-4, *tert-butyl* 8-(m-tolyl)-1,3,4,5-tetrahydro-2H-pyrido[4,3-b]indole-2-carboxylate (137 mg, 0.38 mmol) and HCl solution (4 M in dioxane) (1.89 mL, 7.56 mmol) were used to obtain 91.3 mg of the title compound (yield: 80%).

¹H NMR (400 MHz, DMSO) δ 11.22 (s, 1H), 7.76 (s, 1H), 7.50 (s, 1H), 7.47 (d, J = 7.9 Hz, 1H), 7.42 (d, J = 1.2 Hz, 2H), 7.33 (t, J = 7.6 Hz, 1H), 7.12 (d, J = 7.5 Hz, 1H), 4.37 (s, 2H), 3.48 (s, 2H), 3.05 (t, J = 6.1 Hz, 2H), 2.39 (s, 3H).

### Preparative Example 27-3: Pyridin-3-yl(8-(m-tolyl)-1,3,4,5-tetrahydro-2H-pyrido[4,3-b]indol-2-yl)methanone (Compound 27)

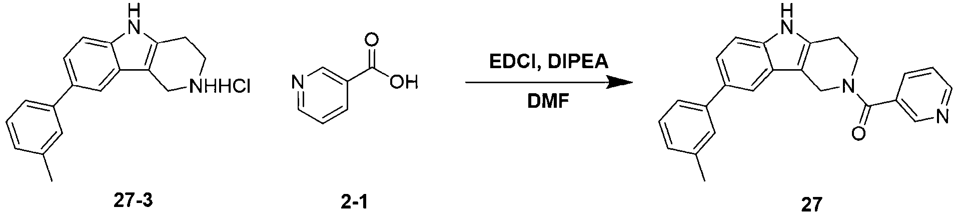

In the same method as in Preparative Example 5, 8-(m-tolyl)-2,3,4,5-tetrahydro-1H-pyrido[4,3-b]indole hydrochloride (20 mg, 0.07 mmol), nicotinic acid (9.88 mg, 0.08 mmol), EDCI (51.3 mg, 0.27 mmol) and DIPEA (0.047 mL, 0.27 mmol) were used to obtain 3.2 mg of the title compound (yield: 13%).

¹H NMR (400 MHz, DMSO) δ 11.05 (s, 1H), 8.70 (s, 2H), 7.94 (s, 1H), 7.53 (d, J = 13.6 Hz, 3H), 7.36 (d, J = 19.3 Hz, 4H), 7.11 (d, J = 7.2 Hz, 1H), 4.89 (s, 1H), 4.68 (s, 1H), 4.06 (s, 1H), 3.69 (s, 2H), 2.92 (s, 2H), 2.37 (d, J = 19.4 Hz, 3H).

### Preparative Example 28: Phenyl(8-(m-tolyl)-1,3,4,5-tetrahydro-2H-pyrido[4,3-b]indol-2-yl)methanone (Compound 28)

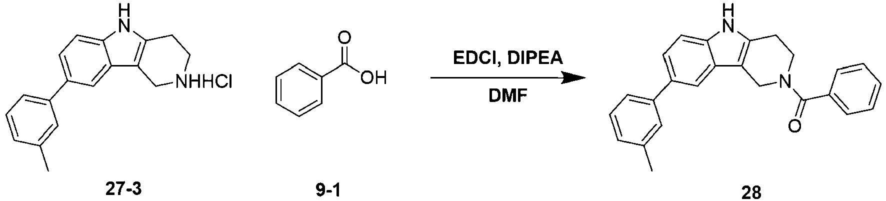

In the same method as in Preparative Example 5, 8-(m-tolyl)-2,3,4,5-tetrahydro-1H-pyrido[4,3-b]indole hydrochloride (20 mg, 0.07 mmol), benzoic acid (9.8 mg, 0.08 mmol), EDCI (51.3 mg, 0.27 mmol) and DIPEA (0.047 mL, 0.27 mmol) were used to obtain 9.7 mg of the title compound (yield: 39%).

¹H NMR (400 MHz, DMSO) δ 11.04 (s, 1H), 7.78 (s, 1H), 7.44 (d, J = 46.5 Hz, 10H), 7.10 (s, 1H), 4.86 (s, 1H), 4.65 (s, 1H), 4.04 (s, 1H), 3.68 (s, 1H), 2.89 (s, 2H), 2.37 (d, J = 16.5 Hz, 3H).

### Preparative Example 29: Preparation of Compound 29

### Preparative Example 29-1: tert-butyl 8-(p-tolyl)-1,3,4,5-tetrahydro-2H-pyrido[4,3-b]indole-2-carboxylate (Compound 29-2)

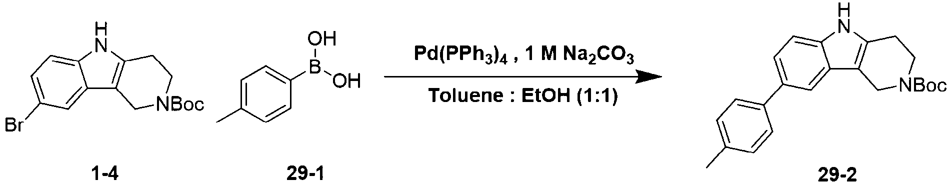

In the same method as in Preparative Example 1-3, *tert-butyl* 8-bromo-1,3,4,5-tetrahydro-2H-pyrido[4,3-b]indole-2-carboxylate (200 mg, 0. 75 mmol), p-tolylboronic acid (464.49 mg, 3.42 mmol), Pd(PPh₃)₄ (32.9 mg, 0.028 mmol) and 1M Na₂CO₃ (1.42 mL, 1.42 mmol) were used to obtain 120 mg of the title compound(yield : 58%).

¹H NMR (400 MHz, DMSO) δ 10.95 (s, 1H), 7.64 (s, 1H), 7.62 - 7.54 (m, 2H), 7.39 - 7.30 (m, 2H), 7.24 (d, J = 7.9 Hz, 2H), 4.58 (s, 2H), 3.72 (t, J = 5.6 Hz, 2H), 2.79 (t, J = 5.6 Hz, 2H), 2.34 (s, 3H), 2.08 (s, 12H), 1.45 (s, 9H).

### Preparative Example 29-2: 8-(p-tolyl)-2,3,4,5-tetrahydro-1H-pyrido[4,3-b]indole hydrochloride (Compound 29-3)

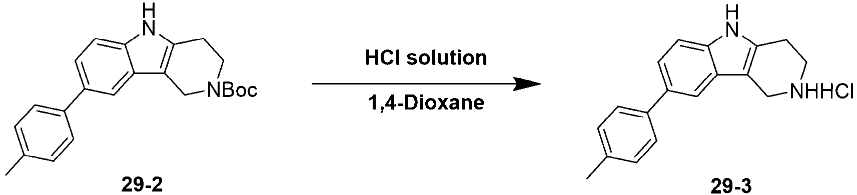

In the same method as in Preparative Example 1-4, *tert-butyl* 8-(p-tolyl)-1,3,4,5-tetrahydro-2H-pyrido[4,3-b]indole-2-carboxylate ( 120 mg, 0.31 mmol) and HCl solution (4 M in dioxane) (1.66 mL, 6.62 mmol) were used to obtain 86.2 mg of the title compound (yield: 87%).

¹H NMR (400 MHz, DMSO) δ 11.22 (s, 1H), 7.75 (s, 1H), 7.61 - 7.54 (m, 2H), 7.40 (d, J = 1.2 Hz, 2H), 7.25 (d, J = 7.9 Hz, 2H), 4.36 (s, 2H), 3.49 (d, J = 5.4 Hz, 2H), 3.05 (t, J = 6.0 Hz, 2H), 2.34 (s, 3H).

### Preparative Example 29-3: Pyridin-3-yl(8-(p-tolyl)-1,3,4,5-tetrahydro-2H-pyrido[4,3-b]indol-2-yl)methanone (Compound 29)

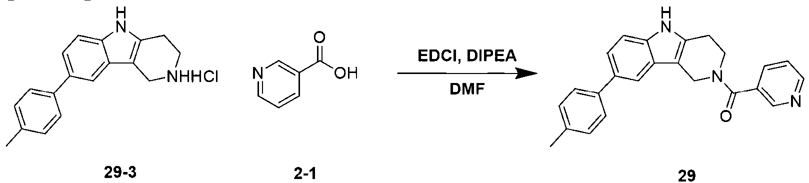

In the same method as in Preparative Example 5, 8-(p-tolyl)-2,3,4,5-tetrahydro-1H-pyrido[4,3-b]indole hydrochloride (20 mg, 0.067 mmol), nicotinic acid (9.88 mg, 0.08 mmol), EDCI (51.3 mg, 0.27 mmol) and DIPEA (0.047 mL, 0.27 mmol) were used to obtain 10.7 mg of the title compound (yield: 43%).

¹H NMR (400 MHz, DMSO) δ 11.04 (s, 1H), 8.70 (s, 2H), 7.95 (d, J = 8.5 Hz, 1H), 7.77 (s, 1H), 7.61 (d, J = 7.6 Hz, 1H), 7.52 (d, J = 7.2 Hz, 2H), 7.35 (d, J = 16.2 Hz, 2H), 7.28 - 7.18 (m, 2H), 4.88 (s, 1H), 4.67 (s, 1H), 4.05 (s, 1H), 3.69 (s, 1H), 2.92 (s, 2H), 2.33 (d, J = 13.0 Hz, 3H).

### Preparative Example 30: Phenyl(8-(p-tolyl)-1,3,4,5-tetrahydro-2H-pyrido[4,3-b]indol-2-yl)methanone (Compound 30)

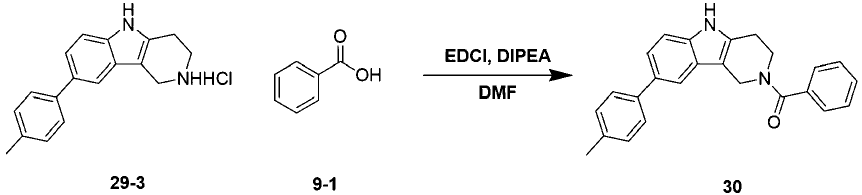

In the same method as in Preparative Example 5, 8-(p-tolyl)-2,3,4,5-tetrahydro-1H-pyrido[4,3-b]indole hydrochloride (20 mg, 0.07 mmol), benzoic acid (9.8 mg, 0.08 mmol), EDCI (51.3 mg, 0.27 mmol) and DIPEA (0.047 mL, 0.27 mmol) were used to obtain 9.7 mg of the title compound (yield: 39%).

¹H NMR (400 MHz, DMSO) δ 11.03 (s, 1H), 7.76 (s, 1H), 7.61 (s, 1H), 7.50 (s, 6H), 7.37 (s, 2H), 7.24 (s, 1H), 7.20 (s, 1H), 4.85 (s, 1H), 4.65 (s, 1H), 4.05 (s, 1H), 3.68 (s, 1H), 2.89 (s, 2H), 2.37 - 2.23 (m, 3H).

### Preparative Example 31: Preparation of Compound 31

### Preparative Example 31-1: tert-butyl 8-(pyridin-3-yl)-1,3,4,5-tetrahydro-2H-pyrido[4,3-b]indole-2-carboxylate (Compound 31-2)

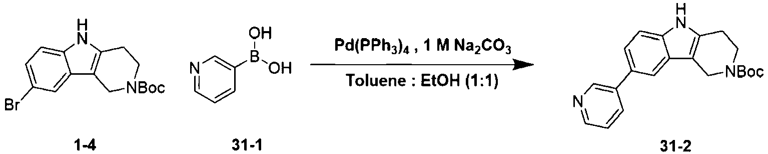

In the same method as in Preparative Example 1-3, *tert-butyl* 8-bromo-1,3,4,5-tetrahydro-2H-pyrido[4,3-b]indole-2-carboxylate (200 mg, 0.57 mmol), pyridin-3-ylboronic acid (209.97 mg, 1.71 mmol), Pd(PPh₃)₄ (32.9 mg, 0.028 mmol) and 1 M Na₂CO₃ (1.42 mL, 1.42 mmol) were used to obtain 104.4 mg of the title compound (yield : 52%).

¹H NMR (400 MHz, CDCl3) δ 8.91 (s, 1H), 8.55 (d, J = 4.7 Hz, 1H), 7.93 (s, 1H), 7.64 (s, 1H), 7.41 (d, J = 8.4 Hz, 1H), 7.35 (d, J = 8.3 Hz, 2H), 4.70 (s, 2H), 3.84 (t, J = 5.8 Hz, 2H), 2.90 - 2.82 (m, 2H), 1.53 (s, 9H).

### Preparative Example 31-2: 8-(pyridin-3-yl)-2,3,4,5-tetrahydro-1H-pyrido[4,3-b]indole hydrochloride (Compound 31-3)

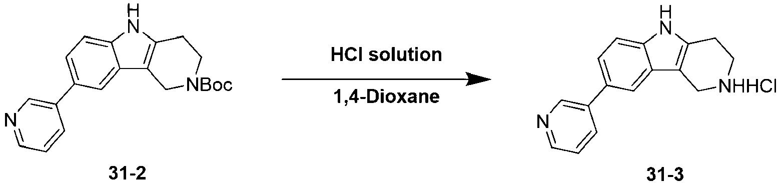

In the same method as in Preparative Example 1-4, tert-butyl 8-(pyridin-3-yl)-1,3,4,5-tetrahydro-2H-pyrido[4,3-b]indole-2-carboxyl ate (104.4 mg, 0.3 mmol) and HCl solution (4 M in dioxane) (1.49 mL, 5.98 mmol) were used to obtain 76.1 mg of the title compound (yield : 89%).

¹H NMR (400 MHz, DMSO) δ 11.50 (s, 1H), 9.21 (d, J = 2.1 Hz, 1H), 8.85 - 8.75 (m, 2H), 8.09 (d, J = 1.8 Hz, 1H), 8.03 (dd, J = 8.3, 5.5 Hz, 1H), 7.61 (dd, J = 8.5, 1.8 Hz, 1H), 7.51 (d, J = 8.5 Hz, 1H), 4.34 (d, J = 4.5 Hz, 2H), 3.48 (d, J = 6.7 Hz, 2H), 3.07 (t, J = 6.1 Hz, 2H).

### Preparative Example 31-3: Pyridin-3-yl(8-(pyridin-3-yl)-1,3,4,5-tetrahydro-2H-pyrido[4,3-b]indol-2-yl)methanone (Compound 31)

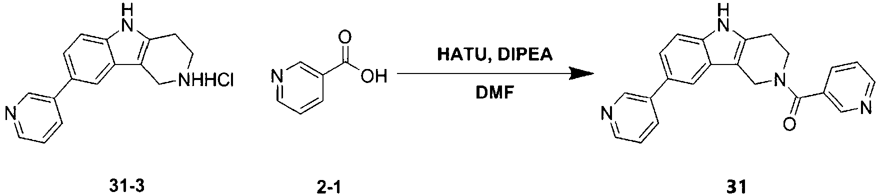

In the same method as in Preparative Example 2, 8-(pyridin-3-yl)-2,3,4,5-tetrahydro-1H-pyrido[4,3-b]indole hydrochloride (25 mg, 0.088 mmol), nicotinic acid (12.93 mg, 0.11 mmol), HATU (66.54 mg, 0.18 mmol) and DIPEA (0.031 mL, 0.18 mmol) were used to obtain 8.5 mg of the title compound (yield: 27%).

¹H NMR (400 MHz, MeOD) δ 8.85 - 8.64 (m, 3H), 8.41 (d, J = 16.6 Hz, 1H), 8.14 - 7.94 (m, 2H), 7.76 - 7.32 (m, 5H), 4.97 (s, 1H), 4.72 (s, 1H), 4.17 (s, 1H), 3.78 (s, 1H), 3.00 (s, 1H), 2.94 (s, 1H).

### Preparative Example 32: Preparation of Compound 32

### Preparative Example 32-1: tert-butyl 8-((4-chlorophenyl)amino)-1,3,4,5-tetrahydro-2H-pyrido[4,3-b]indole-2-carboxylate (Compound 32-2)

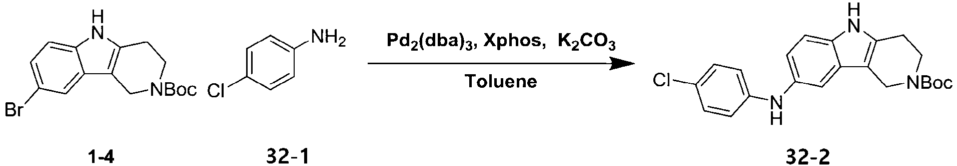

*tert-butyl* 8-bromo-1,3,4,5-tetrahydro-2H-pyrido[4,3-b]indole-2-carboxylate (200 mg, 0.57 mmol), 4-chloroaniline (290.55 mg, 2.28 mmol), Pd₂(dba)₃ (52.14 mg, 0.057 mmol), Xphos (27.14 mg, 0.057 mmol) and K₂CO₃ (314.78 mg, 2.28 mmol) were dissolved in toluene. The reaction mixture was stirred at 100 °C for 12 hours using a sealed tube. After the completion of the reaction, the solvent was extracted using distilled water and ethyl acetate, and the organic layer was dried over anhydrous magnesium sulfate and filtered. The filtrate was concentrated under reduced pressure to obtain 95 mg of the title compound (yield: 41%).

¹H NMR (400 MHz, DMSO) δ 10.78 (s, 1H), 7.88 (s, 1H), 7.22 (d, J = 8.5 Hz, 1H), 7.16 - 7.09 (m, 2H), 7.07 (s, 1H), 6.89 - 6.79 (m, 3H), 4.45 (s, 2H), 3.67 (t, J = 5.7 Hz, 2H), 2.73 (t, J = 5.6 Hz, 2H), 1.41 (s, 9H)

### Preparative Example 32-2: N-(4-chlorophenyl)-2,3,4,5-tetrahydro-1H-pyrido[4,3-B]indol-8-amine hydrochloride (Compound 32-3)

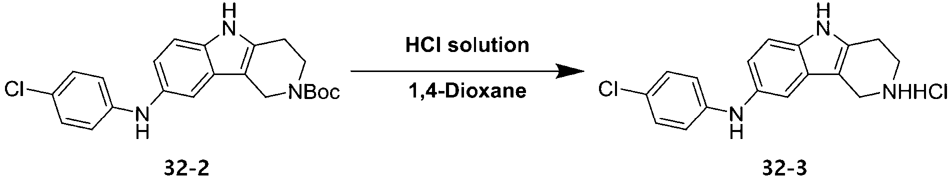

In the same method as in Preparative Example 1-4, *tert-butyl* 8-((4-chlorophenyl)amino)-1,3,4,5-tetrahydro-2H-pyrido[4,3-b]indole-2- Carboxylate (95 mg, 0.24 mmol) and HCl solution (4 M in dioxane) (1.19 mL, 4.78 mmol) were used to obtain 73 mg of the title compound (yield: 91%).

¹H NMR (400 MHz, DMSO) δ 11.07 (s, 1H), 9.26 (s, 1H), 7.30 (d, J = 8.5 Hz, 1H), 7.21 (d, J = 2.1 Hz, 1H), 7.18 - 7.11 (m, 2H), 6.89 (dd, J = 8.8, 2.4 Hz, 3H), 4.27 (s, 2H), 3.57 (s, 2H), 3.02 (t, J = 6.1 Hz, 2H), 1.18 (t, J = 7.1 Hz, 1H).

### Preparative Example 32-3: (8-((4-chlorophenyl)amino)-1,3,4,5-tetrahydro-2H-pyrido[4,3-b]indol-2-yl)(pyridin-3-yl)methanone (Compound 32)

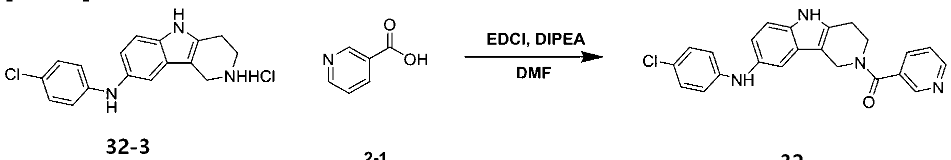

In the same method as in Preparative Example 5, N-(4-chlorophenyl)-2,3,4,5-tetrahydro-1H- pyrido [4,3-b] indol-8-amine hydrochloride (33 mg, 0.099 mmol), nicotinic acid (14.59 mg, 0.12 mmol), EDCI (75.7 mg, 0.39 mmol) and DIPEA (0.069 mL, 0.39 mmol) were used to obtain 14.1 mg of the title compound (yield : 35%).

¹H NMR (400 MHz, DMSO) δ 10.89 (s, 1H), 8.68 (s, 2H), 7.93 (s, 2H), 7.50 (s, 1H), 7.29 - 7.06 (m, 4H), 6.86 (dd, J = 27.9, 14.2 Hz, 3H), 4.76 (s, 1H), 4.54 (s, 1H), 4.02 (s, 1H), 3.65 (s, 1H), 2.87 (s, 2H).

### Preparative Example 33: (8-((4-chlorophenyl)amino)-1,3,4,5-tetrahydro-2H-pyrido[4,3-b]indol-2-yl)(phenyl)methanone (Compound 33)

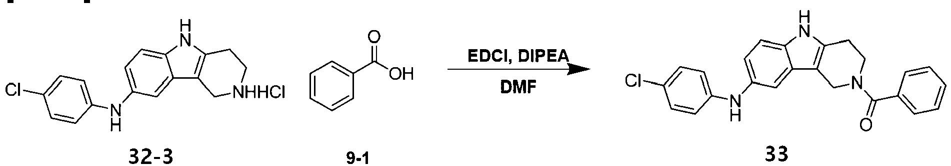

In the same manner as in Preparative Example 5, N-(4-chlorophenyl)-2,3,4,5-tetrahydro-1H- pyrido [4,3-b] indol-8-amine hydrochloride (20 mg, 0.06 mmol), benzoic acid (8.77 mg, 0.072 mmol), EDCI (45.89 mg, 0.24 mmol) and DIPEA (0.042 mL, 0.24 mmol) were used to obtain 4.9 mg of the title compound (yield: 20%).

¹H NMR (400 MHz, DMSO) δ 10.88 (s, 1H), 7.94 (s, 1H), 7.48 (s, 4H), 7.21 (d, J = 41.7 Hz, 4H), 6.88 (s, 4H), 4.75 (s, 2H), 4.03 (s, 1H), 3.65 (s, 1H), 2.86 (s, 2H).

### Preparative Example 34: Preparation of Compound 34

### Preparative Example 34-1: 7-bromo-2,3,4,5-tetrahydro-1H-pyrido[4,3-b]indole / 9-bromo-2,3,4,5-tetrahydro-1H-pyrido[4,3-b]indole (Compound 34-2/34-3)

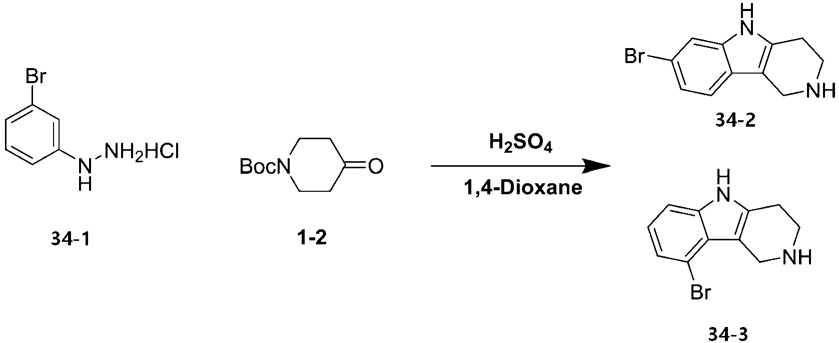

To a solution of (3-Bromophenyl)hydrazine hydrochloride (2 g, 8.95 mmol) and *tert-*butyl 4-oxopiperidine-1-carboxylate (1.87 g, 9.4 mmol) in 1,4-dioxane was added conc. H₂SO₄ (1.12 mL, 8 M) at 0 °C. Then the reaction mixture was heated at 110 °C for 3 hours using a sealed tube. After the reaction was completed, the precipitate was filtered with 1,4-dioxane. The precipitate was dissolved in distilled water and then basified with 1N sodium hydroxide solution. The mixture was extracted using distilled water and dichloromethane, and the organic layer was dried over anhydrous magnesium sulfate and filtered. The filtrate was concentrated under reduced pressure to obtain 552 mg of the mixture of regioisomers.

### Preparative Example 34-2: tert-butyl 7-bromo-1,3,4,5-tetrahydro-2H-pyrido[4,3-b]indole-2-carboxylate (Compound 34-4)

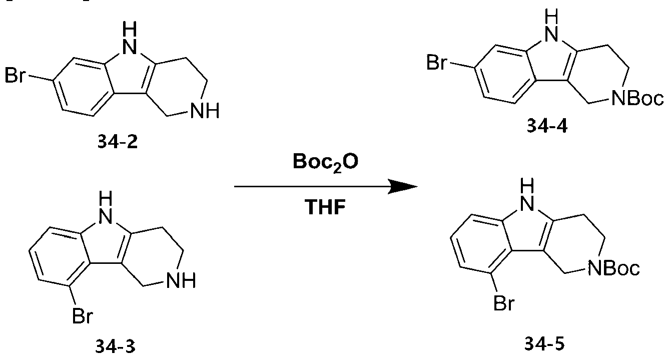

Regioisomers (33-2 and 33-3) (1.13 g, 4.51 mmol) were dissolved in THF solution under nitrogen, and then Boc₂O (0.56 mL, 2.42 mmol) was added. The reaction mixture was stirred at room temperature for 12 hours. After the completion of the reaction, the solvent was extracted using distilled water and ethyl acetate, and the organic layer was dried over anhydrous magnesium sulfate and filtered. The filtrate was concentrated under reduced pressure, and the concentrate was subjected to column chromatography to obtain 354.1 mg of the title compound (yield : 45%).

¹H NMR (400 MHz, DMSO) δ 11.38 (s, 1H), 7.76 (d, J = 1.7 Hz, 1H), 7.65 (d, J = 8.3 Hz, 1H), 7.38 (dd, J = 8.4, 1.8 Hz, 1H), 4.80 (s, 2H), 3.99 (t, J = 5.7 Hz, 2H), 3.05 (t, J = 5.8 Hz, 2H), 1.73 (s, 9H).

### Preparative Example 34-3: tert-butyl 7-phenyl-1,3,4,5-tetrahydro-2H-pyrido[4,3-b]indole-2-carboxylate (Compound 34-6)

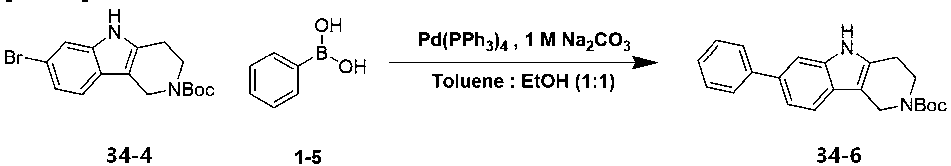

In the same method as in Preparative Example 1-3, *tert-butyl* 7-bromo-1,3,4,5-tetrahydro-2H-pyrido[4,3-b]indole-2-carboxylate (200 mg , 0.57 mmol), phenylboronic acid (208.28 mg, 1.71 mmol), Pd(PPh₃)₄ (33 mg, 0.028 mmol) and 1M Na₂CO₃ (1.42 mL, 1.42 mmol) were used to obtain 105.4 mg of the title compound (yield: 53%).

¹H NMR (400 MHz, DMSO) δ 11.01 (s, 1H), 7.70 - 7.64 (m, 2H), 7.55 (d, J = 1.6 Hz, 1H), 7.45 (td, J = 7.8, 2.9 Hz, 3H), 7.35 - 7.25 (m, 2H), 4.56 (s, 2H), 3.73 (t, J = 5.7 Hz, 2H), 2.81 (t, J = 5.8 Hz, 2H), 1.46 (s, 9H), 1.30 (s, 1H).

### Preparative Example 34-4: 7-phenyl-2,3,4,5-tetrahydro-1H-pyrido[4,3-b]indole hydrochloride (Compound 34-7)

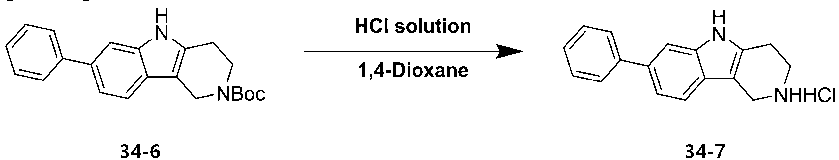

In the same method as in Preparative Example 1-4, *tert-butyl* 7-phenyl-1,3,4,5-tetrahydro-2H-pyrido[4,3-b]indole-2-carboxylate (105.4 mg, 0.3 mmol) and HCl solution (4 M in dioxane) (1.51 mL, 6.05 mmol) were used to obtain 65 mg of the title compound (yield: 75%).

¹H NMR (400 MHz, DMSO) δ 11.27 (s, 1H), 9.33 (s, 2H), 7.70 - 7.66 (m, 2H), 7.61 (d, J = 1.6 Hz, 1H), 7.56 - 7.53 (m, 1H), 7.46 (t, J = 7.7 Hz, 3H), 7.37 - 7.31 (m, 2H), 4.34 (s, 2H), 3.49 (d, J = 6.6 Hz, 2H), 3.07 (t, J = 6.0 Hz, 2H).

### Preparative Example 34-5: 7-phenyl-2-(pyridin-3-ylsulfonyl)-2,3,4,5-tetrahydro-1H-pyrido[4,3-b]indole (Compound 34)

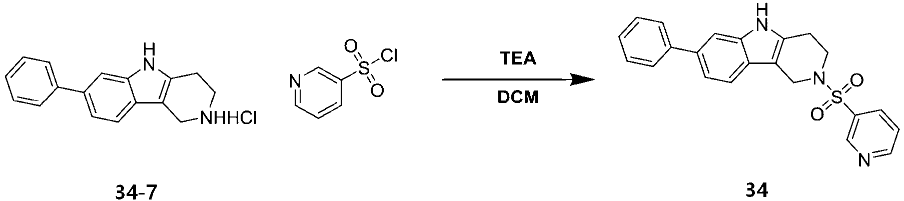

To a solution of 7-phenyl-2,3,4,5-tetrahydro-1H-pyrido[4,3-b]indole hydrochloride (30 mg, 0.11 mmol) in dichloromethane was added pyridine-3-sulphonyl chloride (0.01 mL, 0.1 mmol) and triethylamine (0.029 mL, 0.2 mmol) at °C. The reaction mixture was stirred at room temperature for 3 hours. After the completion of the reaction, the solvent was extracted using distilled water and ethyl acetate, and the organic layer was dried over anhydrous magnesium sulfate and filtered. The filtrate was concentrated under reduced pressure, and the concentrate was subjected to column chromatography to obtain 5.7 mg of the title compound (yield: 14%).

¹H NMR (400 MHz, DMSO) δ 11.01 (s, 1H), 9.03 (dd, J = 2.3, 0.8 Hz, 1H), 8.85 (dd, J = 4.8, 1.6 Hz, 1H), 8.28 (ddd, J = 8.1, 2.4, 1.6 Hz, 1H), 7.66 (ddd, J = 8.1, 2.9, 1.6 Hz, 3H), 7.55 - 7.48 (m, 2H), 7.47 - 7.41 (m, 2H), 7.31 (ddd, J = 8.3, 6.8, 1.3 Hz, 2H), 4.41 (s, 2H), 3.56 (t, J = 5.8 Hz, 2H), 2.85 (t, J = 5.7 Hz, 2H).

### Preparative Example 35: (7-phenyl-1,3,4,5-tetrahydro-2H-pyrido[4,3-b]indol-2-yl)(pyridin-3-yl)methanone (Compound 35)

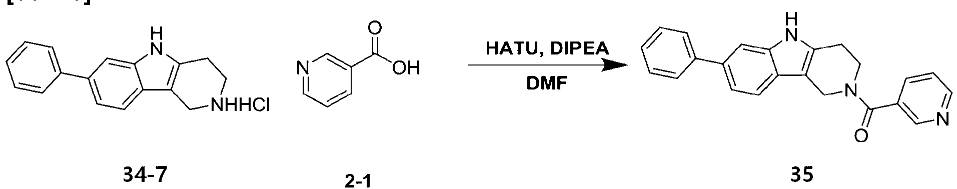

In the same method as in Preparative Example 2, 7-phenyl-2,3,4,5-tetrahydro-1H-pyrido[4,3-b]indole hydrochloride (25 mg, 0.088 mmol), nicotinic acid (12.97 mg, 0.11 mmol), HATU (66.77 mg, 0.18 mmol) and DIPEA (0.031 mL, 0.18 mmol) were used to obtain 13.3 mg of the title compound (yield: 42%).

¹H NMR (400 MHz, DMSO) δ 11.08 (s, 1H), 8.69 (d, J = 4.7 Hz, 2H), 7.94 (d, J = 8.5 Hz, 1H), 7.70 - 7.61 (m, 2H), 7.59 - 7.49 (m, 3H), 7.44 (t, J = 7.4 Hz, 3H), 7.31 (d, J = 6.6 Hz, 2H), 4.84 (s, 1H), 4.62 (s, 1H), 4.05 (s, 1H), 3.68 (s, 1H), 2.92 (s, 2H).

### Preparative Example 36: Cyclohexyl(8-(p-tolyl)-1,3,4,5-tetrahydro-2H-pyrido[4,3-b]indol-2-yl)methanone (Compound 36)

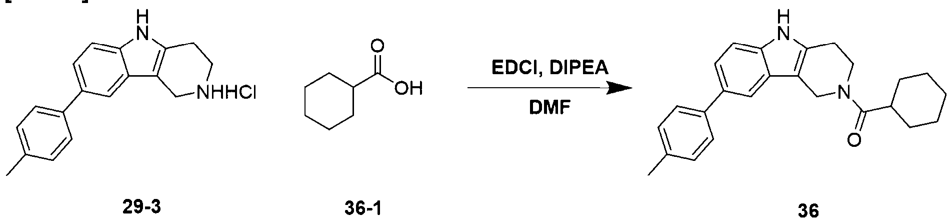

In the same method as in Preparative Example 5, 8-(p-Tolyl)-2,3,4,5-tetrahydro-1H-pyrido[4,3-b]indole hydrochloride (30 mg, 0.10 mmol), cyclohexanecarboxylic acid (15.44 mg, 0.12 mmol), EDCI (76.99 mg, 0.40 mmol) and DIPEA (0.07 mL, 0.40 mmol) were used to obtain 6.8 mg (18% yield) of the title compound.

¹H NMR (400 MHz, DMSO) δ 10.93 (s, 1H), 7.70 (d, J = 40.7 Hz, 1H), 7.56 (d, J = 7.2 Hz, 2H), 7.33 (s, 2H), 7.23 (s, 2H), 4.70 (d, J = 38.8 Hz, 2H), 3.84 (s, 2H), 2.86 (s, 1H), 2.76 - 2.70 (m, 2H), 2.33 (s, 3H), 1.98 (s, 1H), 1.66 (d, J = 21.3 Hz, 6H), 1.35 (s, 4H).

### Preparative Example 37: p-Tolyl(8-(p-Tolyl)-1,3,4,5-tetrahydro-2H-pyrido[4,3-b]indol-2-yl)methanone (Compound 37)

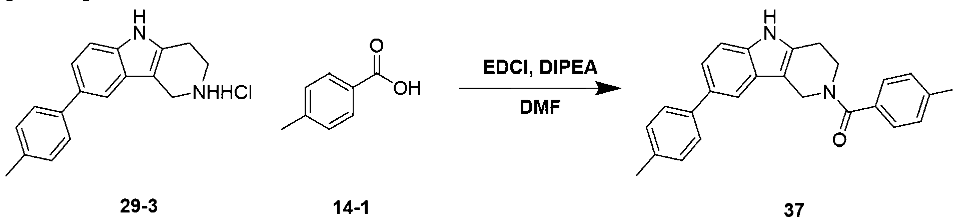

In the same method as in Preparative Example 5, 8-(p-Tolyl)-2,3,4,5-tetrahydro-1H-pyrido[4,3-b]indole hydrochloride (20 mg, 0.07 mmol), 4-methylbenzoic acid (10.94 mg, 0.08 mmol), EDCI (51.3 mg, 0.27 mmol) and DIPEA (0.047 mL, 0.27 mmol) were used to obtain 13.7 mg (yield 53%) of the title compound.

¹H NMR (400 MHz, DMSO) δ 10.99 (s, 1H), 7.65 - 7.19 (m, 11H), 4.73 (d, J = 60.4 Hz, 2H), 3.67 (s, 2H), 2.87 (s, 2H), 2.34 (d, J = 14.5 Hz, 6H).

### Preparative Example 38: m-Tolyl(8-(p-Tolyl)-1,3,4,5-tetrahydro-2H-pyrido[4,3-b]indol-2-yl)methanone (Compound 38)

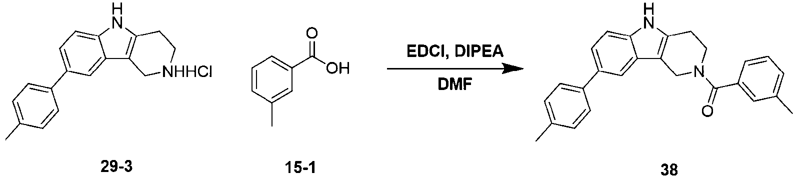

In the same method as in Preparative Example 5, 8-(p-Tolyl)-2,3,4,5-tetrahydro-1H-pyrido[4,3-b]indole hydrochloride (20 mg, 0.07 mmol), 3-methylbenzoic acid (10.94 mg, 0.08 mmol), EDCI (51.3 mg, 0.27 mmol) and DIPEA (0.047 mL, 0.27 mmol) were used to obtain 10 mg (39% yield) of the title compound.

¹H NMR (400 MHz, DMSO) δ 11.00 (s, 1H), 7.76 - 7.15 (m, 11H), 4.72 (d, J = 82.2 Hz, 2H), 4.02 (d, J=8.4 Hz, 1H), 3.66 (s, 1H), 2.87 (s, 2H), 2.44 - 2.24 (m, 6H).

### Preparative Example 39: (4-methoxyphenyl)(8-(p-tolyl)-1,3,4,5-tetrahydro-2H-pyrido[4,3-b]indol-2-yl)methanone (Compound 39)

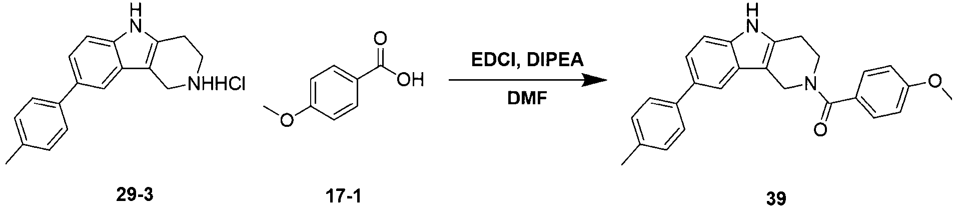

In the same method as in Preparative Example 5, 8-(p-Tolyl)-2,3,4,5-tetrahydro-1H-pyrido[4,3-b]indole hydrochloride (20 mg, 0.07 mmol), 4-methoxybenzoic acid (12.22 mg, 0.08 mmol), EDCI (51.3 mg, 0.27 mmol) and DIPEA (0.047 mL, 0.27 mmol) were used to obtain 11.7 mg of the title compound (44% yield).

¹H NMR (400 MHz, DMSO) δ 11.00 (s, 1H), 7.55 (s, 2H), 7.46 (d, J = 8.2 Hz, 3H), 7.34 (s, 2H), 7.21 (s, 2H), 7.01 (d, J = 8.3 Hz, 2H), 4.75 (s, 2H), 3.81 (s, 5H), 2.88 (s, 2H), 2.32 (s, 3H).

### Preparative Example 40: (6-methylpyridin-3-yl)(8-(p-tolyl)-1,3,4,5-tetrahydro-2H-pyrido[4,3-b]indol-2-yl)methanone (Compound 40)

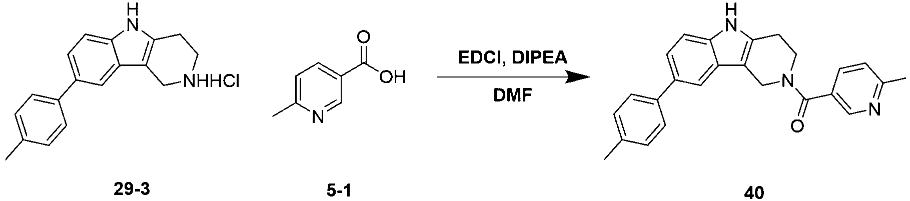

In the same method as in Preparative Example 5, 8.4 mg (32% yield) of the title compound was obtained using 8-(p-Tolyl)-2,3,4,5-tetrahydro-1H-pyrido[4,3-b]indole hydrochloride (20 mg, 0.07 mmol), 6-methylnicotinic acid (11.015 mg, 0.08 mmol), EDCI (51.3 mg, 0.27 mmol) and DIPEA (0.047 mL, 0.27 mmol) were used to obtain 8.4 mg of the title compound (32% yield).

¹H NMR (400 MHz, DMSO) δ 11.03(s, 1H), 8.58(s, 1H), 7.83(s, 2H), 7.58(d, J=25.9 Hz, 2H), 7.37(s, 3H), 7.24(s, 2H), 4.86(s, 1H), 4.69(s, 1H), 4.04(d, J=6.6 Hz, 1H), 3.70(s, 1H), 2.92(s, 2H), 2.55(s, 3H), 2.34(s, 3H).

### Preparative Example 41: (2-methylpyridin-4-yl)(8-(p-tolyl)-1,3,4,5-tetrahydro-2H-pyrido[4, 3-b]indol-2-yl)methanone (Compound 41)

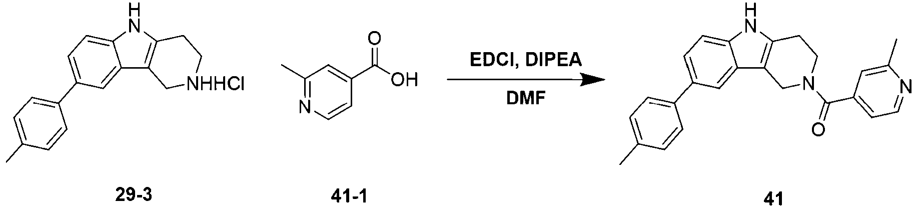

In the same method as in Preparative Example 5, 8-(p-Tolyl)-2,3,4,5-tetrahydro-1H-pyrido[4,3-b]indole hydrochloride (20 mg, 0.07 mmol), 2-methylisonicotinic acid (11.015 mg, 0.08 mmol), EDCI (51.3 mg, 0.27 mmol) and DIPEA (0.047 mL, 0.27 mmol) were used to obtain 5.1 mg of the title compound(yield 19%).

¹H NMR (400 MHz, DMSO) δ 11.00 (s, 1H), 8.54 (s, 1H), 7.74 (s, 1H), 7.59 (d, J = 7.9 Hz, 1H), 7.48 (d, J = 8.5 Hz, 1H), 7.36 - 7.17 (m, 6H), 4.85 (s, 1H), 4.55 (s, 1H), 4.02 (s, 1H), 3.60 (s, 1H), 2.87 (d, J = 18.6 Hz, 2H), 2.52 (s, 3H), 2.31 (d, J = 13.7 Hz, 3H).

### Preparative Example 42: Phenyl(8-(pyridin-3-yl)-1,3,4,5-tetrahydro-2H-pyrido[4,3-b]indol-2-yl)methanone (Compound 42)

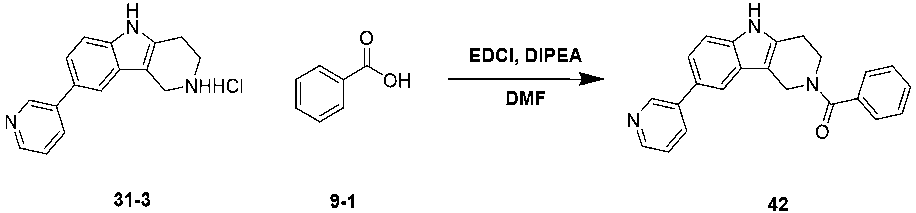

In the same method as in Preparative Example 5, 8-phenyl-2,3,4,5-tetrahydro-1H-pyrido[4,3-b]indole hydrochloride (30 mg, 0.11 mmol), benzoic acid (15.39 mg, 0.13 mmol), EDCI (80.51 mg, 0.42 mmol) and DIPEA (0.073 mL, 0.42 mmol) were used to obtain 13.9 mg (37% yield) of the title compound.

¹H NMR (400 MHz, DMSO) δ 11.11 (s, 1H), 8.88 (d, J = 40.9 Hz, 1H), 8.48 (s, 1H), 8.05 (d, J = 40.5 Hz, 1H), 7.87 (s, 1H), 7.44 (d, J = 24.4 Hz, 8H), 4.85 (s, 1H), 4.65 (s, 1H), 4.02 (s, 1H), 3.66 (s, 1H), 2.88 (s, 2H).

### Preparative Example 43: Preparation of Compound 43

### Preparative Example 43-1: tert-Butyl 5-methyl-8-(p-tolyl)-1,3,4,5-tetrahydro-2H-pyrido[4,3-b]indole-2-carboxylate (Compound 43-1)

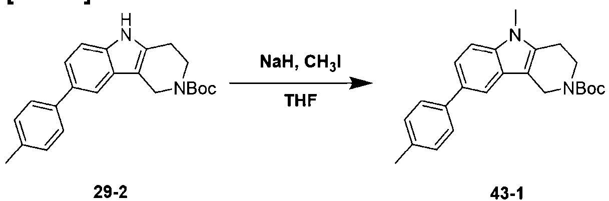

Under nitrogen, *tert-butyl* 8-(p-tolyl)-1,3,4,5-tetrahydro-2H-pyrido[4,3-b]indole-2-carboxylate (100 mg, 0.28 mmol) was dissolved in THF. To this was added NaH (22.07 mg, 0.55 mmol) and CH₃I(0,018 mL,0.28 mmol) at 0 °C . The reaction mixture was stirred at room temperature for 6 hours. Upon completion of the reaction, it was extracted with distilled water and ethyl acetate, and the organic layer was dried over magnesium anhydride and filtered. The filtrate was concentrated under reduced pressure, and the concentrate was purified by column chromatography to obtain 68.3 mg (65% yield) of the title compound.

¹H NMR (400 MHz, CDCl3) δ7.63(d, J=1.7Hz, 1H), 7.54(d, J=7.7Hz, 2H), 7.41(dd, J=8.6, 1.7Hz, 1H), 7.26(dd, J=21.2, 8.1Hz, 3H), 4.67(s, 2H), 3.83(s, 2H), 3.60(s, 3H), 2.78(t, J=5.9Hz, 2H), 2.38(s, 3H), 1.50(s, 9H), 1.30(d, J=13.9Hz, 9H).

### Preparative Example 43-2: 5-methyl-8-(p-tolyl)-2,3,4,5-tetrahydro-1H-pyrido[4,3-b]indole hydrochloride (Compound 43-2)

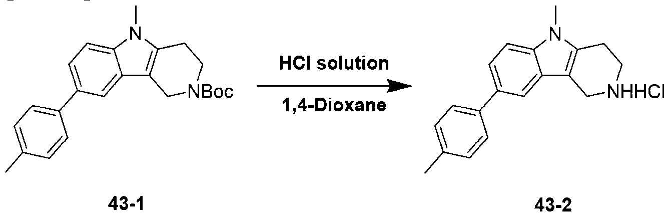

In the same method as in Preparative Examples 1-4, *tert*-butyl 5-methyl-8-(p-tolyl)-1,3,4,5-tetrahydro-2H-pyrido[4,3-b]indole-2-carboxylate (68.3 mg, 0.18 mmol) and HCl solution (4M in dioxane) (0.91 mL, 3.63 mmol) were used to obtain 48.7 mg (85% yield) of the title compound.

¹H NMR (400 MHz, DMSO) δ 9.19 (s, 1H), 7.78 (s, 1H), 7.59 (d, J = 7.8 Hz, 2H), 7.56 - 7.43 (m, 2H), 7.25 (d, J = 7.8 Hz, 2H), 4.37 (s, 2H), 3.69 (s, 3H), 3.53 (s, 2H), 3.07 (s, 2H), 2.34 (s, 3H).

### Preparative Example 43-3: (5-methyl-8-(p-tolyl)-1,3,4,5-tetrahydro-2H-pyrido[4,3-b]indol-2-yl)(phenyl)methanone (Compound 43)

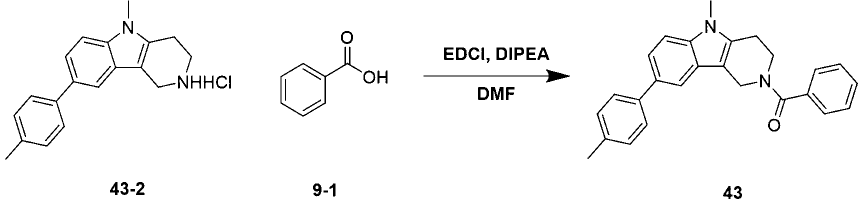

In the same method as in Preparative Example 5, 5-methyl-8-(p-tolyl)-2,3,4,5-tetrahydro-1H-pyrido[4,3-b]indole hydrochloride (22.5 mg, 0.072 mmol), benzoic acid (10.54 mg, 0.086 mmol), EDCI (55.15 mg, 0.29 mmol) and DIPEA (0.05 mL, 0.29 mmol) were used to obtain 12.4 mg of the title compound (45% yield).

¹H NMR (400 MHz, DMSO) δ 7.78 (s, 1H), 7.54 (d, J = 52.7 Hz, 9H), 7.23 (s, 2H), 4.84 (s, 1H), 4.64 (s, 1H), 4.02 (d, J = 11.0 Hz, 1H), 3.65 (s, 4H), 2.91 (s, 2H), 2.32 (s, 3H).

### Preparative Example 44: (5-methyl-8-(p-tolyl)-1,3,4,5-tetrahydro-2H-pyrido[4,3-b]indol-2-yl)(pyridin-3-yl)methanone (Compound 44)

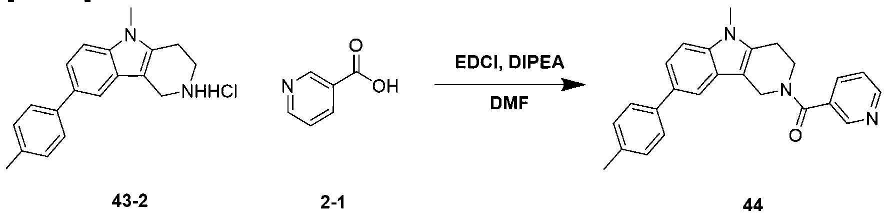

In the same method as in Preparative Example 5, 5-methyl-8-(p-tolyl)-2,3,4,5-tetrahydro-1H-pyrido[4,3-b]indole hydrochloride (26.2 mg, 0.084 mmol), nicotinic acid (12.37 mg, 0.1 mmol), EDCI (64.22 mg, 0.34 mmol) and DIPEA (0.058 mL, 0.34 mmol) were used to obtain 7.4 mg of the title compound (yield 23%).

¹H NMR (400 MHz, DMSO) δ 8.70 (s, 2H), 7.93 (s, 1H), 7.48 (d, J = 29.6 Hz, 6H), 7.26 - 7.18 (m, 2H), 4.88 (s, 1H), 4.67 (s, 1H), 4.07 (s, 1H), 3.67 (s, 4H), 2.94 (s, 2H), 2.34 (s, 3H).

### Preparative Example 45: Preparation of Compound 45

### Preparative Example 45-1: tert-butyl 5-ethyl-8-(p-tolyl)-1,3,4,5-tetrahydro-2H-pyrido[4, 3-b]indole-2-carboxylate (Compound 45-1)

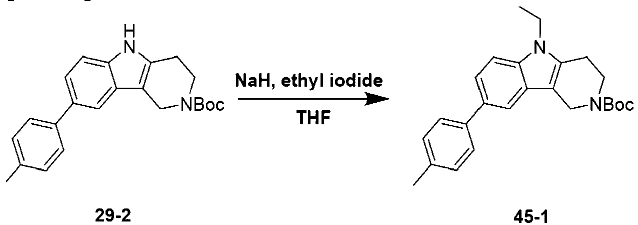

Under nitrogen, *tert-butyl* 8-(p-tolyl)-1,3,4,5-tetrahydro-2H-pyrido[4,3-b]indole-2-carboxylate (200 mg, 0.55 mmol) was dissolved in THF. To this NaH (44.14 mg, 1.1 mmol) and ethyl iodine (0.045 mL, 0.56 mmol) were added at 0°C. The reaction mixture was stirred at room temperature for 4 hours. Upon completion of the reaction, it was extracted with distilled water and ethyl acetate, and the organic layer was dried over magnesium anhydride and filtered. The filtrate was concentrated under reduced pressure, and the concentrate was purified by column chromatography to obtain the title compound 75.9 mg (yield 35%).

¹H NMR (400 MHz, DMSO) δ 7.68 (s, 1H), 7.59 (d, J = 7.8 Hz, 2H), 7.49 (d, J = 8.6 Hz, 1H), 7.44 - 7.37 (m, 1H), 7.25 (d, J = 7.8 Hz, 2H), 4.60 (s, 2H), 4.15 (q, J = 7.1 Hz, 2H), 3.76 (t, J = 5.6 Hz, 2H), 2.83 (s, 2H), 2.35 (s, 3H), 1.46 (s, 9H), 1.25 (t, J = 7.1 Hz, 3H).

### Preparative Example 45-2: 5-ethyl-8-(p-tolyl)-2,3,4,5-tetrahydro-1H-pyrido[4,3-b]indole hydrochloride (Compound 45-2)

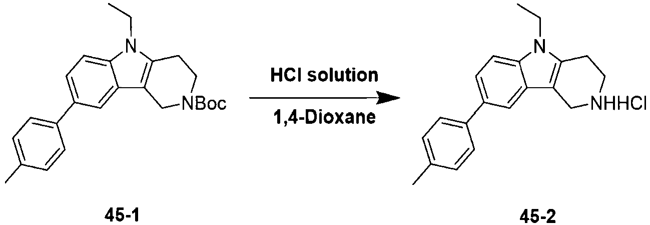

In the same method as in Preparative Examples 1-4, tert-butyl 5-ethyl-8-(p-tolyl)-1,3,4,5-tetrahydro-2H-pyrido[4,3-b]indole-2-carboxylate (75.9 mg, 0.19 mmol) and HCl solution (4 M in dioxane) (0.97 mL, 3.89 mmol) were used to obtain 40.5 mg (63% yield) of the title compound.

¹H NMR (400 MHz, DMSO) δ 9.31 (s, 1H), 7.79 (d, J = 1.8 Hz, 1H), 7.58 (dd, J = 16.5, 8.2 Hz, 3H), 7.47 (dd, J = 8.7, 1.8 Hz, 1H), 7.27 (d, J = 7.8 Hz, 2H), 4.38 (s, 2H), 4.19 (d, J = 7.4 Hz, 2H), 3.54 (s, 2H), 3.11 (d, J = 6.6 Hz, 2H), 2.35 (s, 3H), 1.27 (t, J = 7.1 Hz, 3H).

### Preparative Example 45-3: (5-ethyl-8-(p-tolyl)-1,3,4,5-tetrahydro-2H-pyrido[4,3-b]indol-2-yl)(phenyl)methanone (Compound 45)

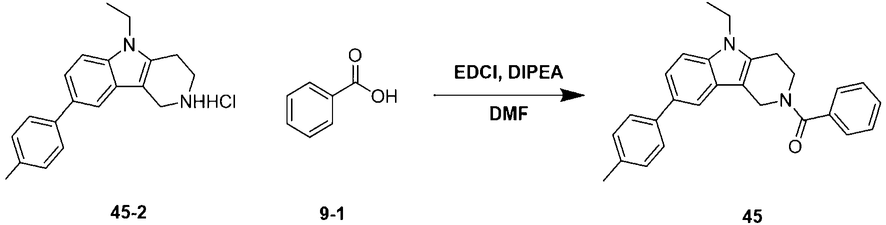

In the same method as in Preparative Example 5, 5-ethyl-8-(p-tolyl)-2,3,4,5-tetrahydro-1H-pyrido[4,3-b]indole hydrochloride (20 mg, 0.061 mmol), benzoic acid (8.97 mg, 0.073 mmol), EDCI (46.93 mg, 0.24 mmol) and DIPEA (0.043 mL, 0.24 mmol) were used to obtain 15.3 mg (63% yield) of the title compound.

¹H NMR (400 MHz, CDCl3) δ 7.64 - 7.20 (m, 13H), 5.04 (s, 1H), 4.73 (s, 1H), 4.23 (s, 1H), 3.83 (s, 1H), 2.95 (d, J = 43.6 Hz, 2H), 2.41 (s, 3H), 1.41 (t, J = 7.2 Hz, 3H).

### Preparative Example 46: (5-ethyl-8-(p-tolyl)-1,3,4,5-tetrahydro-2H-pyrido[4,3-b]indol-2-yl)(pyridin-3-yl)methanone (Compound 46)

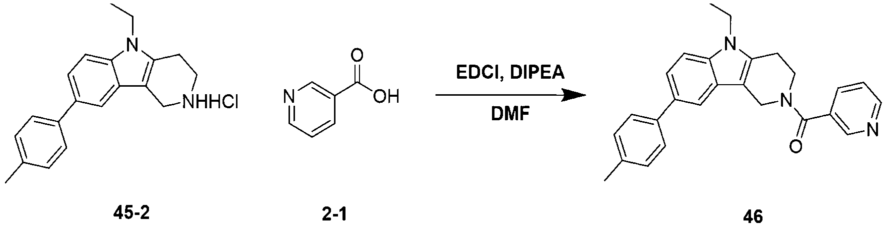

In the same method as in Preparative Example 5, a solution of 5-ethyl-8-(p-tolyl)-2,3,4,5-tetrahydro-1H-pyrido[4,3-b]indole hydrochloride (20 mg, 0.061 mmol), nicotinic acid (9.004 mg, 0.073 mmol), EDCI (46.93 mg, 0.24 mmol) and DIPEA (0.043 mL, 0.24 mmol) were used to obtain 14.5 mg of the title compound (59% yield).

¹H NMR (400 MHz, CDCl₃) 68.80(s, 1H), 8.73(s, 1H), 7.86(s, 1H), 7.59-7.39(m, 6H), 7.24(d, J=8.1Hz, 2H), 5.05(s, 1H), 4.76(s, 1H), 4.24(s, 1H), 4.16(t, J=7.2Hz, 2H), 3.84(s, 1H), 2.97(d, J=33.3Hz, 2H), 2.42(d, J=9.3Hz, 3H), 1.41(t, J=7.2Hz, 3H).

### Preparative Example 47: Preparation of Compound 47

### Preparative Example 47-1: tert-Butyl 8-(6-methylpyridin-3-yl)-1,3,4,5-tetrahydro-2H-pyrido[4,3-b]indole-2-carboxylate (Compound 47-2)

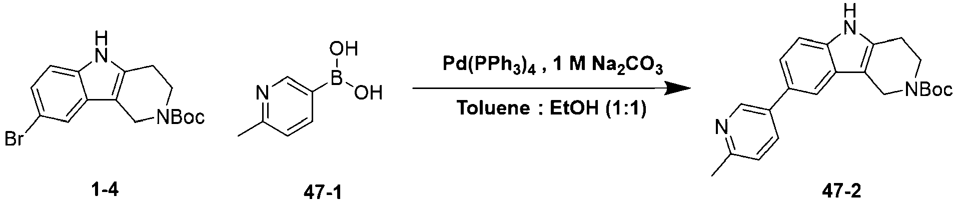

In the same method as in Preparative Examples 1-3, *tert-butyl* 8-bromo-1,3,4,5-tetrahydro-2H-pyrido[4,3-b]indole-2-carboxylate (304.5 mg, 0.87 mmol), (6-methylpyridin-3-yl)boronic acid (356.14 mg, 2.6 mmol), Pd(PPh₃)₄ (50.094 mg,0.043 mmol) and 1 M Na₂CO₃ (2.17 mL, 2.17 mmol) were used to obtain 216.2 mg (yield 68%) of the title compound.

¹H NMR (400 MHz, DMSO) δ 11.01 (s, 1H), 8.76 (d, J = 2.5 Hz, 1H), 7.96 (dd, J = 8.1, 2.4 Hz, 1H), 7.72 (s, 1H), 7.37 (d, J = 2.5 Hz, 2H), 7.29 (d, J = 8.0 Hz, 1H), 4.58 (s, 2H), 3.71 (d, J = 6.0 Hz, 2H), 3.16 (d, J = 5.2 Hz, 3H), 2.78 (s, 2H), 1.44 (s, 9H).

### Preparative Example 47-2: 8-(6-methylpyridin-3-yl)-2,3,4,5-tetrahydro-1H-pyrido[4,3-b]indole hydrochloride (Compound 47-3)

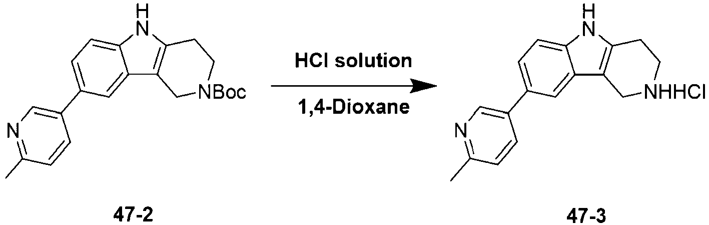

In the same method as in Preparation Examples 1-4, *tert-butyl* 8-(6-methylpyridin-3-yl)-1,3,4,5-tetrahydro-2H-pyrido[4,3-b]indole-2-carboxylate (216.2 mg, 0.59 mmol) and HCl solution (4 M in dioxane) (2.97 mL, 11.896 mmol) were used to obtain 178.32 mg (100% yield) of the title compound.

¹H NMR (400 MHz, DMSO) δ 11.44 (s, 1H), 9.34 (s, 1H), 9.05 (s, 1H), 8.69 (s, 1H), 8.05 (s, 1H), 7.89 (d, J = 8.4 Hz, 1H), 7.59 (d, J = 8.7 Hz, 1H), 7.51 (d, J = 8.5 Hz, 1H), 4.36 (s, 2H), 3.51 (s, 2H), 3.07 (t, J = 5.9 Hz, 2H), 2.73 (s, 2H).

### Preparative Example 47-3: (8-(6-methylpyridin-3-yl)-1,3,4,5-tetrahydro-2H-pyrido[4,3-b]indol-2-yl)(phenyl)methanone (Compound 47)

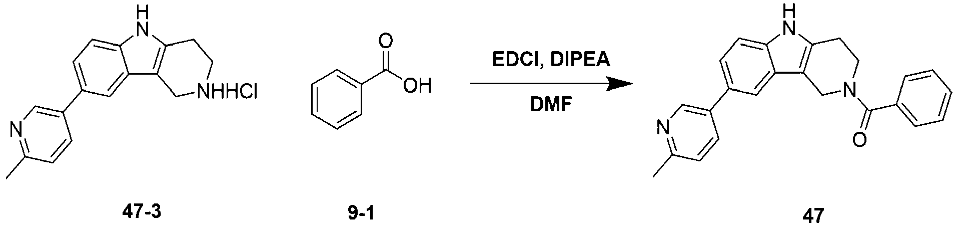

In the same method as in Preparative Example 5, 8-(6-methylpyridin-3-yl)-2,3,4,5-tetrahydro-1H-pyrido[4,3-b]indole hydrochloride (30 mg, 0.1 mmol), benzoic acid (14.67 mg, 0.12 mmol), EDCI (76.76 mg, 0.4 mmol) and DIPEA (0.07 mL, 0.4 mmol) were used to obtain 18.4 mg (yield 50%) of the title compound.

¹H NMR (400 MHz, DMSO) δ 11.07 (s, 1H), 8.72 (d, J = 40.7 Hz, 1H), 7.98 (s, 1H), 7.82 (s, 1H), 7.42 (d, J = 36.0 Hz, 8H), 4.83 (s, 1H), 4.63 (s, 1H), 4.00 (t, J = 7.2 Hz, 1H), 3.65 (s, 1H), 2.87 (s, 2H).

### Preparative Example 48: (8-(6-methylpyridin-3-yl)-1,3,4,5-tetrahydro-2H-pyrido[4,3-b]indol-2-yl)(pyridin-3-yl)methanone (Compound 48)

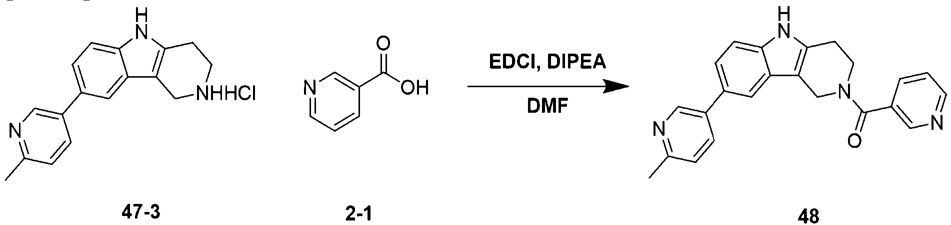

In the same method as in Preparative Example 5, 8-(6-methylpyridin-3-yl)-2,3,4,5-tetrahydro- 1H-pyrido[4,3-b]indole hydrochloride (30 mg, 0.1 mmol), nicotinic acid (14.79 mg, 0.12 mmol), EDCI (76.76 mg, 0.4 mmol) and DIPEA (0.07 mL, 0.4 mmol) were used to obtain 14.1 mg (yield 38%) of the above title compound.

¹H NMR (400 MHz, DMSO) δ 11.10 (s, 1H), 8.75 (d, J = 40.5 Hz, 3H), 8.02 - 7.84 (m, 2H), 7.65 - 7.20 (m, 5H), 4.88 (s, 1H), 4.67 (s, 1H), 4.07 - 4.00 (m, 1H), 3.68 (s, 1H), 2.92 (s, 2H), 2.46 (s, 3H).

### Preparative Example 49: (6-methylpyridin-3-yl)(8-(6-methylpyridin-3-yl)-1,3,4,5-tetrahydro-2H-pyrido[4,3-b]indol-2-yl)methanone (Compound 49)

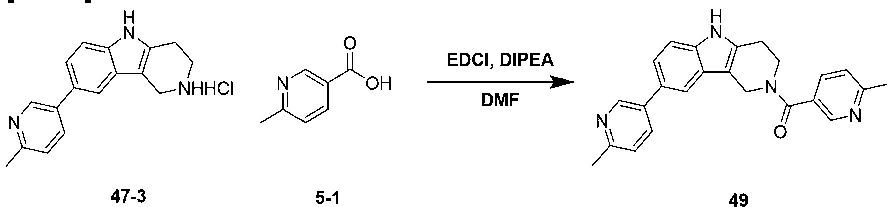

In the same method as in Preparative Example 5, 8-(6-methylpyridin-3-yl)-2,3,4,5-tetrahydro-1H-pyrido[4,3-b]indole hydrochloride (30 mg, 0.1 mmol), 6-methylnicotinic acid (16.47 mg, 0.12 mmol), EDCI (76.76 mg, 0.4 mmol) and DIPEA (0.07 mL, 0.4 mmol) were used to obtain 17.1 mg (yield 44%) of the title compound.

¹H NMR (400 MHz, DMSO) δ 11.10 (s, 1H), 8.76 (d, J = 33.2 Hz, 1H), 8.58 (s, 1H), 7.88 (d, J=31.4 Hz, 3H), 7.43 - 7.25 (m, 4H), 4.87 (s, 1H), 4.70 (s, 1H), 4.03 (t, J=7.1 Hz, 1H), 3.70 (s, 1H), 2.92 (s, 2H), 2.54 (s, 6H).

### Preparative Example 50: Preparation of Compound 50

### Preparative Example 50-1: tert-Butyl 8-(4-cyanophenyl)-1,3,4,5-tetrahydro-2H-pyrido[4,3-b]indole-2-carboxylate (Compound 50-2)

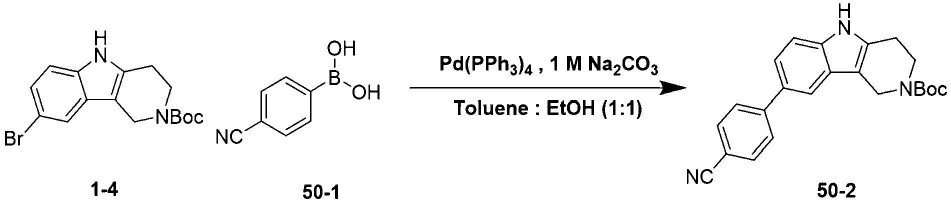

In the same manner as in Examples 1-3, *tert-butyl* 8-bromo-1,3,4,5-tetrahydro-2H-pyrido[4,3-b]indole-2-carboxylate (1 g, 2.85 mmol), (4-cyanophenyl)boronic acid (1.26 g, 8.54 mmol), Pd(PPh₃)₄ (164.55 mg,0.14 mmol) and 1 M Na₂CO₃ (7.12 mL, 7.12 mmol) were used to obtain 427.9 mg (40% yield) of the title compound.

¹H NMR (400 MHz, DMSO) δ 11.09 (s, 1H), 7.93-7.82 (m, 5H), 7.48-7.37 (m, 2H), 4.59 (s, 2H), 3.71 (t, J=5.7 Hz, 2H), 2.79 (d, J=6.1 Hz, 2H), 1.44 (s, 9H).

### Preparative Example 50-2: 4-(2,3,4,5-tetrahydro-1H-pyrido[4,3-b]indol-8-yl)benzonitrile hydrochloride (Compound 50-3)

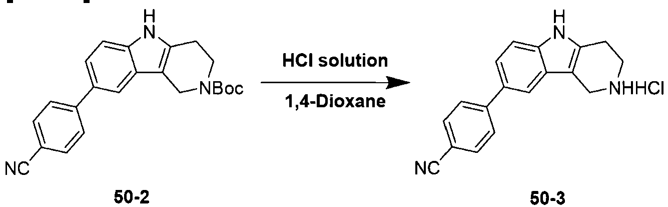

In the same method as in Preparation Examples 1-4, tert-butyl 8-(4-cyanophenyl)-1,3,4,5-tetrahydro-2H-pyrido[4,3-b]indole-2-carboxylate (427.9 mg, 1.15 mmol) and HCl solution (4 M in dioxane) (5.73 mL, 22.92 mmol) were used to obtain 315.1 mg (88% yield) of the title compound.

¹ H NMR (400 MHz, DMSO) δ 11.38 (s, 1H), 9.37 (s, 1H), 7.93 (d, J=13.5 Hz, 5H), 7.56-7.44 (m, 2H), 4.37 (s, 2H), 3.50 (s, 2H), 3.07 (d, J=6.0 Hz, 2H).

### Preparative Example 50-3: (8-(4-Isocyanophenyl)-1,3,4,5-tetrahydro-2H-pyrido[4,3-b]indol-2-yl)(phenyl)methanone (Compound 50)

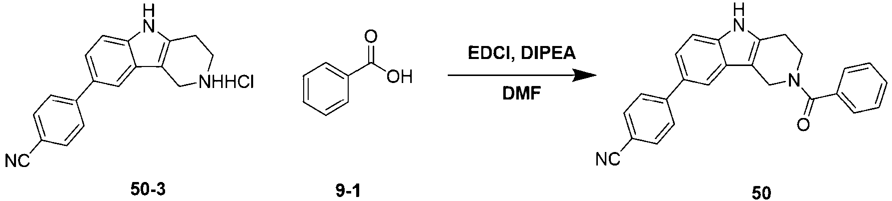

In the same method as in Preparative Example 5, 4-(2,3,4,5-tetrahydro-1H-pyrido[4,3-b]indol-8-yl)benzonitrile hydrochloride (22.7 mg, 0.073 mmol), benzoic acid (10.74 mg, 0.088 mmol), EDCI (56.21 mg, 0.29 mmol) and DIPEA (0.051 mL, 0.29 mmol) were used to obtain 8.8 mg of the title compound (31 % yield).

¹H NMR (400 MHz, DMSO) δ 11.19 (s, 1H), 7.99 - 7.83 (m, 4H), 7.68 (s, 1H), 7.50 (s, 7H), 4.87 (s, 1H), 4.67 (s, 1H), 3.68 (s, 2H), 2.90 (s, 2H).

### Preparative Example 51: ((8-(4-Isocyanophenyl)-1,3,4,5-tetrahydro-2H-pyrido[4,3-b]indol-2-yl)(pyridin-3-yl)methanone (Compound 51)

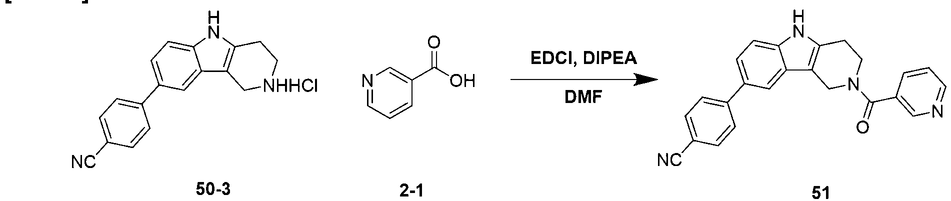

In the same method as in Preparative Example 5, 4-(2,3,4,5-tetrahydro-1H-pyrido[4,3-b]indol-8-yl)benzonitrile hydrochloride (22.7 mg, 0.073 mmol), nicotinic acid (10.82 mg, 0.088 mmol), EDCI (56.19 mg, 0.29 mmol) and DIPEA (0.051 mL, 0.29 mmol) were used to obtain 11.3 mg of the title compound (40% yield).

¹H NMR (400 MHz, DMSO) δ 11.20 (s, 1H), 8.70 (s, 2H), 7.97 (d, J = 9.0 Hz, 2H), 7.92 - 7.72 (m, 4H), 7.55 - 7.40 (m, 3H), 4.90 (s, 1H), 4.69 (s, 1H), 4.06 (s, 1H), 3.69 (s, 1H), 2.93 (s, 2H).

### Preparative Example 52: (8-(4-Isocyanophenyl)-1,3,4,5-tetrahydro-2H-pyrido[4,3-b]indol-2-yl)(p-tolyl)methanone (Compound 52)

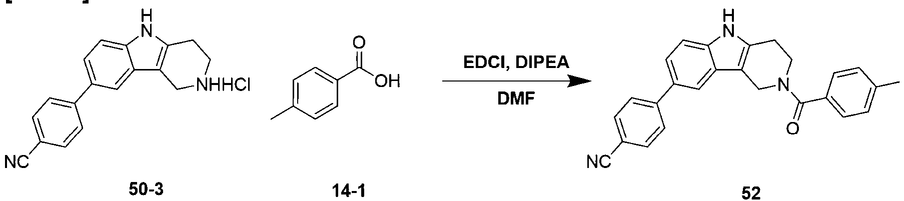

In the same method as in Preparative Example 5, 4-(2,3,4,5-tetrahydro-1H-pyrido[4,3-b]indol-8-yl)benzonitrile hydrochloride (20 mg, 0.065 mmol), 4-methylbenzoic acid (10.55 mg, 0.077 mmol), EDCI (49.5 mg, 0.26 mmol) and DIPEA (0.045 mL, 0.26 mmol) were used to obtain 10 mg (yield 39%) of the title compound.

¹H NMR (400 MHz, DMSO) δ 11.14 (s, 1H), 7.88 (d, J = 33.4 Hz, 5H), 7.43 - 7.34 (m, 4H), 7.27 (d, J = 7.7 Hz, 2H), 4.81 (s, 1H), 4.68 (s, 1H), 4.00 (s, 1H), 3.67 (s, 1H), 2.87 (s, 2H), 2.35 (s, 3H).

### Preparative Example 53: (8-(4-Isocyanophenyl)-1,3,4,5-tetrahydro-2H-pyrido[4,3-b]indol-2-yl)(m-tolyl)methanone (Compound 53)

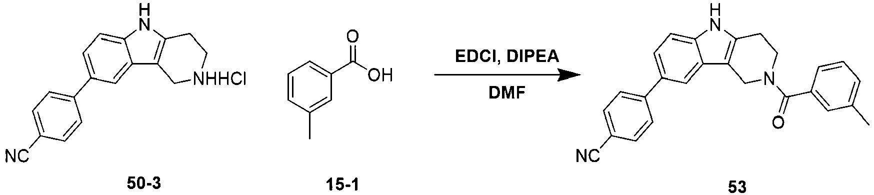

In the same method as in Preparative Example 5, 4-(2,3,4,5-tetrahydro-1H-pyrido[4,3-b]indol-8-yl)benzonitrile hydrochloride (20 mg, 0.065 mmol), 3-methylbenzoic acid (10.55 mg, 0.077 mmol), EDCI (49.5 mg, 0.26 mmol) and DIPEA (0.045 mL, 0.26 mmol) were used to obtain 11.7 mg (yield 46%) of the title compound.

¹H NMR (400 MHz, DMSO) δ 11.16 (s, 1H), 7.95 (s, 2H), 7.85 (s, 2H), 7.67 (s, 1H), 7.42 (s, 2H), 7.36 (s, 1H), 7.29(d, J=11.7 Hz, 3H), 4.85(s, 1H), 4.64(s, 1H), 4.02(d, J=7.7 Hz, 1H), 3.67(s, 1H), 2.88(s, 2H), 2.36(s, 3H).

### Preparative Example 54: (8-(4-Isocyanophenyl)-1,3,4,5-tetrahydro-2H-pyrido[4,3-b]indole- 2-yl)(6-methylpyridin-3-yl)methanone (Compound 54)

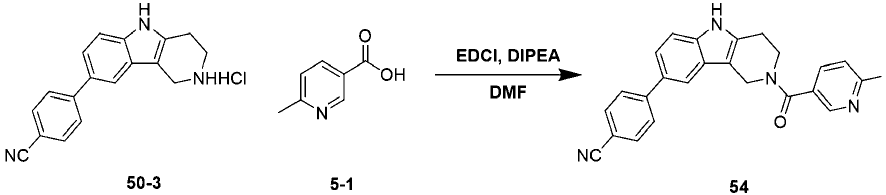

In the same method as in Preparative Example 5, 4-(2,3,4,5-tetrahydro-1H-pyrido[4,3-b]indol-8-yl)benzonitrile hydrochloride (20 mg, 0.065 mmol), 6-methylnicotinic acid (10.62 mg, 0.077 mmol), EDCI (49.5 mg, 0.26 mmol) and DIPEA (0.045 mL, 0.26 mmol) were used to obtain 10.8 mg (yield 42%) of the title compound.

¹H NMR (400 MHz, DMSO) δ 11.16 (s, 1H), 8.57 (s, 1H), 7.90 (d, J = 40.3 Hz, 6H), 7.40 (d, J = 19.9 Hz, 3H), 4.78 (d, J = 66.6 Hz, 2H), 4.03 (s, 1H), 3.69 (s, 1H), 2.91 (s, 2H), 2.53 (s, 3H).

### Preparative Example 55: 3-(8-(4-Isocyanophenyl)-2,3,4,5-tetrahydro-1H-pyrido[4,3-b]indole-2-carbonyl)benzonitrile (Compound 55)

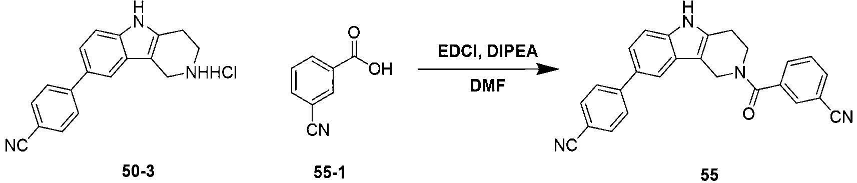

In the same method as in Preparative Example 5, 4-(2,3,4,5-tetrahydro-1H-pyrido[4,3-b]indole- 8-yl)benzonitrile hydrochloride (20 mg, 0.065 mmol), 3-cyanobenzoic acid (11.4 mg, 0.077 mmol), EDCI (49.5 mg, 0.26 mmol) and DIPEA (0.045 mL, 0.26 mmol) were used to obtain 12.2 mg of the title compound (46% yield).

¹H NMR (400 MHz, DMSO) δ 11.17 (s, 1H), 7.95 (s, 4H), 7.85 (d, J = 20.0 Hz, 4H), 7.69 (s, 1H), 7.46 (s, 1H), 7.41 (s, 1H), 4.87 (s, 1H), 4.61 (s, 1H), 3.63 (s, 2H), 2.89 (s, 2H).

### Preparative Example 56: Preparation of Compound 56

### Preparative Example 56-1: tert-butyl 8-(3-cyanophenyl)-1,3,4,5-tetrahydro-2H-pyrido[4,3-b]indole-2-carboxylate (Compound 56-2)

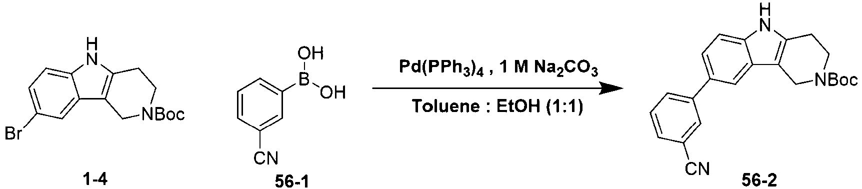

In the same manner as in Examples 1-3, *tert-butyl* 8-bromo-1,3,4,5-tetrahydro-2H-pyrido[4,3-b]indole-2-carboxylate (1 g, 2.85 mmol), (3-cyanophenyl)boronic acid (1.26 g, 8.54 mmol), Pd(PPh₃) ₄ (164.55 mg, 0.14 mmol) and 1 M Na₂CO₃ (7.12 mL, 7.12 mmol) were used to obtain 278.3 mg (26% yield) of the title compound.

¹H NMR (400 MHz, DMSO) δ 11.06 (s, 1H), 8.19 (s, 1H), 8.06 (d, J=7.6 Hz, 1H), 7.85 (s, 1H), 7.74 (d, J=7.6 Hz, 1H), 7.64(d, J=8.1 Hz, 1H), 7.46-7.36(m, 2H), 4.60(s, 2H), 3.73(s, 2H), 2.80(s, 2H), 1.45(d, J=3.6 Hz, 9H).

### Preparative Example 56-2: 3-(2,3,4,5-tetrahydro-1H-pyrido[4,3-b]indol-8-yl)benzonitrile hydrochloride (Compound 56-3)

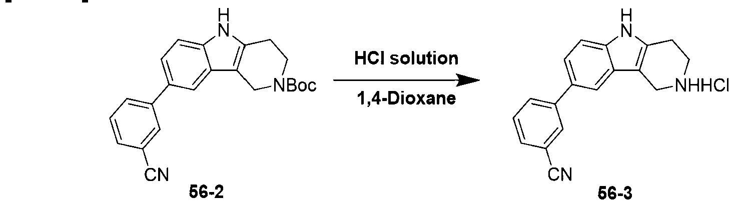

In the same method as in Preparation Examples 1-4, tert-butyl 8-(3-cyanophenyl)-1,3,4,5-tetrahydro-2H-pyrido[4,3-b]indole-2-carboxylate (278.3 mg, 0.75 mmol) and HCl solution (4 M in dioxane) (3.73 mL, 14.9 mmol) were used to obtain 198.8 mg (86% yield) of the title compound.

¹H NMR (400 MHz, DMSO) δ 11.32(s, 1H), 9.34(s, 2H), 8.16(d, J=1.8 Hz, 1H), 8.05(dt, J = 7.9, 1.5 Hz, 1H), 7.93(d, J=1.8 Hz, 1H), 7.75(dt, J = 7.7, 1.3 Hz, 1H), 7.65(t, J = 7.8 Hz, 1H), 7.51(dd, J = 8.5, 1.8 Hz, 1H), 7.45(d, J=8.4 Hz, 1H), 4.36(s, 2H), 3.49(d, J=6.5 Hz, 2H), 3.05(t, J=6.0 Hz, 2H).

### Preparative Example 56-3: 3-(2-(4-Methylbenzoyl)-2,3,4,5-tetrahydro-1H-pyrido[4,3-b]indol-8-yl)benzonitrile (Compound 56)

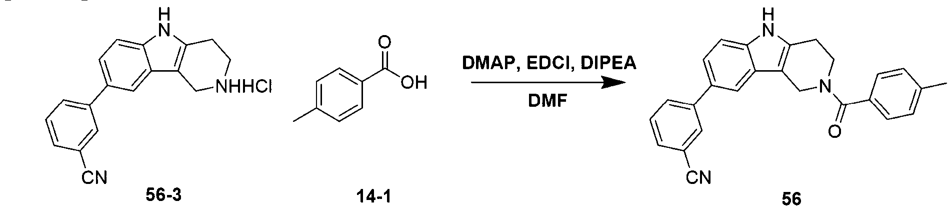

Under nitrogen, 3-(2,3,4,5-tetrahydro-1H-pyrido[4,3-b]indol-8-yl)benzonitrile hydrochloride (20 mg, 0.065 mmol) was dissolved in dimethylformamide. 4-Methylbenzoic acid (10.55 mg, 0.077 mmol), DMAP (31.54 mg, 0.26 mmol), EDCI (49.5 mg, 0.26 mmol) and DIPEA (0.045 mL, 0.26 mmol) were added. The reaction mixture was stirred at room temperature for 12 hours. Upon completion of the reaction, it was extracted with distilled water and ethyl acetate, and the organic layer was dried over magnesium anhydride and filtered. The filtrate was concentrated under reduced pressure, and the concentrate was purified by column chromatography to obtain 10.9 mg (43% yield) of the title compound.

¹H NMR (400 MHz, DMSO) δ 11.11 (s, 1H), 8.23-7.93 (m, 3H), 7.66 (d, J = 40.0 Hz, 3H), 7.33 (dd, J = 41.3, 8.0 Hz, 6H), 4.83(s, 1H), 4.68(s, 1H), 4.02 (d, J = 7.0 Hz, 1H), 3.68 (s, 1H), 2.88(s, 2H), 2.36(s, 3H).

### Preparative Example 57: 3-(2-(6-Methylnicotinoyl)-2,3,4,5-tetrahydro-1H-pyrido[4,3-b]indol-8-yl)benzonitrile (Compound 57)

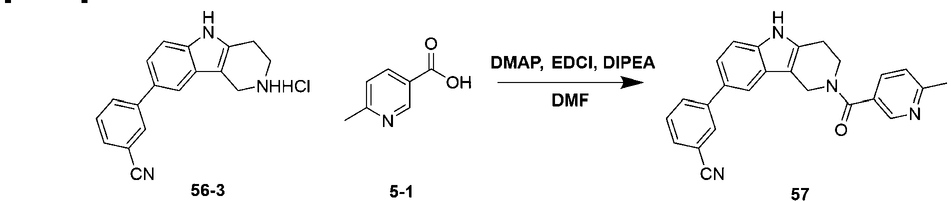

In the same method as in Preparative Example 56-3, 3-(2,3,4,5-tetrahydro-1H-pyrido[4,3-b]indol-8-yl)benzonitrile hydrochloride (20 mg, 0.065 mmol), 6-methylnicotinic acid (10.62 mg, 0.077 mmol), DMAP (31.54 mg, 0.26 mmol), EDCI (49.5 mg, 0.26 mmol) and DIPEA (0.045 mL, 0.26 mmol) were used to obtain 6 mg (yield 23%) of the title compound.

¹H NMR (400 MHz, DMSO) δ 11.12 (s, 1H), 8.57 (s, 1H), 8.21 (s, 1H), 8.10 (s, 1H), 7.97 (s, 1H), 7.81 (s, 1H), 7.73 (d, J = 8.5 Hz, 2H), 7.61 (s, 1H), 7.36 (d, J = 8.0 Hz, 2H), 4.86 (s, 1H), 4.70 (s, 1H), 4.03 (d, J = 5.0 Hz, 1H), 3.69 (s, 1H), 2.91 (s, 2H), 2.53 (s, 3H).

### Preparative Example 58: 3-(2-benzoyl-2,3,4,5-tetrahydro-1H-pyrido[4,3-b]indol-8-yl)benzonitrile (Compound 58)

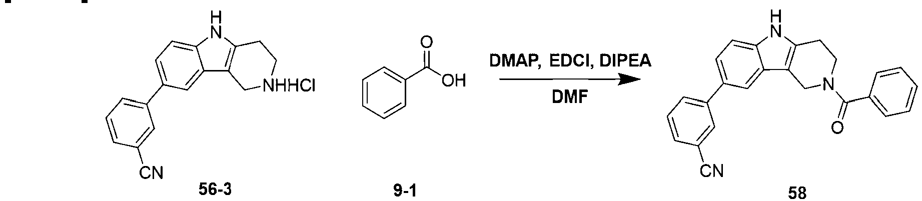

In the same manner as in Example 56-3, 3-(2,3,4,5-tetrahydro-1H-pyrido[4,3-b]indol-8-yl)benzonitrile hydrochloride (20 mg, 0.065 mmol), benzoic acid (9.46 mg, 0.077 mmol), DMAP (31.54 mg, 0.26 mmol), EDCI (49.5 mg, 0.26 mmol) and DIPEA (0.045 mL, 0.26 mmol) were used to obtain 9.8 mg of the title compound (40% yield).

¹H NMR (400 MHz, DMSO) δ 11.12 (s,1H), 8.22 (s,1H), 8.09 (s,1H), 7.97 (s,1H), 7.72 (s,1H), 7.63 (s, 1H), 7.49 (s, 5H), 7.41 (s, 2H), 4.86 (s, 1H), 4.66 (s, 1H), 4.03 (s, 1H), 3.68 (s, 1H), 2.90 (s, 2H).

### Preparative Example 59: Preparation of Compound 59

### Preparative Example 59-1: tert-butyl 8-(2-cyanophenyl)-1,3,4,5-tetrahydro-2H-pyrido[4,3-b]indole-2-carboxylate (Compound 59-2)

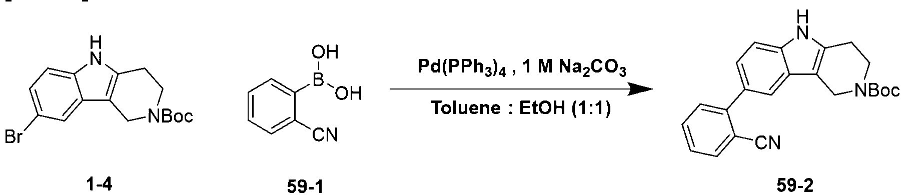

In the same method as in Preparative Examples 1-3, *tert-butyl* 8-bromo-1,3,4,5-tetrahydro-2H-pyrido[4,3-b]indole-2-carboxylate (1 g, 2.85 mmol), (2-cyanophenyl)boronic acid (1.26 g, 8.54 mmol), Pd(PPh₃)₄ (164.55 mg, 0.14 mmol) and 1 M Na₂CO₃ (7.12 mL, 7.12 mmol) were used to obtain 798 mg (75% yield) of the title compound.

¹H NMR (400 MHz, DMSO) δ 11.14 (s, 1H), 7.90 (dd, J = 7.8, 1.4 Hz, 1H), 7.75 (td, J = 7.6, 1.4 Hz, 1H), 7.68 - 7.57 (m, 2H), 7.51 (td, J = 7.6, 1.3 Hz, 1H), 7.43 (d, J = 8.4 Hz, 1H), 7.25 (dd, J = 8.4, 1.8 Hz, 1H), 4.57 (s, 2H), 3.72 (t, J = 5.7 Hz, 2H), 2.80 (t, J = 5.8 Hz, 2H), 1.43 (s, 9H).

### Preparative Example 59-2: 2-(2,3,4,5-tetrahydro-1H-pyrido[4,3-b]indol-8-yl)benzonitrile hydrochloride (Compound 59-3)

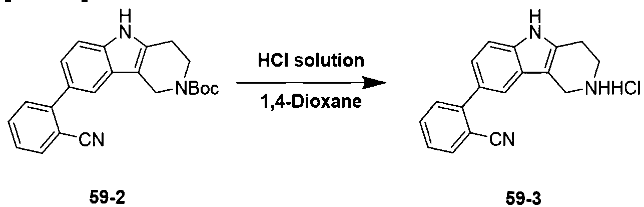

In the same method as in Preparative Examples 1-4, *tert-butyl* 8-(2-cyanophenyl)-1,3,4,5-tetrahydro-2H-pyrido[4,3-b]indole-2-carboxylate (798 mg, 2.14 mmol) and HCl solution (4 M in dioxane) (10.68 mL, 42.74 mmol) were used to obtain 465.2 mg (70% yield) of the title compound.

¹H NMR (400 MHz, DMSO) δ 11.40 (s, 1H), 9.36 (s, 1H), 7.91 (dd, J = 7.7, 1.3 Hz, 1H), 7.76 (td, J = 7.7, 1.4 Hz, 1H), 7.68 (d, J = 1.7 Hz, 1H), 7.63 (d, J = 7.8 Hz, 1H), 7.59 - 7.45 (m, 2H), 7.30 (dd, J = 8.4, 1.8 Hz, 1H), 4.33 (s, 2H), 3.48 (d, J = 6.4 Hz, 2H), 3.06 (t, J = 6.0 Hz, 2H).

### Preparative Example 59-3: 2-(2-(4-Methylbenzoyl)-2,3,4,5-tetrahydro-1H-pyrido[4,3-b]indol-8-yl)benzonitrile (Compound 59)

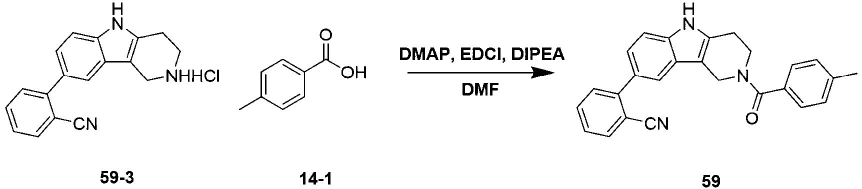

In the same method as in Preparative Example 56-3, 2-(2,3,4,5-tetrahydro-1H-pyrido[4,3-b]indol-8-yl)benzonitrile hydrochloride (20 mg, 0.065 mmol), 4-methylbenzoic acid (10.55 mg, 0.077 mmol), DMAP (31.54 mg, 0.26 mmol), EDCI (49.5 mg, 0.26 mmol) and DIPEA (0.045 mL, 0.26 mmol) were used to obtain 16.5 mg (65% yield) of the title compound.

¹H NMR (400 MHz, DMSO) δ 11.20 (s, 1H), 7.89 (s, 1H), 7.73 (s, 3H), 7.50 (s, 1H), 7.44 (d, J = 8.3 Hz, 1H), 7.37 (d, J = 7.5 Hz, 2H), 7.27 (s, 3H), 4.79 (s, 1H), 4.66 (s, 1H), 3.69 (s, 2H), 2.90 (s, 2H), 2.35 (s, 3H).

### Preparative Example 60: 2-(2-(6-Methylnicotinoyl)-2,3,4,5-tetrahydro-1H-pyrido[4,3-b]indol-8-yl)benzonitrile (Compound 60)

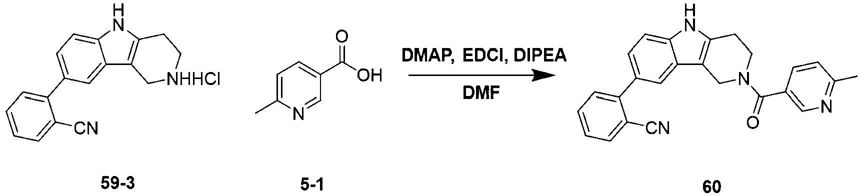

In the same method as in Preparative Example 56-3, 2-(2,3,4,5-tetrahydro-1H-pyrido[4,3-b]indol-8-yl)benzonitrile hydrochloride (20 mg, 0.065 mmol), 6-methylnicotinic acid (10.62 mg, 0.077 mmol), DMAP (31.54 mg, 0.26 mmol), EDCI (49.5 mg, 0.26 mmol) and DIPEA (0.045 mL, 0.26 mmol) were used to obtain 15.3 mg of the title compound (60% yield).

¹H NMR (400 MHz, DMSO) δ 11.21 (s, 1H), 8.56 (s, 1H), 7.90 - 7.68 (m, 4H), 7.39 (dd, J = 63.4, 30.2 Hz, 5H), 4.83 (s, 1H), 4.67 (s, 1H), 4.03 (s, 1H), 3.69 (s, 1H), 2.92 (s, 2H), 2.52 (s, 3H).

### Preparative Example 61: 2-(2-benzoyl-2,3,4,5-tetrahydro-1H-pyrido[4,3-b]indol-8-yl)benzonitrile (Compound 61)

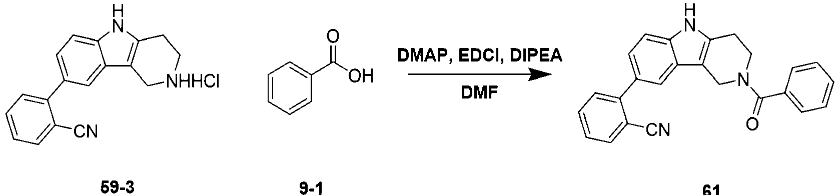

In the same method as in Preparative Example 56-3, 2-(2,3,4,5-tetrahydro-1H-pyrido[4,3-b]indol-8-yl)benzonitrile hydrochloride (20 mg, 0.065 mmol), benzoic acid (9.46 mg, 0.077 mmol), DMAP (31.54 mg, 0.26 mmol), EDCI (49.5 mg, 0.26 mmol) and DIPEA (0.045 mL, 0.26 mmol) were used to obtain 13.7 mg (yield 56%) of the title compound.

¹H NMR (400 MHz, DMSO) δ 11.21 (s, 1H), 7.90 (s, 1H), 7.71 (s, 3H), 7.52 - 7.43 (m, 7H), 7.27 (s, 1H), 4.83 (s, 1H), 4.63 (s, 1H), 3.85 (d, J = 136.5 Hz, 2H), 2.91 (s, 2H).

### Preparative Example 62: Preparation of Compound 62

### Preparative Example 62-1: tert-Butyl 8-(4-methoxyphenyl)-1,3,4,5-tetrahydro-2H-pyrido[4,3-b]indole-2-carboxylate (Compound 62-2)

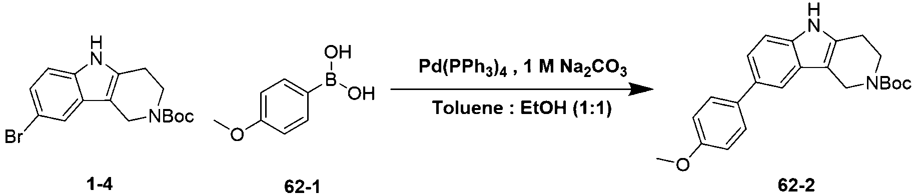

In the same manner as in Examples 1-3, *tert-butyl* 8-bromo-1,3,4,5-tetrahydro-2H-pyrido[4,3-b]indole-2-carboxylate (200 mg, 0.57 mmol), (4-methoxyphenyl)boronic acid (450 mg, 2.96 mmol), Pd(PPh₃)₄ (32.9 mg, 0.028 mmol) and 1 M Na₂CO₃ (1.42 mL, 1.42 mmol) were used to obtain 160.6 mg (74% yield) of the title compound.

¹H NMR (400 MHz, DMSO) δ 10.91 (s, 1H), 7.62 - 7.55 (m, 3H), 7.36 - 7.25 (m, 2H), 6.98 (d, J = 8.7 Hz, 2H), 4.56 (s, 2H), 3.78 (s, 3H), 3.70 (t, J = 5.7 Hz, 2H), 2.77 (s, 2H), 1.44 (s, 9H).

### Preparative Example 62-2: 8-(4-Methoxyphenyl)-2,3,4,5-tetrahydro-1H-pyrido[4,3-b]indole hydrochloride (Compound 62-3)

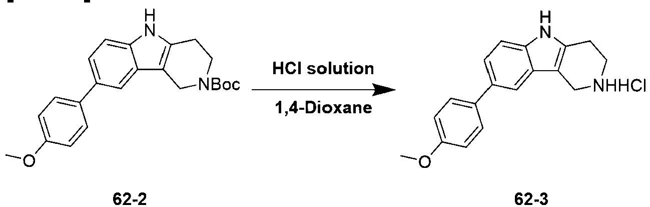

Using the same method as in Examples 1-4, *tert-butyl* 8-(4-methoxyphenyl)-1,3,4,5-tetrahydro-2H-pyrido[4,3-b]indole-2-carboxylate (160.6 mg, 0.42 mmol) and HCl solution (4 M in dioxane) (2.12 mL, 8.486 mmol) were used to obtain 116.4 mg (87% yield) of the title compound.

¹H NMR (400 MHz, DMSO) δ 11.18 (s,1H), 9.28 (s,1H), 7.70 (s,1H), 7.60 (d,J = 8.4 Hz,2H), 7.41 - 7.34 (m, 2H), 7.01 (d, J = 8.4 Hz, 2H), 4.35 (s, 2H), 3.79 (s, 3H), 3.49 (s, 2H), 3.03 (d, J = 8.3 Hz, 2H).

### Preparative Example 62-3: (8-(4-Methoxyphenyl)-1,3,4,5-tetrahydro-2H-pyrido[4,3-b]indol-2-yl)(phenyl)methanone (Compound 62)

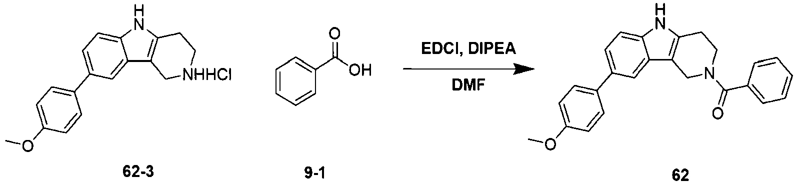

In the same method as in Preparative Example 5, 8-(4-methoxyphenyl)-2,3,4,5-tetrahydro-1H- pyrido[4,3-b]indole hydrochloride (30 mg, 0.079 mmol), benzoic acid (11.62 mg, 0.095 mmol), EDCI (60.79 mg, 0.32 mmol) and DIPEA (0.055 mL, 0.32 mmol) were used to obtain 8.2 mg of the title compound (yield 27%).

¹H NMR (400 MHz, DMSO) δ 10.98 (s, 1H), 7.65 (d, J = 31.6 Hz, 2H), 7.47 (s, 5H), 7.30 (d, J = 17.7 Hz, 3H), 6.98 (s, 2H), 4.83 (s, 1H), 4.62 (s, 1H), 3.77 (s, 4H), 3.65 (s, 1H), 2.87 (s, 2H).

### Preparative Example 63: (8-(4-Methoxyphenyl)-1,3,4,5-tetrahydro-2H-pyrido[4,3-b]indol-2-yl)(pyridin-3-yl)methanone (Compound 63)

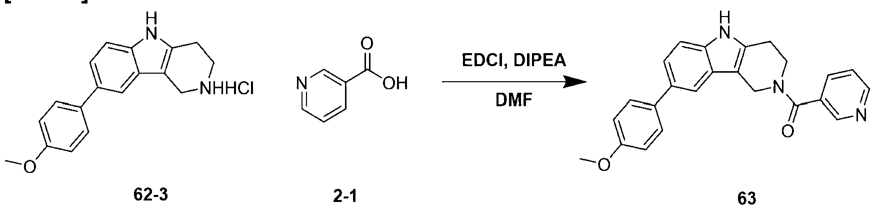

In the same method as in Preparative Example 5, 8-(4-methoxyphenyl)-2,3,4,5-tetrahydro-1H-pyrido[4,3-b]indole hydrochloride (30 mg, 0.079 mmol), nicotinic acid (11.71 mg, 0.095 mmol), EDCI (60.79 mg, 0.32 mmol) and DIPEA (0.055 mL, 0.32 mmol) were used to obtain 8.9 mg of the title compound (yield 29%).

¹H NMR (400 MHz, DMSO) δ 10.98 (s, 1H), 8.68 (s, 2H), 7.93 (s, 1H), 7.70 (s, 1H), 7.62 (d, J = 8.4 Hz, 1H), 7.50 (s, 2H), 7.32 (s, 2H), 6.98 (d, J = 8.4 Hz, 2H), 4.85 (s, 1H), 4.64 (s, 1H), 3.76 (d, J = 11.7 Hz, 4H), 3.66 (s, 1H), 2.88 (d, J = 7.2 Hz, 2H).

### Preparative Example 64: Preparation of Compound 64

### Preparative Example 64-1: tert-butyl 8-(4-fluorophenyl)-1,3,4,5-tetrahydro-2H-pyrido[4,3-b]indole-2-carboxylate (Compound 64-2)

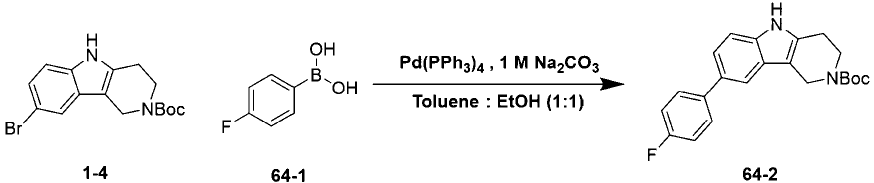

In the same manner as in Examples 1-3, *tert-butyl* 8-bromo-1,3,4,5-tetrahydro-2H-pyrido[4,3-b]indole-2-carboxylate (200 mg, 0.57 mmol), (4-fluorophenyl)boronic acid (478 mg, 3.42 mmol), Pd(PPh₃)₄(32.9 mg, 0.028 mmol) and 1 M Na₂CO₃(1.42 mL, 1.42 mmol) were used to obtain 119.4 mg (57% yield) of the title compound.

¹H NMR (400 MHz, DMSO) δ 10.97 (s, 1H), 7.68 (dd, J = 16.5, 9.5 Hz, 3H), 7.33 (q, J = 8.1 Hz, 2H), 7.24 (t, J = 8.6 Hz, 2H), 4.57 (s, 2H), 3.70 (d, J = 6.0 Hz, 2H), 2.77 (s, 2H), 1.44 (s, 9H).

### Preparative Example 64-2: 8-(4-fluorophenyl)-2,3,4,5-tetrahydro-1H-pyrido[4,3-b]indole hydrochloride (Compound 64-3)

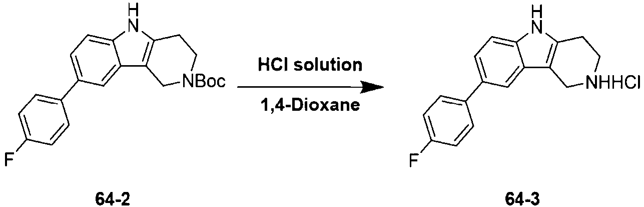

In the same method as in Preparative Examples 1-4, *tert*-butyl 8-(4-fluorophenyl)-1,3,4,5-tetrahydro-2H-pyrido[4,3-b]indole-2-carboxylate (119.4 mg, 0.33 mmol) and HCl solution (4 M in dioxane) (1.63 mL, 6.52 mmol) were used to obtain 71.8 mg (72% yield) of the title compound.

¹H NMR (400 MHz, DMSO) δ 11.23 (s, 1H), 9.29 (s, 1H), 7.77 - 7.65 (m, 3H), 7.40 (t, J = 6.6 Hz, 2H), 7.26 (t, J = 8.8 Hz, 2H), 4.33 (s, 2H), 3.47 (s, 2H), 3.04 (d, J = 6.2 Hz, 2H).

### Preparative Example 64-3: (8-(4-fluorophenyl)-1,3,4,5-tetrahydro-2H-pyrido[4,3-b]indol-2-yl)(phenyl)methanone (Compound 64)

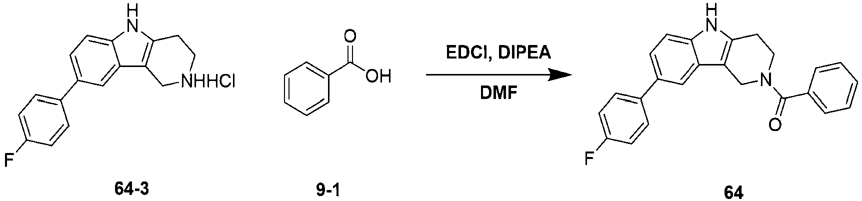

In the same method as in Preparative Example 5, 8-(4-fluorophenyl)-2,3,4,5-tetrahydro-1H-pyrido[4,3-b]indole hydrochloride (30 mg, 0.099 mmol), benzoic acid (14.52 mg, 0.12 mmol), EDCI (75.97 mg, 0.4 mmol) and DIPEA (0.069 mL, 0.4 mmol) were used to obtain 18.6 mg (yield 50%) of the title compound.

¹H NMR (400 MHz, DMSO) δ 11.05 (s, 1H), 7.74 (s, 1H), 7.63 (s, 1H), 7.49 (s, 5H), 7.36 (s, 2H), 7.25 (s, 3H), 4.85 (s, 1H), 4.64 (s, 1H), 3.67 (s, 2H), 2.89 (s, 2H).

### Preparative Example 65: (8-(4-fluorophenyl)-1,3,4,5-tetrahydro-2H-pyrido[4,3-b]indol-2-yl)(pyridin-3-yl)methanone (Compound 65)

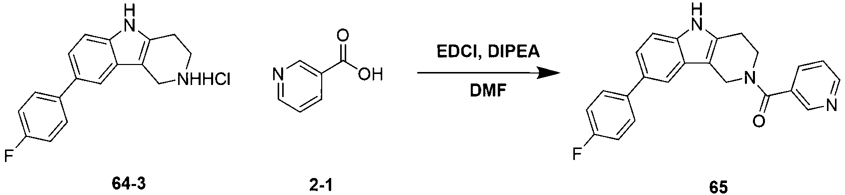

In the same method as in Preparative Example 5, 8-(4-fluorophenyl)-2,3,4,5-tetrahydro-1H-pyrido[4,3-b]indole hydrochloride (30 mg, 0.099 mmol), nicotinic acid (14.64 mg, 0.12 mmol), EDCI (75.97 mg, 0.4 mmol) and DIPEA (0.069 mL, 0.4 mmol) were used to obtain 19.2 mg (yield 52%) of the title compound.

¹H NMR (400 MHz, DMSO) δ 11.06 (s,1H), 8.69 (s,2H), 7.92 (s,1H), 7.75 (d,J = 17.1 Hz,2H), 7.63 (s,1H), 7.52 (s, 1H), 7.36 (s, 2H), 7.24 (s, 2H), 4.87 (s, 1H), 4.66 (s, 1H), 4.03 (s, 1H), 3.67 (s, 1H), 2.90 (s, 2H).

### Preparative Example 66: Preparation of Compound 66

### Preparative Example 66-1: tert-butyl 8-(4-cyclohexylphenyl)-1,3,4,5-tetrahydro-2H-pyrido[4,3-b]indole-2-carboxylate (Compound 66-2)

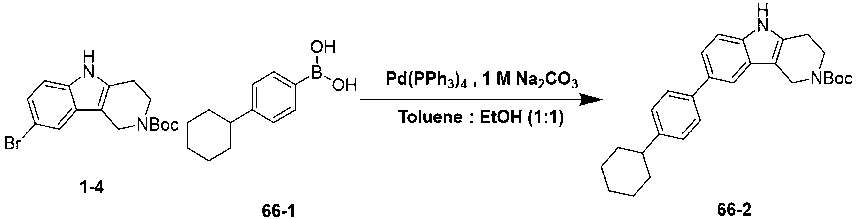

In the same manner as in Preparative Examples 1-3, *tert-butyl* 8-bromo-1,3,4,5-tetrahydro-2H-pyrido[4,3-b]indole-2-carboxylate (300 mg, 0.85 mmol), (4-cyclohexylphenyl)boronic acid (522.91 mg, 2.56 mmol), Pd(PPh₃)₄ (49.35 mg, 0.043 mmol) and 1 M Na₂CO₃ (2.14 mL, 2.14 mmol) were used to obtain 288.7 mg of the title compound (78% yield).

¹H NMR (400 MHz, DMSO) δ 10.94 (s, 1H), 7.65 - 7.54 (m, 3H), 7.33 (s, 2H), 7.27 (d, J = 7.8 Hz, 2H), 4.57 (s, 2H), 3.71 (d, J = 7.1 Hz, 2H), 2.78 (s, 2H), 2.49 (s, 1H), 1.81 (d, J = 9.2 Hz, 4H), 1.72 (d, J = 12.8 Hz, 1H), 1.51 - 1.35 (m, 15H).

### Preparative Example 66-2: 8-(4-Cyclohexylphenyl)-2,3,4,5-tetrahydro-1H-pyrido[4,3-b]indole hydrochloride (Compound 66-3)

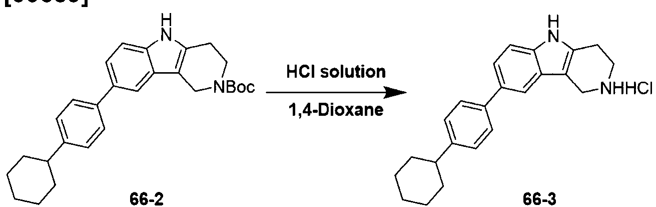

In the same manner as in Preparative Examples 1-4, 8-(4-cyclohexylphenyl)-1,3,4,5-tetrahydro-2H-pyrido[4,3-b]indole-2-carboxylate (288.7 mg, 0.67 mmol) and HCl solution (4 M in dioxane) (3.35 mL, 13.41 mmol) were used to obtain 220.6 mg (89% yield) of the title compound.

¹H NMR (400 MHz, DMSO) δ 11.20 (s, 1H), 9.26 (s, 2H), 7.73 (s, 1H), 7.58 (d, J = 7.9 Hz, 2H), 7.39 (s, 2H), 7.39 (s, 2H), 7.28 (d, J = 7.9 Hz, 2H), 4.35 (s, 2H), 3.48 (s, 2H), 3.04 (s, 2H), 2.49 (s, 1H), 1.81 (d, J = 9.7 Hz, 5H), 1.41 (q, J = 12.1 Hz, 5H).

### Preparative Example 66-3: (8-(4-Cyclohexylphenyl)-1,3,4,5-tetrahydro-2H-pyrido[4,3-b]indol-2-yl)(phenyl)methanone (Compound 66)

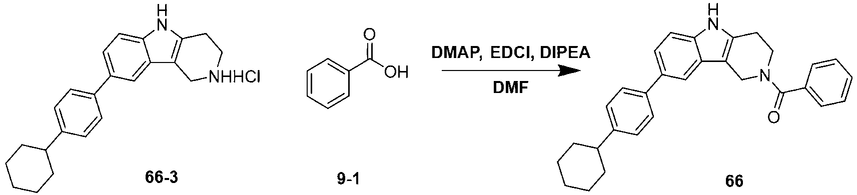

In the same method as in Preparative Example 56-3, 8-(4-cyclohexylphenyl)-2,3,4,5-tetrahydro-1H-pyrido[4,3-b]indole hydrochloride (20 mg, 0.055 mmol), benzoic acid (7.99 mg, 0.065 mmol), DMAP (26.63 mg, 0.22 mmol), EDCI (41.79 mg, 0.22 mmol) and DIPEA (0.038 mL, 0.22 mmol) were used to obtain 9.3 mg of the title compound (39% yield).

¹H NMR (400 MHz, DMSO) δ 11.02 (s,1H), 7.75 (s,1H), 7.62 (s,2H), 7.50 (s,5H), 7.37 (s,2H), 7.28 (s,2H), 4.85 (s,1H), 4.64 (s, 1H), 4.04 (s, 1H), 3.68 (s, 1H), 2.89 (s, 2H), 1.82 (s, 4H), 1.73 (d, J = 12.6 Hz, 1H), 1.42 (s, 4H), 1.26 (d, J = 10.9 Hz, 1H).

### Preparative Example 67: (8-(4-Cyclohexylphenyl)-1,3,4,5-tetrahydro-2H-pyrido[4,3-b]indol-2-yl)(6-methylpyridin-3-yl)methanone (Compound 67)

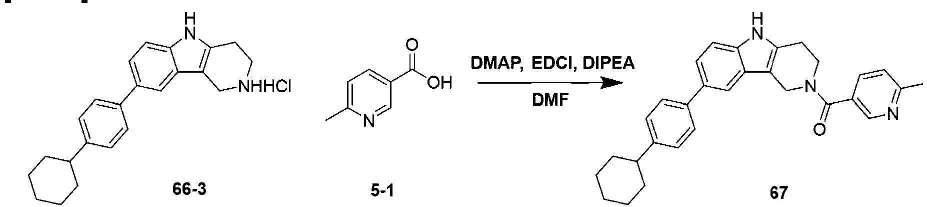

In the same method as in Preparative Example 56-3, 8-(4-cyclohexylphenyl)-2,3,4,5-tetrahydro-1H-pyrido[4,3-b]indole hydrochloride (20 mg, 0.055 mmol), 6-methylnicotinic acid (8.97 mg, 0.077 mmol), DMAP (26.63 mg, 0.22 65 mmol), EDCI (41.79 mg, 0.22 mmol) and DIPEA (0.038 mL, 0.22 mmol) were used to obtain 7.3 mg (yield 29%) of the title compound.

¹H NMR (400 MHz, DMSO) δ 11.00 (s,1H), 8.56 (s,1H), 7.80 (s,2H), 7.60 (s,1H), 7.52 (s,1H), 7.35 (s,3H), 7.26 (s,2H), 4.84 (s,1H), 4.67 (s, 1H), 3.68 (s, 2H), 2.90 (s, 2H), 2.53 (s, 3H), 1.80 (s, 4H), 1.71 (d, J = 12.5 Hz, 1H), 1.40 (s, 4H), 1.24 (d, J = 11.6 Hz, 1H).

### Preparative Example 68: Preparation of Compound 68

### Preparative Example 68-1: 8-Methyl-2,3,4,5-tetrahydro-1H-pyrido[4,3-b]indole (Compound 68-2)

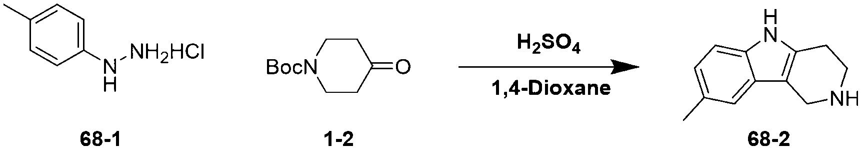

In the same method as in Preparative Example 1-1, p-tolylhydrazine hydrochloride (200 mg, 1.26 mmol), *tert-butyl* 4-oxopiperidine-1-carboxylate (263.83 mg, 1.32 mmol) and H₂SO₄ (0.16 mL, 8 M) were used to obtain 95.3 mg (40% yield) of the title compound.

¹H NMR (400 MHz, DMSO) δ 10.55 (s, 1H), 7.13 (d, J = 8.1 Hz, 1H), 7.08 (s, 1H), 6.81 (dd, J = 8.2, 1.7 Hz, 1H), 3.81 (s, 2H), 3.57 (s, 1H), 3.00 (t, J = 5.6 Hz, 2H), 2.65 (d, J = 5.7 Hz, 2H), 2.34 (s, 3H).

### Preparative Example 68-2: (8-methyl-1,3,4,5-tetrahydro-2H-pyrido[4,3-b]indol-2-yl)(phenyl)methanone (Compound 68)

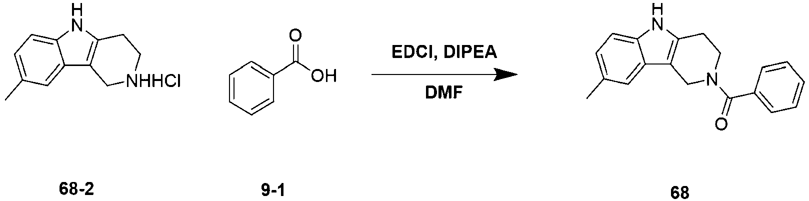

In the same method as in Preparative Example 5, 8-methyl-2,3,4,5-tetrahydro-1H-pyrido[4,3-b]indole (20 mg, 0.11 mmol), benzoic acid (15.74 mg, 0.13 mmol), EDCI (82.35 mg, 0.43 mmol) and DIPEA (0.075 mL, 0.43 mmol) were used to obtain 11.6 mg (37% yield) of the title compound.

¹H NMR (400 MHz, DMSO) δ 10.83 (s,1H), 7.49 (s,5H), 7.25 (s,1H), 7.18 (s,1H), 6.92 - 6.82 (m, 1H), 4.76 (s, 1H), 4.55 (s, 1H), 4.01 (s, 1H), 3.64 (s, 1H), 2.85 (s, 2H), 2.33 (d, J = 40.9 Hz, 3H).

### Preparative Example 69: (4-(Dimethylamino)phenyl)(8-(p-tolyl)-1,3,4,5-tetrahydro-2H-pyrido[4,3-b]indol-2-yl)methanone (Compound 69)

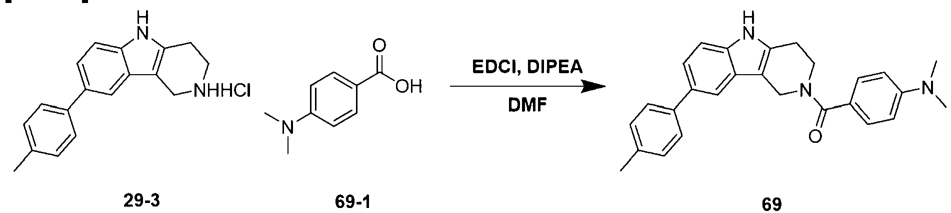

In the same method as in Preparative Example 5, 8-(p-Tolyl)-2,3,4,5-tetrahydro-1H-pyrido[4,3-b]indole hydrochloride (20 mg, 0.07 mmol), 4-(dimethylamino)benzoic acid (22.12 mg, 0.13 mmol), EDCI (51.3 mg, 0.27 mmol) and DIPEA (0.047 mL, 0.27 mmol) were used to obtain 13.1 mg (yield 47%) of the title compound.

¹H NMR (400 MHz, DMSO) δ 11.01 (s, 1H), 7.70 - 7.53 (m, 3H), 7.42 - 7.30 (m, 4H), 7.22 (d, J = 7.9 Hz, 2H), 6.75 (d, J = 8.8 Hz, 2H), 4.76 (s, 2H), 3.85 (s, 2H), 2.97 (s, 6H), 2.90 (s, 2H), 2.33 (s, 3H).

### Preparative Example 70: Preparation of Compound 70

### Preparative Example 70-1: tert-Butyl 8-(4-chlorophenyl)-1,3,4,5-tetrahydro-2H-pyrido[4,3-b]indole-2-carboxylate (Compound 70-2)

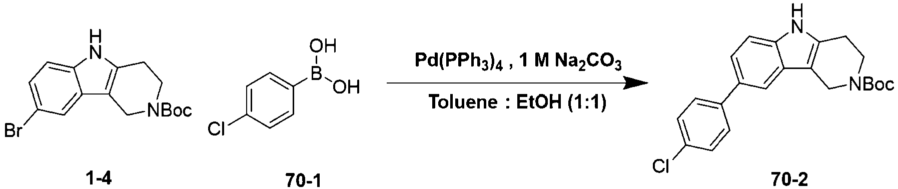

In the same manner as in Examples 1-3, *tert-butyl* 8-bromo-1,3,4,5-tetrahydro-2H-pyrido[4,3-b]indole-2-carboxylate (100 mg, 0.28 mmol), (4-chlorophenyl)boronic acid (66.79 mg, 0.43 mmol), Pd(PPh₃)₄ (16.46 mg, 0.014 mmol) and 1 M Na₂CO₃ (0.71 mL, 0.71 mmol) were used to obtain 66.7 mg (yield 61%) of the title compound.

¹H NMR (400 MHz, DMSO) δ 11.03 (s, 1H), 7.75 - 7.65 (m, 3H), 7.52 - 7.44 (m, 2H), 7.37 (t, J = 1.4 Hz, 2H), 4.59 (s, 2H), 3.72 (t, J = 5.9 Hz, 2H), 2.80 (d, J = 5.4 Hz, 2H), 1.45 (s, 9H).

### Preparative Example 70-2: 8-(4-Chlorophenyl)-2,3,4,5-tetrahydro-1H-pyrido[4,3-b]indole hydrochloride (Compound 70-3)

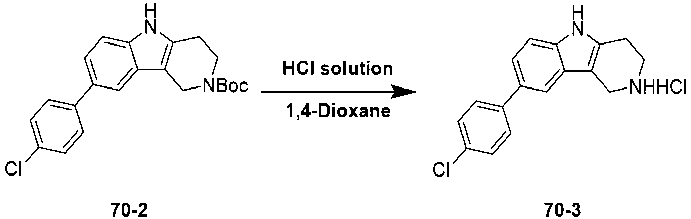

In the same method as in Preparative Examples 1-4, *tert-butyl* 8-(4-chlorophenyl)-1,3,4,5-tetrahydro-2H-pyrido[4,3-b]indole-2-carboxylate (66.7 mg, 0.17 mmol) and HCl solution (4 M in dioxane) (0.87 mL, 3.48 mmol) were used to obtain 41.7 mg (74% yield) of the title compound.

¹H NMR (400 MHz, DMSO) δ 11.31 (s, 1H), 9.45 (s, 1H), 7.81 (s, 1H), 7.75 - 7.65 (m, 2H), 7.53 - 7.44 (m, 2H), 7.43 (s, 2H), 4.35 (s, 2H), 3.51 - 3.44 (m, 2H), 3.05 (t, J = 6.4 Hz, 2H).

### Preparative Example 70-3: (8-(4-chlorophenyl)-1,3,4,5-tetrahydro-2H-pyrido[4,3-b]indol-2-yl)(phenyl)methanone (Compound 70)

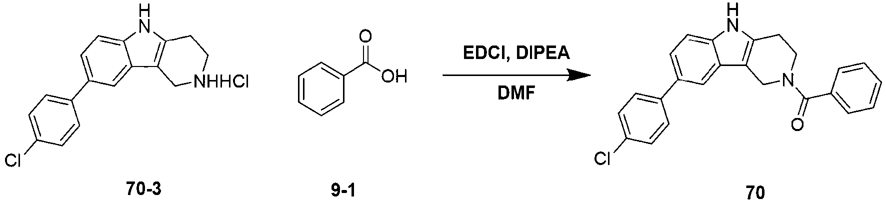

In the same method as in Preparative Example 5, 8-(4-chlorophenyl)-2,3,4,5-tetrahydro-1H-pyrido[4,3-b]indole hydrochloride (20 mg, 0.063 mmol), benzoic acid (9.18 mg, 0.075 mmol), EDCI (48.04 mg, 0.25 mmol) and DIPEA (0.044 mL, 0.25 mmol) were used to obtain 12.3 mg (yield 50%) of the title compound.

¹H NMR (400 MHz, DMSO) δ 11.10 (s, 1H), 7.83 (s, 1H), 7.76 (d, J = 7.8 Hz, 1H), 7.65 (s, 1H), 7.50 (s, 6H), 7.39 (s, 3H), 4.86 (s, 1H), 4.65 (s, 1H), 4.04 (q, J = 7.1 Hz, 1H), 3.67 (s, 1H), 2.89 (s, 2H).

### Preparative Example 71: Preparation of Compound 71

### Preparative Example 71-1: tert-butyl 8-(4-nitrophenyl)-1,3,4,5-tetrahydro-2H-pyrido[4,3-b]indole-2-carboxylate (Compound 71-2)

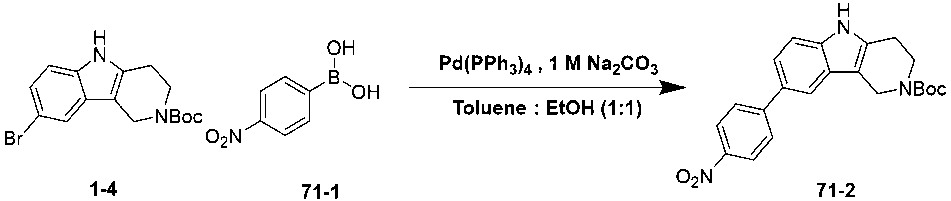

In the same method as in Preparative Examples 1-3, *tert-butyl* 8-bromo-1,3,4,5-tetrahydro-2H-pyrido[4,3-b]indole-2-carboxylate (100 mg, 0.28 mmol), (4-nitrophenyl) boronic acid (142.57 mg, 0.85 mmol), Pd(PPh₃)₄ (16.46 mg, 0.014 mmol) and 1 M Na₂CO₃ (0.71 mL, 0.71 mmol) were used to obtain 33.7 mg (yield 30%) of the title compound.

¹H NMR (400 MHz, DMSO) δ 11.16 (s, 1H), 8.27 (d, J = 8.4 Hz, 2H), 8.01 (d, J=8.5 Hz, 2H), 7.91 (s, 1H), 7.51 (d, J=8.6 Hz, 1H), 7.43 (d, J=8.4 Hz, 1H), 4.61 (s, 2H), 3.73 (t, J = 5.7 Hz, 2H), 2.84-2.77 (m, 2H), 1.45 (s, 9H).

### Preparative Example 71-2: 8-(4-nitrophenyl)-2,3,4,5-tetrahydro-1H-pyrido[4,3-b]indole hydrochloride (Compound 71-3)

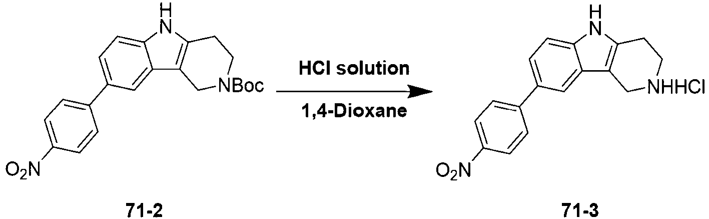

In the same method as in Preparative Examples 1-4, tert-butyl 8-(4-nitrophenyl)-1,3,4,5-tetrahydro-2H-pyrido[4,3-b]indole-2-carboxylate (33.7 mg, 0.086 mmol) and HCl solution (4 M in dioxane) (0.43 mL, 1.71 mmol) were used to obtain 23.8 mg (84% yield) of the title compound.

¹H NMR (400 MHz, DMSO) δ 11.40 (s, 1H), 9.18 (s, 1H), 8.31 (d, J = 8.6 Hz, 2H), 8.00 (d, J = 7.5 Hz, 3H), 7.58 (d, J = 8.5 Hz, 1H), 7.50 (d, J = 8.5 Hz, 1H), 4.39 (s, 2H), 3.52 (d, J = 6.1 Hz, 2H), 3.07 (d, J = 6.2 Hz, 2H).

### Preparative Example 71-3: (8-(4-nitrophenyl)-1,3,4,5-tetrahydro-2H-pyrido[4,3-b]indol-2-yl)(phenyl)methanone (Compound 71)

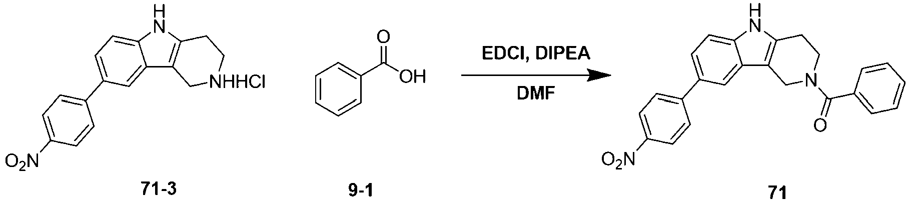

In the same method as in Preparative Example 5, 8-(4-nitrophenyl)-2,3,4,5-tetrahydro-1H-pyrido[4,3-b]indole hydrochloride (23.8 mg, 0.072 mmol), benzoic acid (10.58 mg, 0.087 mmol), EDCI (55.34 mg, 0.29 mmol) and DIPEA (0.05 mL, 0.29 mmol) were used to obtain 25.7 mg (89% yield) of the title compound.

¹H NMR (400 MHz, DMSO) δ 11.24 (s, 1H), 8.28 (s, 1H), 8.23 (s, 1H), 8.04 (s, 2H), 7.95 (s, 1H), 7.74 (s, 1H), 7.50 (s, 6H), 4.88 (s, 1H), 4.68 (s, 1H), 4.04 (s, 1H), 3.69 (s, 1H), 2.90 (s, 2H).

### Preparative Example 72: (8-(4-Aminophenyl)-1,3,4,5-tetrahydro-2H-pyrido[4,3-b]indol-2-yl)(phenyl)methanone (Compound 72)

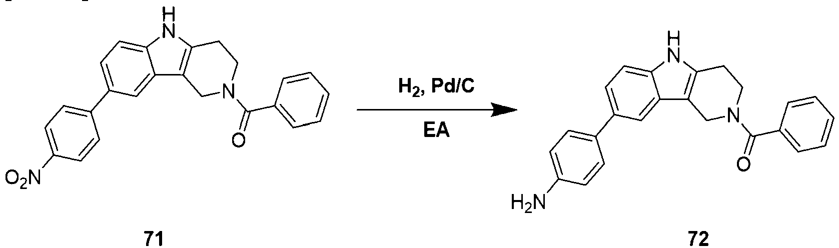

(8-(4-Nitrophenyl)-1,3,4,5-tetrahydro-2H-pyrido[4,3-b]indol-2-yl)(phenyl) methanone (20 mg, 0.05 mmol) and 10% Pd/C (5.32 mg, 0.005 mmol) were dissolved in ethyl acetate. The reaction mixture was stirred under hydrogen for 3 h at room temperature. After the reaction was terminated, the reaction mixture was filtered using ethyl acetate, the filtrate was concentrated under reduced pressure, and then solidified with ether to give 6.3 mg (34% yield) of the title compound.

¹H NMR (400 MHz, DMSO) δ 10.91 (s, 1H), 7.61 (s, 1H), 7.49 (s, 5H), 7.38 (s, 1H), 7.29 (d, J = 10.9 Hz, 3H), 6.63 (s, 1H), 6.59 (s, 1H), 5.06 (s, 2H), 4.83 (s, 1H), 4.62 (s, 1H), 4.03 (s, 1H), 3.67 (s, 1H), 2.87 (s, 2H).

### Preparative Example 73: Preparation of Compound 73

### Preparative Example 73-1: tert-Butyl 8-(4-(dimethylamino)phenyl)-1,3,4,5-tetrahydro-2H-pyrido[4,3-b]indole-2-carboxylate (Compound 73-2)

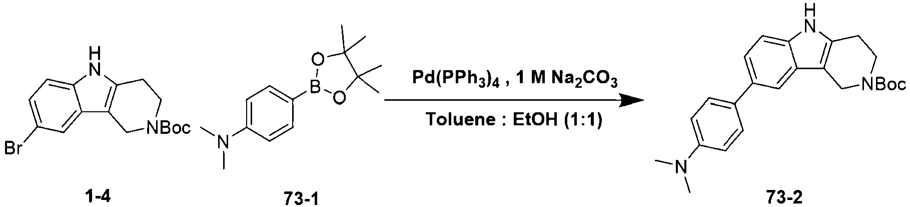

In the same method as in Preparative Examples 1-3, tert-butyl 8-bromo-1,3,4,5-tetrahydro-2H-pyrido[4,3-b]indole-2-carboxylate (100 mg, 0.28 mmol), N,N-dimethyl-4-(4,4,5,5-tetramethyl-1,3,2-dioxaborolan-2-yl)aniline (211.08 mg, 0.85 mmol), Pd(PPh₃)₄ (16.46 mg, 0.014 mmol) and 1 M Na₂CO₃ (0.71 mL, 0.71 mmol) were used to obtain 25.7 mg (yield 23%) of the title compound.

¹H NMR (400 MHz, DMSO) δ 10.85 (s, 1H), 7.55 - 7.46 (m, 3H), 7.28 (t, J = 7.0 Hz, 2H), 6.78 (d, J = 8.4 Hz, 2H), 4.55 (s, 2H), 3.70 (t, J = 5.6 Hz, 2H), 2.91 (s, 6H), 2.74 (d, J = 15.5 Hz, 2H), 1.43 (s, 9H).

### Preparative Example 73-2: N,N-dimethyl-4-(2,3,4,5-tetrahydro-1H-pyrido[4,3-b]indol-8-yl)aniline hydrochloride (Compound 73-3)

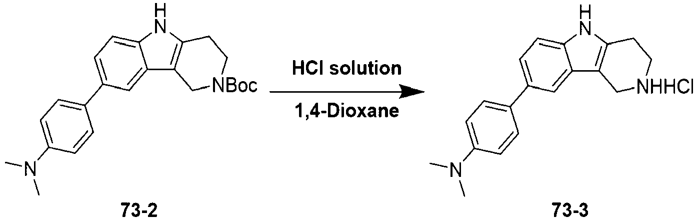

In the same method as in Preparative Examples 1-4, tert-butyl 8-(4-(dimethylamino)phenyl)-1,3,4,5-tetrahydro-2H-pyrido[4,3-b]indole-2-carboxylate (25.7 mg, 0.065 mmol) and HCl solution (4 M in dioxane) (0.33 mL, 1.31 mmol) were used to obtain 16.4 mg (76% yield) of the title compound.

¹H NMR (400 MHz, DMSO) δ 11.22 (s, 1H), 9.31 (s, 2H), 7.74 (s, 1H), 7.67 (s, 3H), 7.40 (s, 3H), 4.35 (s, 2H), 3.50 (s, 2H), 3.04 (s, 8H).

### Preparative Example 73-3: (8-(4-(Dimethylamino)phenyl)-1,3,4,5-tetrahydro-2H-pyrido[4,3-b]indol-2-yl)(phenyl)methanone (Compound 73)

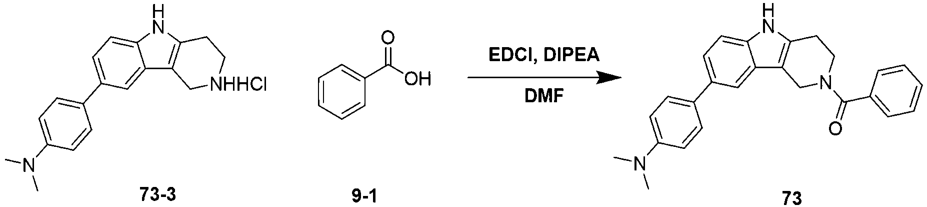

In the same method as in Preparative Example 5, N,N-dimethyl-4-(2,3,4,5-tetrahydro-1H-pyrido[4,3-b]indol-8-yl)aniline hydrochloride (15 mg, 0.046 mmol), benzoic acid (6.7 mg, 0.055 mmol), EDCI (35.08 mg, 0.18 mmol) and DIPEA (0.032 mL, 0.18 mmol) gave 10 mg (yield 55%) of the title compound.

¹H NMR (400 MHz, DMSO) δ 10.95 (s, 1H), 7.67 (s, 1H), 7.50 (s, 6H), 7.32 (s, 3H), 6.78 (d, J = 20.1 Hz, 2H), 4.84 (s, 1H), 4.63 (s, 1H), 4.04 (d, J = 7.3 Hz, 1H), 3.67 (s, 1H), 2.93 (s, 8H).

### Preparative Example 74: N-phenyl-8-(p-tolyl)-1,3,4,5-tetrahydro-2H-pyrido[4,3-b]indole-2-carbothioamide (Compound 74)

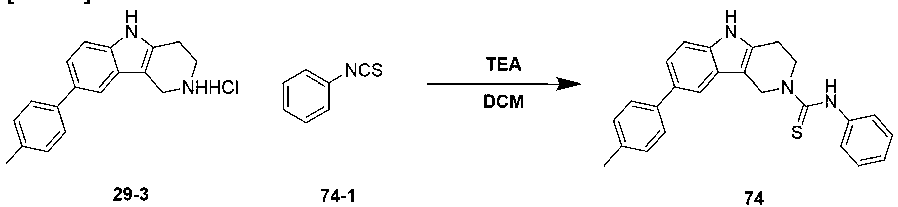

Under nitrogen, 8-(p-Tolyl)-2,3,4,5-tetrahydro-1H-pyrido[4,3-b]indole hydrochloride (40 mg, 0.13 mmol) was dissolved in dichloromethane solution. Isothiocyanatobenzene (0.019 mL, 0.16 mmol) and TEA (0.072 mL, 0.52 mmol) were added and the reaction mixture was stirred at room temperature for 18 hours. Upon completion of the reaction, it was extracted with distilled water and dichloromethane, and the organic layer was dried over magnesium anhydride and filtered. The filtrate was concentrated under reduced pressure, and the concentrate was purified by column chromatography, followed by filtration of the precipitate with diethyl ether to obtain 23 mg (45% yield) of the title compound.

¹H NMR (400 MHz, DMSO) δ 11.04 (s, 1H), 9.44 (s, 1H), 7.62 (d, J=1.5 Hz, 1H), 7.56 (d, J=8.1 Hz, 2H), 7.42 - 7.27(m, 6H), 7.24(d, J=8.0 Hz, 2H), 7.13 (s, 1H), 5.10(s, 2H), 4.35(t, J=5.7 Hz, 2H), 2.96(s, 2H), 2.34(s, 3H).

### Preparative Example 75: N-phenyl-8-(p-tolyl)-1,3,4,5-tetrahydro-2H-pyrido[4,3-b]indole-2-carboxamide (Compound 75)

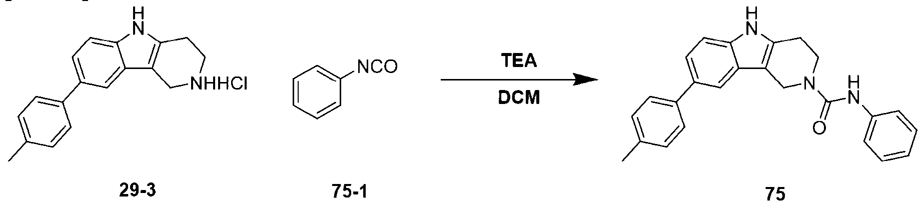

Using the same method as Preparative Example 74, 8-(p-Tolyl)-2,3,4,5-tetrahydro-1H-pyrido[4,3-b]indole hydrochloride (30 mg, 0.1 mmol), isocyanatobenzene (0.013 mL, 0.12 mmol), and TEA (0.056 mL, 0.04 mmol) were used to obtain 28 mg (73% yield) of the title compound.

¹H NMR (400 MHz, DMSO) δ 10.97 (s, 1H), 8.62 (s, 1H), 7.66 (s, 1H), 7.58 (d, J = 8.0 Hz, 2H), 7.51 (d, J = 8.0 Hz, 2H), 7.39 - 7.34 (m, 2H), 7.28 - 7.22 (m, 4H), 6.95 (t, J = 7.4 Hz, 1H), 4.74 (s, 2H), 3.87 (t, J = 5.6 Hz, 2H), 2.87 (s, 2H), 2.35 (s, 3H).

### Preparative Example 76: N,8-di-p-tolyl-1,3,4,5-tetrahydro-2H-pyrido[4,3-b]indole-2-carbothioamide (Compound 76)

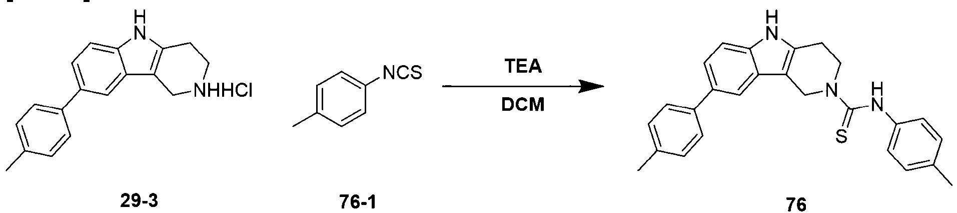

In the same way as Preparative Example 74, 18 mg (36% yield) of the title compound was obtained using 8-(p-Tolyl)-2,3,4,5-tetrahydro-1H-pyrido[4,3-b]indole hydrochloride (35 mg, 0.12 mmol), 1-isothiocyanato-4-methylbenzene (27 mg, 0.18 mmol) and TEA (0.067 mL, 0.48 mmol).

¹H NMR (400 MHz, DMSO) δ 11.05 (s, 1H), 9.37 (s, 1H), 7.61 (s, 1H), 7.56 (d, J = 8.0 Hz, 2H), 7.42 - 7.32 (m, 2H), 7.24 (d, J = 7.8 Hz, 2H), 7.20 (d, J = 8.2 Hz, 2H), 7.12 (d, J = 8.2 Hz, 2H), 5.09 (s, 2H), 4.34 (s, 2H), 2.95 (s, 2H), 2.34 (s, 3H), 2.29 (s, 3H).

### Preparative Example 77: N-(4-cyanophenyl)-8-(p-tolyl)-1,3,4,5-tetrahydro-2H-pyrido[4,3-b]indole-2-carbothioamide (Compound 77)

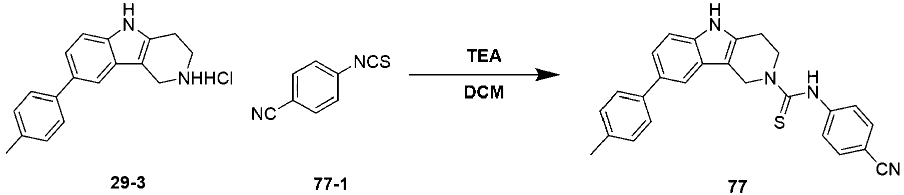

In the same manner as Preparative Example 74, 32 mg (63% yield) of the title compound was obtained using 8-(p-Tolyl)-2,3,4,5-tetrahydro-1H- pyrido[4,3-b]indole hydrochloride (35 mg, 0.12 mmol), 4-isothiocyanatobenzonitrile (29 mg, 0.18 mmol), and TEA (0.067 mL, 0.48 mmol).

¹H NMR (400 MHz, DMSO) δ 11.07 (s, 1H), 9.79 (s, 1H), 7.75 (d, J = 8.4 Hz, 2H), 7.63 (s, 1H), 7.58 (dd, J = 11.5, 8.1 Hz, 4H), 7.42-7.32(m, 2H), 7.24 (d, J=7.8 Hz, 2H), 5.12(s, 2H), 4.35(s, 2H), 3.00(s, 2H), 2.33(s, 3H).

### Results

### Cell-based assay for Tau-Synaptogyrin3 interaction

Since Synaptogyrin3 is a 4-transmembrane neuronal vesicle protein, developing an in vitro assay for detecting Tau-Synaptogyrin3 interaction which includes recombinant protein expression and purification is expected to be difficult. Therefore, a cell-based assay was designed based on HEK293T cells to avoid interference of endogenous Synaptogyrin3 and Tau expression and preference in large scale handling. Ectopic expression of both proteins in HEK293T was confirmed by transfecting EGFP-fused Tau (pTK231: P_{CMV}-Tau-EGFP) and Synaptogyrin3 (pTK233: P_{CMV}-EGFP-Synaptogyrin3). Both proteins were well-expressed in HEK293T cells, Tau-EGFP showed cytosolic localization while EGFP-fused Synaptogyrin3 was localized mostly in granules (FIG. 1), which implicated that the Tau-Synaptogyrin3 interaction can be captured in HEK293T cells.

Protein complementary assay (PCA) has been widely used to detect protein-protein interactions. Bimolecular fluorescent complementation and split luciferase were already extensively used to develop assays to monitor protein-protein interactions of Tau [2, 3]. Split luciferase system called NanoBit that comprises small bit (sBit) and large bit (Lbit) based on NanoLuc [4] used in the assay design due to its small size, lack of oligomerization for functionality and availability of live cell assay substrate. All combinations of sBit/Lbit and Synatogyrin3/Tau fusion proteins (Table 1. pTK225: Tau-sBit, pTK226: Tau-Lbit, pTK227: sBit-Tau, pTK228: Lbit-Tau, pTK245: Synaptogyrin3-sBit, pTK246: Synaptogyrin3-Lbit, pTK247: sBit- Synaptogyrin3 and pTK248: Lbit- Synaptogyrin3) were cloned under CMV promoter for constitutive expression in HEK293T cells. Co-transfection of all possible combinations in HEK293T cells revealed that Lbit-Synaptogyrin3 and sBit-Tau combination showed the highest signal increase, while all Tau/Lbit fusion constructs showed significantly low signal (FIG. 2). Therefore, Lbit-Synaptogyrin3 and sBit-Tau combination was selected for further development.

### Stable cell line production and validation

To increase the efficiency of stable cell line production, dual-promoter and multi-cistronic design was applied to express all necessary components in a single plasmid. Tau-Synaptogyrin3 interaction assay components including fluorescent protein and puromycin resistant gene were cloned into a single plasmid using multiple steps (see Table 1, pTK252: P_{EF1alpha}-sBit-Tau-IRES-EGFP/P_{PGK}-Lbit-Synaptogyrin3-IRES-copGFP-T2A-puroR). Two GFPs (EGFP and copGFP) were integrated into each expression unit of the vector due to convenience of cloning. After puromycin selection of pTK252-transfected HEK293T cells, 8 GFP-positive clones were selected for validation (1E2, 1D6, 2B5, 2C9, 2C10, 3B5, 4D7 and 4F2).

Functionality of Tau-Synaptogyrin3 interaction assay in HEK293T-TK252 stable clones were indirectly validated with competitive expression of native Tau using phloretin-inducible gene expression (FIGS. 3 and 4). Lower luciferase activity was expected as more native Tau without split protein is co-expressed, native Tau is to compete with sBit-Tau to bind to Synaptogyrin3 (FIG. 4a). Phloretin-inducible gene expression system was used to control native Tau expression in dose-responsive manner. Phloretin-inducible gene expression system comprises a synthetic transcription activator TtgA and a synthetic promoter P_{TtgR}, derived from flavonoid-regulated TtgR operon from *Pseudomonas putida* DOT-T1E strain (FIG. 3a) [5]. TtgR binds to its operator site (O_{TtgR}) on DNA and TtgR is released from DNA upon phloretin binding to TtgR. The synthetic transcription factor is a fusion protein of TtgR and Herpes simplex-derived transactivation domain VP16, and the synthetic promoter consists of O_{TtgR} at 5' upstream of CMV minimal promoter. In the absence of phloretin, TtgA binds to P_{TtgR} to activate transcription; in the presence of phloretin, TtgA released from DNA and transcription is not activated. Co-transfection of phloretin-inducible EGFP expression plasmids (pTK269: P_{CAG}-TtgA and pTK274: P_{TtgR}-EGFP) showed dose-dependent EGFP signal (0 - 50 µM phloretin), 4-fold difference between 0 and 50 µM treatment (FIG. 3b). Phloretin-inducible Tau expression plasmids (pTK269: P_{CAG}-TtgA and pTK285: P_{TtgR}-Tau) were co-transfected to HEK293T-TK252 clones and different concentrations of phloretin were treated (0, 10 and 50 µM phloretin). Expected competitive behavior was observed in clone 1E2, 2C9 and 2C10. 1E2 was selected for screening (FIG. 4b); it had the lowest maximum signal, but showed the best dose-response profile with 2-fold difference between 0 and 50 µM treatment. Other clones were not responsive to phloretin (1D6, 4F2 and 4D7) or clearly opposite direction (3B5 and 2B5). Phloretin treatment did not significantly alter EGFP signal among the experimental sets and clones (FIG. 4c).

### Screening and assessing derivatives of hit compound

1,728 compounds were treated at 10 µM to HEK293T-TK252-1E2 cells and incubated for 48 hrs. Phenol red interfered assay significantly (data not shown), so phenol red free-media was used in all steps from cell culture to measurement. Single wash step with culture media without FBS was included before measurement to discard dead cells and to remove FBS that affects luciferase assay. Luciferase activity and fluorescent intensity were measured at single point rather than multiple points in a well, that luciferase signal was gradually reduced in few minutes so measurement speed was critical. Fluorescent intensity was taken for normalization, which is expected to be proportion to the cell number at the measuring point. L/F value which is luciferase activity normalized with fluorescent intensity (L/F = Luciferase activity/ Fluorescent intensity) was calculated for all measurements and compared to L/F value of DMSO controls to calculated the size of inhibitory effect. Average coefficient of variation (CV) calculated with L/F values of triplicates of all measurements was 0.116, indicated that the cell-based assay was stable. Fluorescence area scan (FA) performed right after luciferase activity and fluorescent intensity measurement, to validate total remaining fluorescent cells in a well as a proxy to the cellular toxicity. FA value was in proportion to WST assay results (data not shown), and average CV of triplicates of all measurements was 0.04.

61 compounds were further tested for dose-response profile, based on inhibitory effect that was measured by L/F value (<60%) and FA-based cell viability (>90%) at 10 µM compared to DMSO control. 10 compounds were considered as hit compounds through three times of reproduction. IC₅₀ values of the derivative compounds (indicated as Formula #) were shown in Table 4.

**[Table 4]**

| Formula | Activity | Formula | Activity | Formula | Activity | Formula | Activity |
|---|---|---|---|---|---|---|---|
| 2 | ++ | 21 | ++ | 40 | +++ | 59 | ++ |
| 3 | ++ | 22 | ++ | 41 | ++ | 60 | ++ |
| 4 | ++ | 23 | ++ | 42 | + | 61 | ++ |
| 5 | ++ | 24 | ++ | 43 | ++ | 62 | ++ |
| 6 | + | 25 | ++ | 44 | +++ | 63 | +++ |
| 7 | ++ | 26 | + | 45 | ++ | 64 | +++ |
| 8 | ++ | 27 | +++ | 46 | ++ | 65 | +++ |
| 9 | ++ | 28 | +++ | 47 | ++ | 66 | + |
| 10 | ++ | 29 | +++ | 48 | + | 67 | ++ |
| 11 | ++ | 30 | +++ | 49 | + | 68 | ++ |
| 12 | + | 31 | + | 50 | +++ | 69 | ++ |
| 13 | + | 32 | +++ | 51 | ++ | 70 | +++ |
| 14 | +++ | 33 | ++ | 52 | +++ | 71 | +++ |
| 15 | ++ | 34 | ++ | 53 | ++ | 72 | +++ |
| 16 | ++ | 35 | ++ | 54 | ++ | 73 | ++ |
| 17 | ++ | 36 | ++ | 55 | + | 74 | ++ |
| 18 | ++ | 37 | +++ | 56 | ++ | 75 | + |
| 19 | ++ | 38 | + | 57 | ++ | 76 | ++ |
| 20 | ++ | 39 | ++ | 58 | ++ | 77 | + |

| | | | | | | | |
|---|---|---|---|---|---|---|---|
| +++, IC₅₀ < 3 µM ++, 3 µM ≤ IC₅₀ ≤ 12 µM +, 12 µM < IC₅₀ ≤ 25 µM | | | | | | | |

Having described specific embodiment of the present invention in detail above, it is to be understood that variants and modifications thereof falling within the spirit of the invention may become apparent to those skilled in this art, and the scope of this invention is to be determined by appended claims and their equivalents.

### References

1. Schlatter, S., et al: *Gene,* **282** (1-2): 19-31 (2002).
2. Wegmann, S., et al, *J Neurochem,* **139**(6): 1163-1174 (2016).
3. Tak, H., et al, *PLoS One,* **8**(12): e81682 (2013).
4. Dixon, A.S., et al, *ACS Chem Biol,* **11**(2): 400-408 (2016).
5. Gitzinger, M., et al, *Proc Natl Acad Sci USA,* **106**(26): 10638-10643 (2009).

## Claims

1. A compound represented by the following Formula 1: 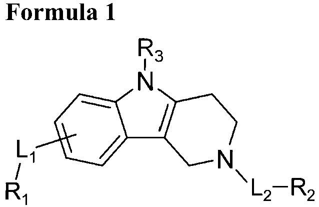
wherein R₁ is C₁-C₃ alkyl; or C₆-C₁₀ aryl or 5 to 12-membered heteroaryl unsubstituted or substituted with C₁-C₃ alkyl, C₃-C₇ cycloalkyl, C₁-C₃ alkoxy, halogen, -CN, -NO₂ or -NR₃ R₄ (where R₃ and R₄ are each independently hydrogen or C₁-C₃alkyl);
R₂ is C₆-C₁₀ aryl, 5 to 12-membered heteroaryl, C₃-C₇ cycloalkyl or 6-membered heterocycloalkyl unsubstituted or substituted with C₁-C₃ alkyl, C₁-C₃ haloalkyl, C₁-C₃ alkoxy, halogen, -CN, -NO₂ or -NR₅ R₆ (where R₅ and R₆ are each independently hydrogen or C₁-C₃ alkyl);
R₃ is hydrogen or C₁-C₃ alkyl;
L₁ is a direct bond or -NH-;
L₂ is a direct bond, -C(O)-, -C(S)-, -C(O)NH-, -C(S)NH- or -S(O₂)-.

2. The compound of claim 1, wherein R₁ is C₁-C₂ alkyl; or a phenyl or 5 to 9-membered heteroaryl unsubstituted or substituted with C₁-C₃ alkyl, C₅-C₆ cycloalkyl, C₁-C₃ alkoxy, halogen, -CN, -NO₂, -NH₂ or -N(CH₃)₂.

3. The compound of claim 2, wherein the heteroaryl is pyridine.

4. The compound of claim 1, wherein R₂ is phenyl, 5 to 9-membered heteroaryl, C₅-C₆ cycloalkyl or morpholine unsubstituted or substituted with C₁-C₃ alkyl, trihalomethyl, C₁-C₃ alkoxy, -N(CH₃)₂), -CN or halogen.

5. The compound of claim 4, wherein the heteroaryl is selected from the group consisting of pyridine, pyrimidine, pyrazine, thiazole and indole.

6. The compound of claim 1, wherein L₂ is -C(O)NH- or -C(S)NH-; and R₂ is phenyl unsubstituted or substituted with C₁-C₂ alkyl or -CN.

7. The compound of claim 1, wherein the compound represented by Formula 1 is selected from the group consisting of the compounds represented by following Formula 2 to 77:

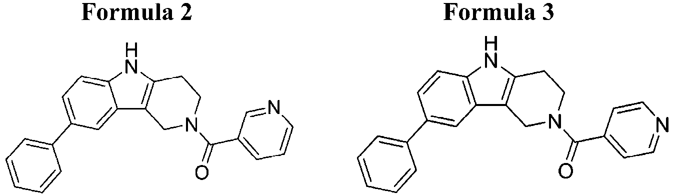

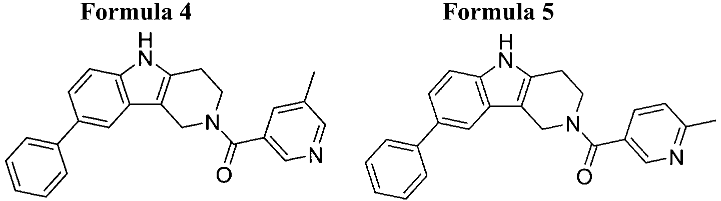

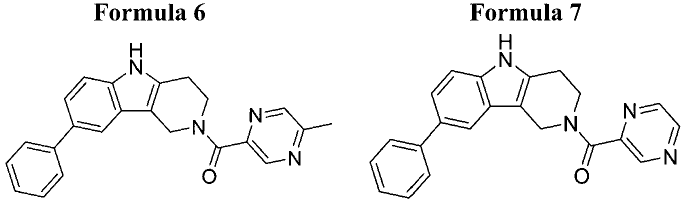

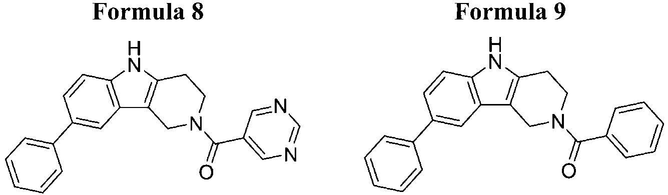

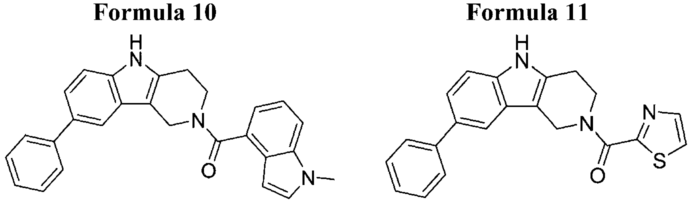

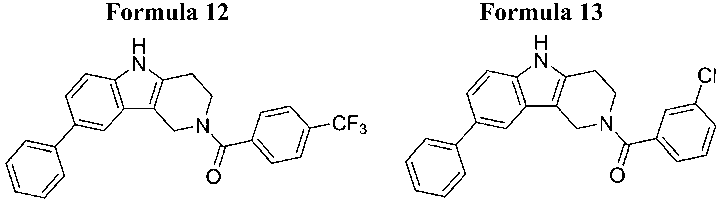

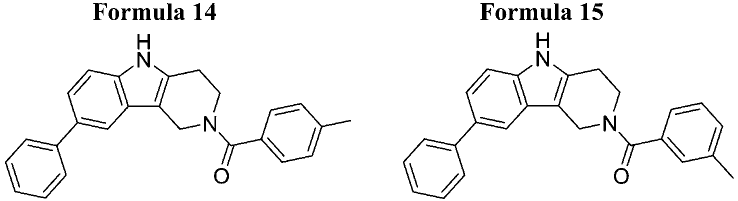

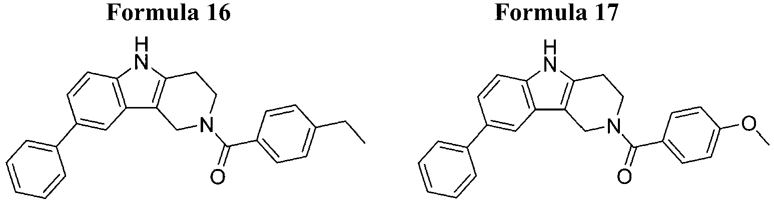

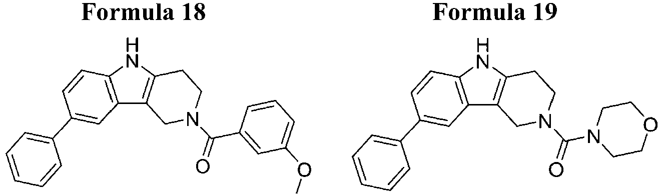

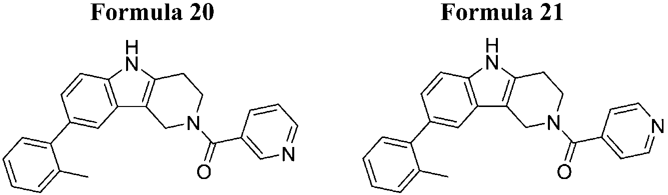

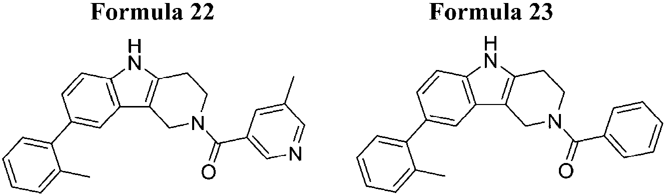

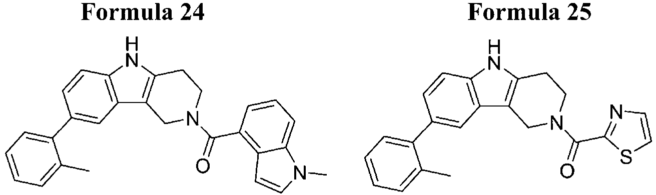

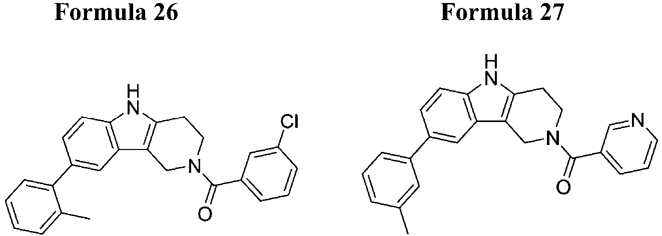

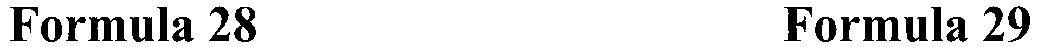

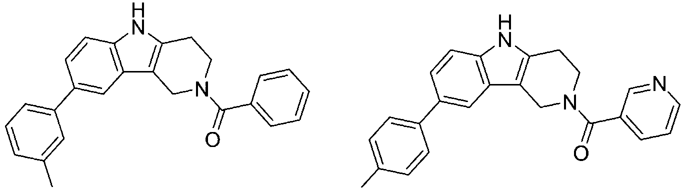

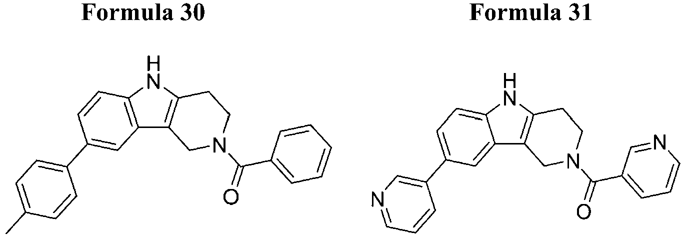

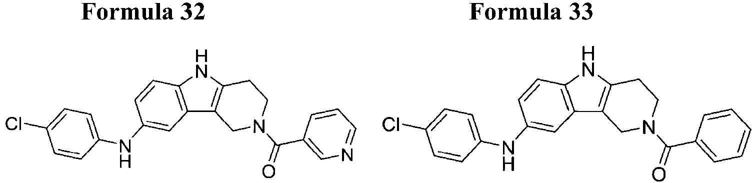

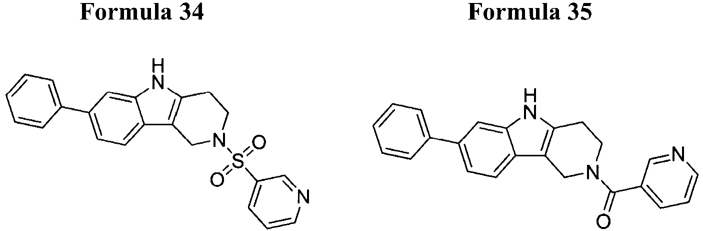

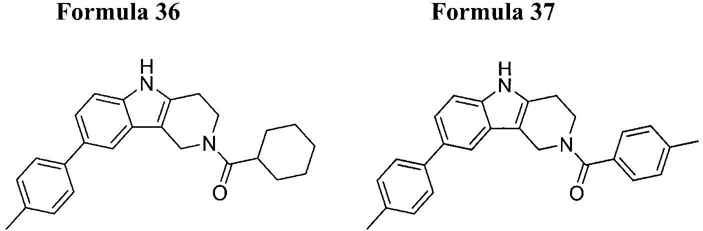

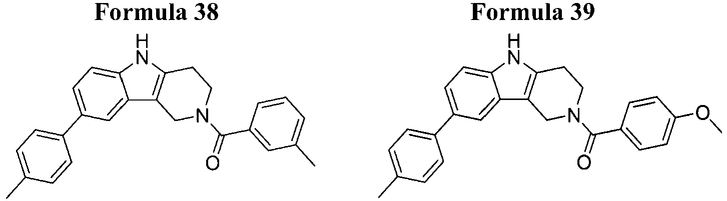

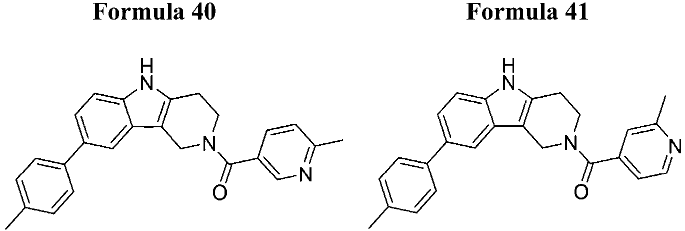

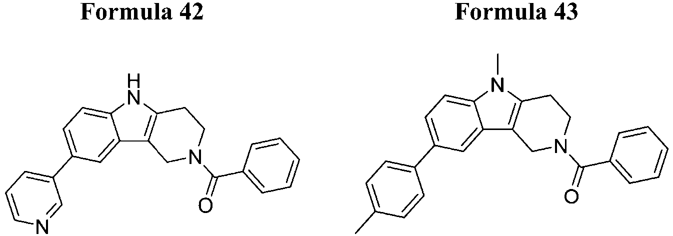

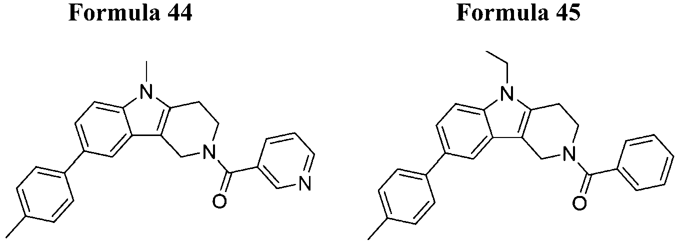

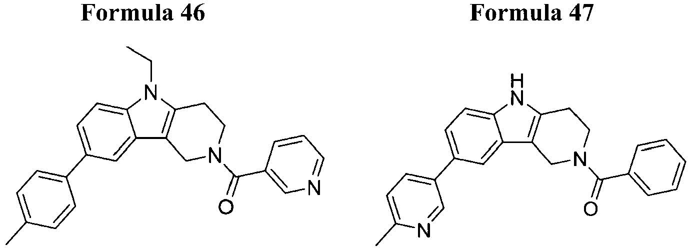

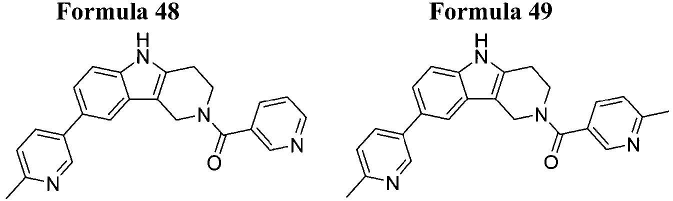

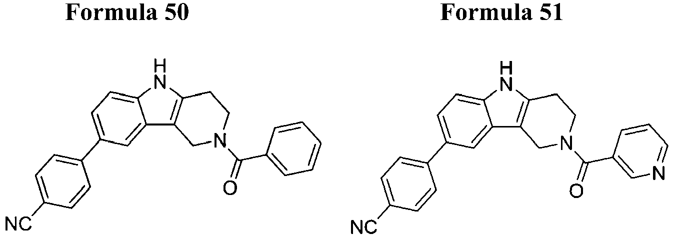

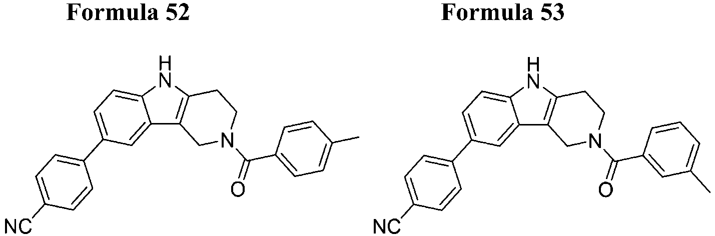

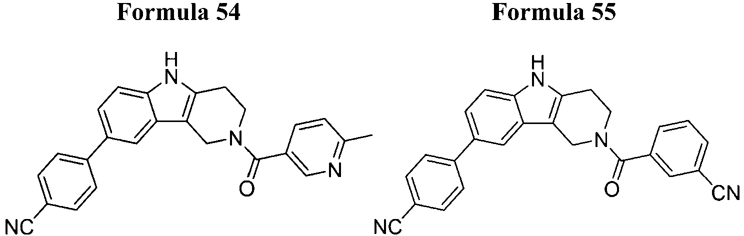

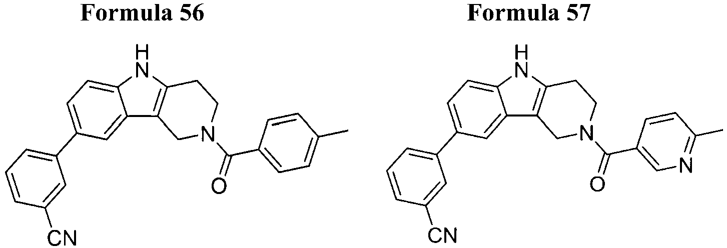

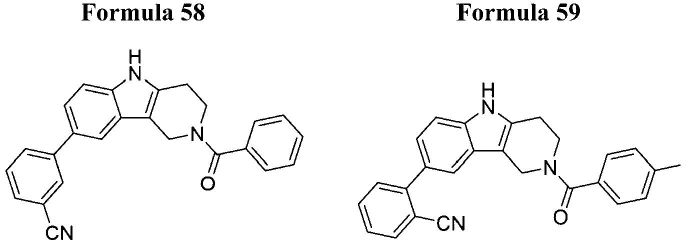

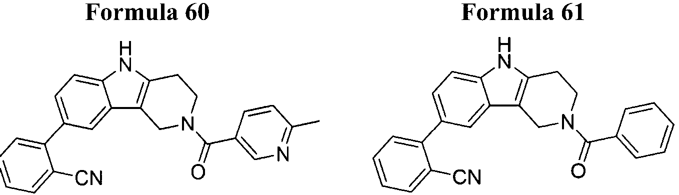

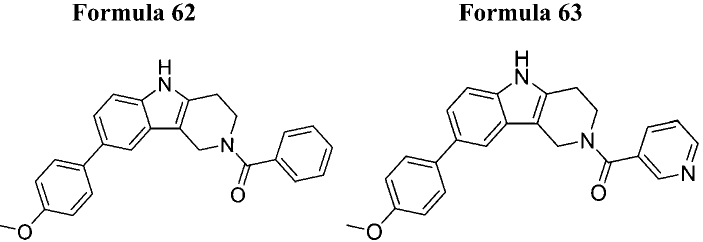

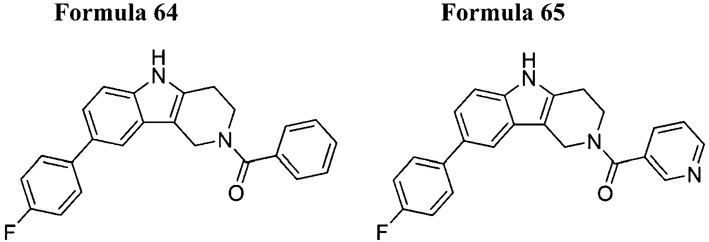

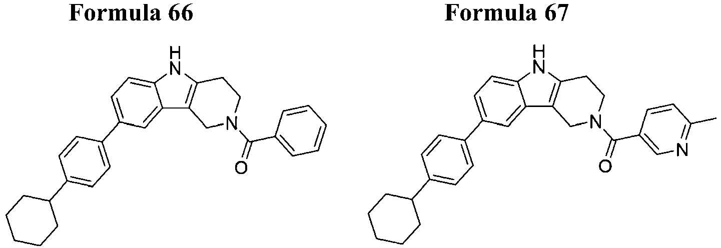

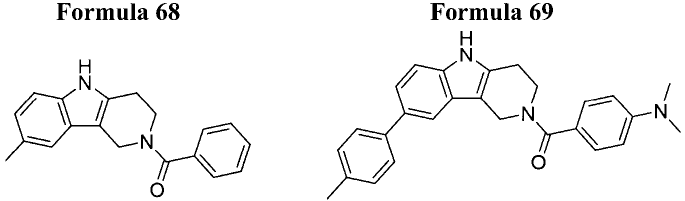

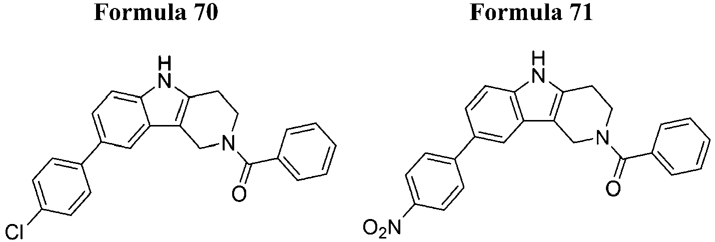

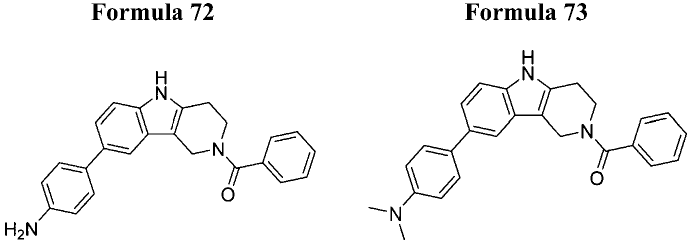

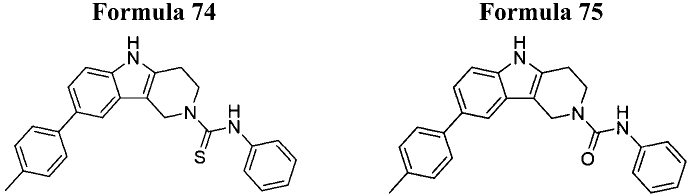

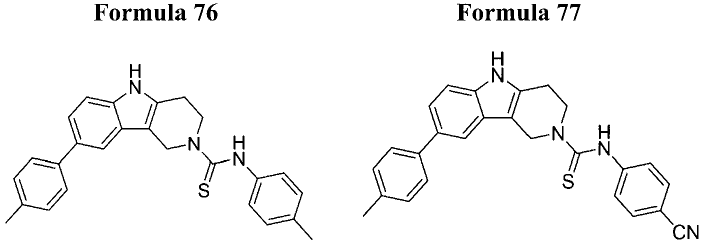

8. A composition for preventing or treating a neurodegenerative disease comprising a compound of any one of claims 1 to 7 or a pharmaceutically acceptable salt thereof, as an active ingredient.

9. The composition of claim 8, wherein the neurodegenerative disease is selected from the group consisting of Alzheimer's disease, AGD (argyrophilic grain disease), Lewy body dementia, frontotemporal dementia, PSP (Progressive supranuclear palsy), PSP-P (progressive supranuclear palsy-Parkinsonism), Richardson's syndrome, Pick's disease, Niemann-Pick disease, Rasmussen's syndrome, Parkinson's disease, Atypical parkinsonism in Guadeloupe, FTDP-17 (Frontotemporal dementia with parkinsonism associated with chromosome 17), Progressive subcortical gliosis, Primary Progressive Aphasia, Globular gilal tauopathy, Lytico-Bodig disease, Neurodegeneration with brain iron accumulation, PKAN (Pantothenate kinase-associated neurodegeneration), Postencephalitic parkinsonism, CTE (chronic traumatic encephalopathy), Familial British dementia, Familial Danish dementia, Huntington's disease, Down's syndrome, Gerstmann-Straussler-Scheinker disease, Myotonic dystrophy, White matter tauopathy, ALS (Amyotrophic Lateral Sclerosis), Cerebral amyloid angiopathy, Senile dementia of the neurofibrillary tangle type, Motor neuron disease with neurofibrillary tangles, Diffuse neurofibrillary tangles with calcification, Corticobasal degeneration, Primary age-related tauopathy and Traumatic brain injury.

10. The composition of claim 8, wherein the composition inhibits the interaction between tau and synaptogyrin 3.

11. A functional food composition for preventing or alleviating a neurodegenerative disease, comprising a compound of any one of claims 1 to 7 or a sitologically acceptable salt thereof, as an active ingredient.

12. A functional food composition for inhibiting the interaction between tau and synaptogyrin 3, comprising a compound of any one of claims 1 to 7 or a sitologically acceptable salt thereof, as an active ingredient.
